# EUROPÄISCHE PATENTSCHRIFT

(11) **EP 3 831 278 B1**
(45) Veröffentlichungstag und Bekanntmachung des Hinweises auf die Patenterteilung: **27.08.2025**
(21) Anmeldenummer: 20208612.0
(22) Anmeldetag: 19.11.2020
(51) Int. Cl.: A61B 5/00

(54) **ANORDNUNG UND VERFAHREN ZUM DARSTELLEN VON MEDIZINISCHEN ALARMEN**
ARRANGEMENT AND METHOD FOR PRESENTING MEDICAL ALARMS
AGENCEMENT ET PROCÉDÉ DE REPRÉSENTATION DES ALARMES MÉDICALES

(30) Priorität: 04.12.2019 DE 102019008406
(43) Veröffentlichungstag der Anmeldung: 09.06.2021
(73) Patentinhaber: Drägerwerk AG & Co. KGaA, 23558 Lübeck (DE)
(72) Erfinder: Ise, Edgar, 23558 Lübeck (DE); Gömann, Michael, 23558 Lübeck (DE); Fischer, Sebastian, 23558 Lübeck (DE); Kern, Andi, 23558 Lübeck (DE); Windhorst, Konradin, 23558 Lübeck (DE)

(56) Entgegenhaltungen:
- EP-A1- 2 777 488
- WO-A1-2016/188741
- US-A1- 2013 032 149

## Beschreibung

Die Erfindung betrifft eine Anordnung mit einem medizinischen Gerät, einer Signalverarbeitungseinheit, einer Ausgabeeinheit und bevorzugt einer Eingabeeinheit, wobei die Signalverarbeitungseinheit Messwerte von mindestens einem Patienten-Sensor empfängt, Alarme detektiert und die Ausgabeeinheit ansteuert, wobei die angesteuerte Ausgabeeinheit visuell Informationen an einen Benutzer auszugeben vermag und wobei die optionale Eingabeeinheit Benutzereingaben zu erfassen vermag. Weiterhin betrifft die Erfindung ein Verfahren zum Darstellen von medizinischen Informationen unter Verwendung einer solchen Anordnung.

In DE 10 2016 001 139 A1 wird ein Beatmungssystem mit einer Versorgungseinrichtung und einer Anzeigeeinrichtung 15 beschrieben. Entlang einer Zeitachse 19 wird für aufeinanderfolgende Zeitpunkte ein Erzeugermarker 23 angezeigt. Der Erzeugermarker 23 ist derart positioniert, dass das Verhältnis seines Abstandes von der Zeitachse 19 zu dem Abstand zwischen der Zeitachse und einer Begrenzungslinie 21 gleich dem Verhältnis eines ersten Erzeugerparameters zu der Summe aus dem ersten Erzeugerparameter und einem zweiten Erzeugerparameter abzüglich eines ersten Verbraucherparameters ist.

US 2014 / 0 275 819 A1 zeigt ein Patienten-Überwachungsgerät (medical monitoring device) mit mehreren Sensoren, die jeweils einen physiologischen Parameter eines Patienten überwachen. Eine Signalverarbeitungseinheit (processing circuitry 204) empfängt Messwerte von den Sensoren und steuert eine Ausgabeeinheit (device 200 mit displays 202) sowie eine Alarmierungseinheit (alarm mechanism 216) an. Auf dem Bildschirm werden der jeweilige zeitliche Verlauf von verschiedenen Signalen, welche jeweils einen physiologischen Parameter des Patienten beschreiben, sowie bei von einem Normalwert abweichenden Werten alarm marker shadings und alarm marker lines an den jeweiligen Zeitpunkten dargestellt. Ein Benutzer kann den dargestellten Zeitraum verschieben und eine Darstellung vergrößern.

Vorrichtungen und Verfahren, um einen Patienten zu überwachen (patient monitoring) und um medizinische Alarme anzuzeigen, werden auch in US 2013 / 0 032 149 A1, WO 2016 / 188741 A1, EP 2 777 488 A1, US 2003 / 0 200 117 A1, US 2005 / 0 038 332 A1, US 2008/ 0 078 390 A1, US 2011 / 0 138 311 A1, US 2013 / 0 246 089 A1, US 2018 / 0 277 243 A1 und US 2018 / 0 300 919 A1 beschrieben.

Der Erfindung liegt die Aufgabe zugrunde, eine Anordnung umfassend ein medizinisches Gerät, eine Ausgabeeinheit und eine Signalverarbeitungseinheit sowie ein Verfahren zum Darstellen von Alarmen auf einer Ausgabeeinheit bereitzustellen, wobei es möglich sein soll, eine Vielzahl von Alarmen auch dann ergonomisch darzustellen, wenn eine Darstellungsfläche der Ausgabeeinheit im Vergleich zu der Anzahl von detektierten Alarmen klein ist.

Die Aufgabe wird durch eine Anordnung mit den Merkmalen des Anspruchs 1 und durch ein Verfahren mit den Merkmalen des Anspruchs 11 gelöst. Vorteilhafte Ausgestaltungen sind in den Unteransprüchen angegeben. Vorteilhafte Ausgestaltungen der erfindungsgemäßen Anordnung sind auch vorteilhafte Ausgestaltungen des erfindungsgemäßen Verfahrens und umgekehrt, soweit sinnvoll.

Die erfindungsgemäße Anordnung umfasst ein medizinisches Gerät, insbesondere ein Beatmungsgerät oder ein Anästhesiegerät. Das medizinische Gerät umfasst mindestens einen Patienten-Sensor, bevorzugt mehrere Patienten-Sensoren, oder lässt sich wenigstens zeitweise mit mindestens einem Patienten-Sensor verbinden. Der oder jeder Patienten-Sensor vermag jeweils mindestens eine Größe zu messen, die an oder in einem Patienten auftritt, bevorzugt mindestens einen Vitalparameter des Patienten.

Die erfindungsgemäße Anordnung umfasst weiterhin eine Ausgabeeinheit, welche Informationen visuell an einen Benutzer auszugeben vermag, beispielsweise auf einem Bildschirm. Weiterhin umfasst die Anordnung eine datenverarbeitende Signalverarbeitungseinheit, welche die Ausgabeeinheit anzusteuern vermag und bevorzugt eine Benutzereingabe zu erfassen vermag.

Die Signalverarbeitungseinheit vermag Messwerte zu empfangen, welche der oder mindestens ein Patienten-Sensor erzeugt hat. Indem die Signalverarbeitungseinheit empfangene Messwerte auswertet, vermag die Signalverarbeitungseinheit mindestens ein Signal zu erzeugen. Dieses Signal korreliert mit einer zeitlich veränderlichen Größe, die am oder im Patienten auftritt, beispielsweise mit einer Größe für die spontane Atmung oder für den Herzschlag oder für den Sauerstoffgehalt im Blut oder den CO2-Gehalt in der ausgeatmeten Atemluft des Patienten. Bevorzugt vermag die Signalverarbeitungseinheit mehrere Signale zu erzeugen und hierfür bevorzugt Messwerte von verschiedenen Patienten-Sensoren zu verarbeiten.

Vorgegeben ist mindestens ein Alarm-Kriterium. Das oder jedes vorgegebene Alarm-Kriterium bezieht sich auf das oder mindestens ein Signal, welches die Signalverarbeitungseinheit aus Messwerten des oder mindestens eines Patienten-Sensors herzuleiten vermag.

Die Signalverarbeitungseinheit vermag automatisch zu entscheiden, ob das oder mindestens ein vorgegebenes Alarm-Kriterium erfüllt ist. Für diese Entscheidung vermag die Signalverarbeitungseinheit das oder jeweils mindestens ein erzeugtes Signal auszuwerten. Möglich ist, dass verschiedene vorgegebene Alarm-Kriterien sich auf unterschiedliche Signale oder auch auf das gleiche Signal beziehen

Wenn die Signalverarbeitungseinheit entschieden hat, dass das oder ein Alarm-Kriterium erfüllt ist, so hat sie automatisch einen Alarm detektiert. Ein Alarm liegt vor, wenn mindestens ein Signal zu einem bestimmten Zeitpunkt ein vorgegebenes Alarm-Kriterium erfüllt. Die Signalverarbeitungseinheit vermag diesen Alarm sowie den Zeitpunkt, an dem das Alarm-Kriterium erfüllt und somit der Alarm aufgetreten ist, zu detektieren. Dasselbe Alarm-Kriterium kann mehrmals erfüllt sein, nämlich zu unterschiedlichen Zeitpunkten. Für dasselbe Signal können mindestens zwei unterschiedliche Alarm-Kriterien vorgegeben sein und daher gleichzeitig oder zu verschiedenen Zeitpunkten erfüllt sein. Jedes Mal, wenn das oder ein Alarm-Kriterium erfüllt und dieses Ereignis detektiert ist, hat die Signalverarbeitungseinheit erneut einen Alarm detektiert. Jedes Alarm-Kriterium legt jeweils eine Alarm-Art fest. Jeder Alarm gehört zu jeweils einer Alarm-Art. Möglich ist, dass das gleiche Alarm-Kriterium mehrmals erfüllt ist, nämlich zu unterschiedlichen Zeitpunkten. Dann sind nacheinander mehrere gleichartige Alarm-Arten aufgetreten.

Die Signalverarbeitungseinheit vermag die Ausgabeeinheit so anzusteuern, dass die angesteuerte Ausgabeeinheit wenigstens zeitweise verschiedene Darstellungen gleichzeitig anzeigt, nämlich gleichzeitig mindestens die folgenden Darstellungen:
- eine Alarm-Gesamt-Abfolge,
- einen Alarm-Referenz-Abschnitt,
- eine Positionierungs-Darstellung und
- eine Signalverlaufs-Darstellung oder eine Alarm-Referenz-Abfolge oder sowohl eine Signalverlaufs-Darstellung als auch eine Alarm-Referenz-Abfolge.

Die Alarm-Gesamt-Abfolge zeigt eine zeitliche Abfolge von Alarmen, die in einem vorgegebenen Gesamt-Zeitraum detektiert wurden. Bevorzugt zeigt die Alarm-Gesamt-Abfolge alle Alarme, die in dem Gesamt-Zeitraum detektiert wurden, und zwar bevorzugt grafisch.

Der Alarm-Referenz-Abschnitt zeigt eine zeitliche Abfolge von Alarmen, die in einem vorgegebenen und bevorzugt veränderbaren Referenz-Zeitfenster detektiert wurden. Dieses Referenz-Zeitfenster ist ein Ausschnitt, also ein Teil, des Gesamt-Zeitraums. Mindestens ein weiterer Teil des Gesamt-Zeitraums liegt nicht im Referenz-Zeitfenster. Der Alarm-Referenz-Abschnitt ist ein Abschnitt der Alarm-Gesamt-Abfolge.

Sowohl die Alarm-Gesamt-Abfolge als auch der Alarm-Referenz-Abschnitt erstrecken sich auf der Ausgabeeinheit in dieselbe Zeitachsen-Darstellungs-Richtung. In vielen Anwendungen ist dies eine horizontale Darstellungs-Richtung auf dem Bildschirm, und zwar von links nach rechts oder auch von rechts nach links. Möglich ist auch eine vertikale Darstellungs-Richtung.

Der Alarm Referenz-Abschnitt zeigt Alarme im Referenz-Zeitfenster und stellt zusätzlich eine Positionierungs-Darstellung bereit. Diese Positionierungs-Darstellung zeigt, wie das Referenz-Zeitfenster relativ zum Gesamt-Zeitraum zeitlich positioniert ist.

Die Signalverlaufs-Darstellung zeigt den zeitlichen Verlauf des oder mindestens eines erzeugten Signals, und zwar den jeweiligen zeitlichen Verlauf des Signals im Referenz-Zeitfenster.

Die Alarm-Referenz-Abfolge zeigt eine Abfolge von Alarmen, die im Referenz-Zeitfenster detektiert worden sind, bevorzugt jeden Alarm, der im Referenz-Zeitfenster detektiert worden ist.

Wenigstens zeitweise stellt die angesteuerte Ausgabeeinheit die Alarm-Gesamt-Abfolge, den Alarm-Referenz-Abschnitt mitsamt der bereitgestellten Positionierungs-Darstellung sowie die Signalverlaufs-Darstellung und / oder die Alarm-Referenz-Abfolge gleichzeitig dar.

Sowohl die Alarm-Gesamt-Abfolge als auch die Signalverlaufs-Darstellung als auch die Alarm-Referenz-Abfolge erstrecken sich in dieselbe Zeitachsen-Darstellungs-Richtung.

Der Zeitmaßstab für die Signalverlaufs-Darstellung ist feiner als der Zeitmaßstab für die Alarm-Gesamt-Abfolge und feiner als der Zeitmaßstab für den Alarm-Referenz-Abschnitt. Auch der Zeitmaßstab für die Alarm-Referenz-Abfolge ist feiner als der Zeitmaßstab für die Alarm-Gesamt-Abfolge und feiner als der Zeitmaßstab für den Alarm-Referenz-Abschnitt.

Sowohl der Alarm-Referenz-Abschnitt als auch die Alarm-Referenz-Abfolge beziehen sich auf das Referenz-Zeitfenster. Die Alarm-Referenz-Abfolge zeigt aber Alarme im Referenz-Zeitfenster mit einem feineren Zeitmaßstab als der Alarm-Referenz-Abschnitt. Der Alarm-Referenz-Abschnitt erleichtert es einen Benutzer, die im Referenz-Zeitfenster aufgetretenen Alarme mit jenen, die im Gesamt-Zeitraum aufgetreten sind, zu vergleichen.

Möglich ist, dass ein einziger feinerer Zeitmaßstab für die Signalverlaufs-Darstellung und für die Alarm-Referenz-Abfolge und ein einziger gröberer Zeitmaßstab für die Alarm-Gesamt-Abfolge und den Alarm-Referenz-Abschnitt verwendet wird. Möglich sind auch mehr als zwei unterschiedliche Zeitmaßstäbe.

Der Begriff "feinerer Zeitmaßstab" wird nachfolgend definiert. Er entspricht dem Begriff "feinerer Abbildungsmaßstab" für geographische Darstellungen, z.B. für Landkarten und Stadtplänen. Das Gegenteil von "feinerer Zeitmaßstab" ist "gröberer Zeitmaßstab".

Sowohl die Signalverlaufs-Darstellung als auch die Alarm-Referenz-Abfolge nehmen auf einer Darstellungsfläche der Ausgabeeinheit jeweils eine bestimmte räumliche Ausdehnung in die Zeitachsen-Darstellungs-Richtung ein. Unter dem Begriff "Zeitmaßstab" wird das Verhältnis der räumlichen Ausdehnung zu dem dargestellten Zeitraum verstanden, beispielsweise in [mm] pro [sec], in [cm] oder [inch] pro [min] oder in [cm] oder [inch] pro [h]. In einer Darstellung mit einem feineren Zeitmaßstab wird derselbe Zeitraum mit einer größeren räumlichen Ausdehnung in die Zeitachsen-Darstellungs-Richtung dargestellt als bei einem gröberen Zeitmaßstab. Anders formuliert: Ein Zeitmaßstab A ist feiner als ein Zeitmaßstab B, wenn eine Darstellung mit dem Zeitmaßstab A denselben Zeitraum mit einer größeren räumlichen Ausdehnung als eine Darstellung mit dem Zeitmaßstab B darstellt.

Gerade im medizinischen Bereich wird einerseits häufig gewünscht, eine große Anzahl von Alarmen auf einer Ausgabeeinheit in einer einzigen Darstellung anzuzeigen. Ein Betrachter kann diese globale Darstellung verwenden, um sich einen Überblick über eine zeitliche Entwicklung und über zeitliche Häufungen von Alarmen zu verschaffen. Andererseits will ein Benutzer das Dargestellte erkennen können, und die maximal mögliche Ausdehnung, die eine Darstellungsfläche der Ausgabeeinheit einnehmen kann, ist häufig beschränkt, um Anforderungen an medizinischen Abläufen gerecht zu werden und / oder weil nicht mehr Platz zur Verfügung steht. Die Erfindung zeigt einen Weg auf, um trotz dieser einander widersprechenden Anforderungen relativ übersichtlich und ergonomisch den internen Status des medizinischen Geräts der erfindungsgemäßen Anordnung auf der Ausgabeeinheit anzeigen zu können.

Erfindungsgemäß werden mindestens ein feinerer Zeitmaßstab und mindestens ein gröberer Zeitmaßstab verwendet, nämlich jeweils ein feinerer Zeitmaßstab für die Signalverlaufs-Darstellung und die Alarm-Referenz-Abfolge und ein gröberer Zeitmaßstab für die Alarm-Gesamt-Abfolge und den Alarm-Referenz-Abschnitt. Jede Darstellung mit dem oder einem feineren Zeitmaßstab vermag in vielen Fällen einen Sachverhalt auch dann noch relativ rasch wahrnehmbar und / oder ausreichend detailliert darzustellen, wenn die verwendete Ausgabeeinheit eine relativ geringe Abmessung und / oder eine relativ geringe Auflösung, beispielsweise relativ wenige Bildpunkte (Pixel), aufweist. Jede Darstellung mit dem oder einem gröberen Zeitmaßstab vermag in vielen Fällen eine größere Anzahl von Alarmen gleichzeitig darzustellen. Gerade die Signalverarbeitungseinheit in einer medizinischen Anordnung detektiert häufig in einem relativ kurzen Zeitraum eine große Anzahl von Alarmen, die sich auf einen Patienten beziehen und die in einer einzigen Darstellung angezeigt werden sollen. Gerade im medizinischen Kontext sollte jede Situation, die für einen Patienten gefährlich sein kann oder ein Indiz für eine mögliche Gefährdung ist, angezeigt werden.

Erfindungsgemäß werden auf der Ausgabeeinheit gleichzeitig mindestens eine Darstellung mit dem feineren Zeitmaßstab und mindestens eine Darstellung mit dem gröberen Zeitmaßstab gezeigt. Dieses Merkmal erspart die Notwendigkeit, zwischen verschiedenen Zeitmaßstäben umzuschalten, und spart somit eine Benutzerinteraktion, Arbeitszeit und Aufmerksamkeit des Benutzers ein, in manchen Fällen außerdem eine aufgrund der Benutzerinteraktion erforderliche Desinfektion der Ausgabeeinheit sowie in manchen Ausgestaltungen Rechenzeit. Insbesondere ist es nicht erforderlich, in das Referenz-Zeitfenster hinein zu "zoomen" und wieder zurück aus dem Referenz-Zeitfenster in den Gesamt-Zeitraum zu "zoomen". Vielmehr werden erfindungsgemäß mehrere Darstellungen mit mindestens zwei unterschiedlichen Zeitmaßstäben gleichzeitig auf derselben Anzeigeeinheit angezeigt. Außerdem zeigt die Positionierungs-Darstellung, die von dem Alarm-Referenz-Abschnitt bereitgestellt wird, wie das Referenz-Zeitfenster relativ zum Gesamt-Zeitraum positioniert ist. Eine solche Positionierungs-Darstellung wäre bei einem reinen Umschalten zwischen zwei unterschiedlichen Zeitmaßstäbe nicht vorhanden.

Die angesteuerte Ausgabeeinheit zeigt gleichzeitig eine Abfolge von Alarmen, die im Gesamt-Zeitraum aufgetreten sind, und eine Abfolge von Alarmen im Referenz-Zeitfenster an. Bevorzugt zeigt die Alarm-Gesamt-Abfolge mindestens jeden Alarm an, der in dem Gesamt-Zeitraum gezeigt wird. Der Alarm-Referenz-Abschnitt zeigt Alarme, bevorzugt alle Alarme, an, die im Referenz-Zeitfenster detektiert wurden. Dadurch lässt sich rasch erkennen, ob im Alarm-Referenz-Abschnitt außergewöhnlich viele oder außergewöhnlich wenige oder durchschnittlich viele Alarme - verglichen mit dem Gesamt-Zeitraum - aufgetreten sind.

Erfindungsgemäß zeigt die Ausgabeeinheit mit Hilfe der Positionierungs-Darstellung, wie das Referenz-Zeitfenster zeitlich relativ zum Gesamt-Zeitraum positioniert ist. Diese Positionierungs-Darstellung wird mithilfe des Alarm-Referenz-Abschnitts und der Alarm-Gesamt-Abfolge bereitgestellt, bevorzugt indem der Alarm-Referenz-Abschnitt zeitrichtig relativ zu der Alarm-Gesamt-Abfolge positioniert dargestellt ist, besonders bevorzugt indem derjenige Abschnitt der Alarm-Gesamt-Abfolge, der sich auf das Referenz-Zeitfenster bezieht, hervorgehoben wird. Beispielsweise wird ein Kasten um denjenigen Abschnitt der Alarm-Gesamt-Abfolge gelegt, der in das Referenz-Zeitfenster fällt, und zeigt damit den Alarm-Referenz-Abschnitt an. Die erfindungsgemäß bereitgestellte Positionierungs-Darstellung erleichtert es einem Betrachter, die oder jede Darstellung, die sich auf das Referenz-Zeitfenster bezieht, zeitlich einzuordnen und / oder mit der Alarm-Gesamt-Abfolge zu vergleichen. Außerdem zeigt der Alarm-Referenz-Abschnitt Alarme im Referenz-Zeitfenster, bevorzugt alle Alarme. Dank des Alarm-Referenz-Abschnittes ist es möglich, aber nicht erforderlich, zusätzlich zu den dargestellten Alarmen die relative zeitliche Positionierung in einer separaten Darstellung anzuzeigen oder numerische Zeitangaben darzustellen. Dies spart Platz ein verglichen mit einer separaten Darstellung der zeitlichen Positionierung.

Erfindungsgemäß vermag die Signalverarbeitungseinheit die Ausgabeeinheit so anzusteuern, dass die angesteuerte Ausgabeeinheit eine Signalverlaufs-Darstellung und / oder eine Alarm-Referenz-Abfolge sowie eine Alarm-Gesamt-Abfolge darstellt. Die Signalverlaufs-Darstellung zeigt den zeitlichen Verlauf mindestens eines Signals im Referenz-Zeitfenster. Die Alarm-Gesamt-Abfolge zeigt eine zeitliche Abfolge von Alarmen im Gesamt-Zeitraum. Diese gleichzeitig angezeigten Darstellungen erleichtert es einem Benutzer, eine Erklärung für einen Alarm und für dessen Entwicklung zu finden, und zwar ohne durch eine Benutzer-Interaktion zwischen verschiedenen Darstellungen umschalten zu müssen. Die Alarm-Referenz-Abfolge zeigt eine Abfolge von Alarmen im Referenz-Zeitfenster. Bevorzugt ist die Ausdehnung der Signalverlaufs-Darstellung und die Ausdehnung der Alarm-Referenz-Abfolge - gesehen in die Zeitachsen-Darstellungs-Richtung - mindestens so groß wie die Ausdehnung der Alarm-Gesamt-Abfolge.

Zusammenfassend bieten die erfindungsgemäße Anordnung und das erfindungsgemäße Verfahren eine höhere Ergonomie - verglichen mit Anordnungen und Verfahren, bei denen alle Alarme und zeitlichen Signalverläufe mit demselben Zeitmaßstab dargestellt werden, und auch verglichen mit Anordnungen und Verfahren, bei denen ein Benutzer durch eine Benutzer-Interaktion zwischen verschiedenen Darstellungen mit unterschiedlichen Zeitmaßstäben umschalten muss. Diese höhere Ergonomie fällt insbesondere bei einer relativ kleinen Ausgabeeinheit ins Gewicht. Die erfindungsgemäße Anordnung und das erfindungsgemäße Verfahren ersparen die Notwendigkeit, zwischen verschiedenen Darstellungen, insbesondere Darstellungen mit unterschiedlichen Zeitmaßstäben, umzuschalten. Auch diese Wirkung erhöht die Ergonomie.

Erfindungsgemäß ist das Referenz-Zeitfenster ein Ausschnitt des Gesamt-Zeitraums. In einer Ausgestaltung beträgt die Länge des Referenz-Zeitfensters höchstens 70 %, bevorzugt höchstens 50 %, besonders bevorzugt höchstens 35 %, insbesondere höchstens 10 %, der Länge des Gesamt-Zeitraums. Diese Ausgestaltung ermöglicht es einerseits, Alarme und / oder Signalverläufe, die im Referenz-Zeitfenster aufgetreten sind, relativ detailliert darzustellen, und andererseits, alle Alarme in der Alarm-Gesamt-Abfolge darzustellen. Außerdem lässt sich die zeitliche Positionierung eines im Verhältnis zum Gesamt-Zeitraum relativ kurzen Referenz-Zeitfensters darstellen. Dies alles wird gleichzeitig auf der Ausgabeeinheit angezeigt, ohne dass eine Benutzerinteraktion zum Umschalten erforderlich ist. Möglich, aber nicht erforderlich ist es, das Referenz-Zeitfenster durch Zahlenangaben darzustellen. Solche Zahlenangaben lassen sich nicht so schnell erfassen wie graphische Angaben.

Bevorzugt ist die zeitliche Positionierung des Referenz-Zeitfensters relativ zum Gesamt-Zeitraum veränderbar. Insbesondere lässt sich das Referenzzeitfenster so verschieben, dass zwischen dem Referenz-Zeitfenster und dem aktuellen Zeitpunkt ein zeitlicher Abstand auftritt. Bevorzugt werden der Alarm-Referenz-Abschnitt, die Alarm-Referenz-Abfolge und die Signalverlaufs-Darstellung automatisch an eine Veränderung des Referenz-Zeitfensters angepasst.

Erfindungsgemäß vermag die Signalverarbeitungseinheit die Ausgabeeinheit so anzusteuern, dass die angesteuerte Ausgabeeinheit die Signalverlaufs-Darstellung und / oder die Alarm-Referenz-Abfolge darstellt. In einer Ausgestaltung stellt die angesteuerte Ausgabeeinheit sowohl die Signalverlaufs-Darstellung als auch die Alarm-Referenz-Abfolge dar. Bevorzugt beziehen sich die Signalverlaufs-Darstellung und die Alarm-Referenz-Abfolge auf demselben feineren Zeitmaßstab und sind bevorzugt zeitrichtig zueinander positioniert. Möglich ist auch, dass die angesteuerte Ausgabeeinheit nur die Signalverlaufs-Darstellung oder nur die Alarm-Referenz-Abfolge darstellt. In einer anderen Ausgestaltung stellt die angesteuerte Ausgabeeinheit wahlweise die Signalverlaufs-Darstellung oder die Alarm-Referenz-Abfolge dar, beispielsweise abhängig von einer entsprechenden Benutzereingabe.

Bevorzugt werden die Signalverlaufs-Darstellung und die Alarm-Referenz-Abfolge gleichzeitig mit demselben feineren Zeitmaßstab dargestellt und erstrecken sich in dieselbe Zeitachsen-Darstellungs-Richtung. Besonders bevorzugt ist die Signalverlaufs-Darstellung auf der Ausgabeeinheit zeitrichtig relativ zur Alarm-Referenz-Abfolge positioniert. Diese gemeinsame und bevorzugt zeitrichtig positionierte Darstellung erleichtert es einem Benutzer, rasch zu erfassen, welche Signalwerte zu einem Alarm, der in der Alarm-Referenz-Abfolge gezeigt wird, geführt haben und wo dieser Alarm und diejenigen Signalwerte, die zu dem Alarm geführt haben, zeitlich positioniert sind.

Bevorzugt werden die Alarm-Gesamt-Abfolge und der Alarm-Referenz-Abschnitt mit demselben gröberen Zeitmaßstab dargestellt. Bevorzugt ist der Alarm-Referenz-Abschnitt so dargestellt, dass er zeitrichtig relativ zur Alarm-Gesamt-Abfolge positioniert ist. Dies erleichtert es einen Benutzer, die zeitliche Positionierung des Referenz-Zeitfensters relativ zum Gesamt-Zeitraum und die zeitliche Positionierung der Alarme im Referenz-Zeitfenster relativ zu den Alarmen im Gesamt-Zeitraum zu erfassen.

Erfindungsgemäß stellt die angesteuerte Ausgabeeinheit dar, wie der Alarm-Referenz-Abschnitt relativ zu der Alarm-Gesamt-Abfolge zeitlich positioniert ist, und zwar mit Hilfe der Positionierungs-Darstellung. In einer Ausgestaltung wird der Alarm-Referenz-Abschnitt als Teil der Alarm-Gesamt-Abfolge und bevorzugt in der Alarm-Gesamt-Abfolge hervorgehoben dargestellt, und dadurch wird die relative Positionierung dargestellt. Diese Ausgestaltung erfordert keinen zusätzlichen Platz auf der Ausgabeeinheit, um die zeitliche Positionierung darzustellen.

In einer anderen Ausgestaltung wird der Alarm-Referenz-Abschnitt getrennt von der Alarm-Gesamt-Abfolge dargestellt, ist aber auch in dieser anderen Ausgestaltung bevorzugt zeitrichtig positioniert, und beide Darstellungen während gleichzeitig und gemeinsam angezeigt. Durch die zeitrichtige Positionierung wird die relative zeitliche Positionierung gezeigt. Möglich ist auch, dass die angesteuerte Ausgabeeinheit eine Zeitachse für den Gesamt-Zeitraum auf der Ausgabeeinheit darstellt und in dieser Zeitachse das Referenz-Zeitfenster markiert darstellt.

Erfindungsgemäß zeigt die Ausgabeeinheit, wie der Alarm-Referenz-Abschnitt relativ zu der Alarm-Gesamt-Abfolge zeitlich positioniert ist. Bevorzugt ist diese Darstellung der zeitlichen Positionierung eine grafische Darstellung. Dies erspart es einem Benutzer, numerische Zeitangaben zu lesen und im Kopf auswerten und / oder bewerten zu müssen. Mithilfe der grafischen Positionierungs-Darstellung lässt sich die zeitliche Positionierung des Alarm-Referenz-Abschnitts relativ zu der Alarm-Gesamt-Abfolge von einem Benutzer rascher und intuitiver als andere denkbare Darstellungen erfassen.

Erfindungsgemäß vermag die Signalverarbeitungseinheit die Ausgabeeinheit so anzusteuern, dass die angesteuerte Ausgabeeinheit mindestens zwei zeitliche Abfolgen von Alarmen darstellt, nämlich eine Alarm-Gesamt-Abfolge und einen Alarm-Referenz-Abschnitt. Die Alarm-Gesamt-Abfolge zeigt die Alarme, die im Gesamt-Zeitraum aufgetreten sind, und der Alarm-Referenz-Abschnitt zeigt diejenigen Alarme der Alarm-Gesamt-Abfolge, die im Referenz-Zeitfenster aufgetreten sind. Bevorzugt werden die Alarm-Gesamt-Abfolge und der Alarm-Referenz-Abschnitt mit demselben gröberen Zeitmaßstab dargestellt. Besonders bevorzugt stellt die angesteuerte Ausgabeeinheit den Alarm-Referenz-Abschnitt als einen Abschnitt der Alarm-Gesamt-Abfolge dar, beispielsweise hervorgehoben in der Alarm-Gesamt-Abfolge. Diese Ausgestaltung spart Platz auf der Ausgabeeinheit ein - verglichen mit einer Darstellung, bei welcher der Alarm-Referenz-Abschnitt räumlich getrennt von der Alarm-Gesamt-Abfolge dargestellt wird. Möglich ist aber auch, dass der Alarm-Referenz-Abschnitt getrennt von der Alarm-Gesamt-Abfolge angezeigt wird.

Bevorzugt umfasst die Anordnung weiterhin eine Eingabeeinheit, welche Benutzereingaben zu erfassen vermag, beispielsweise einen Touchscreen. Mithilfe dieser Eingabeeinheit vermag ein Benutzer insbesondere einen angezeigten Alarm auszuwählen und das Referenz-Zeitfenster zu verändern. Insbesondere vermag der Benutzer das Referenz-Zeitfenster im Gesamt-Zeitraum hin und her zu schieben und an einen gewünschten Zeitpunkt zu positionieren.

In einer Fortbildung dieser Ausgestaltung vermag die Signalverarbeitungseinheit die Auswahl eines angezeigten Alarms durch einen Benutzer zu erfassen. Möglich ist, dass dieser angezeigten Alarm zwar im Gesamt-Zeitraum, aber nicht im Referenz-Zeitfenster liegt. Nach Auswahl eines Alarms vermag die Signalverarbeitungseinheit die Ausgabeeinheit so anzusteuern, dass das Referenz-Zeitfenster automatisch verschoben wird und der ausgewählte Alarm nunmehr im Referenz-Zeitfenster liegt.

Bevorzugt vermag der Benutzer einen Alarm auszuwählen, der in der Alarm-Gesamt-Abfolge und / oder in dem Alarm-Referenz-Abschnitt oder in der Alarm-Referenz-Abfolge gezeigt wird. Nach Auswahl eines Alarms vermag die angesteuerte Ausgabeeinheit mindestens eine Information über den ausgewählten Alarm darzustellen. Dargestellt wird beispielsweise mindestens eine der folgenden Informationen:
- eine textliche Beschreibung eines Alarm-Kriteriums, das als erfüllt detektiert worden ist und zu dem Alarm geführt hat,
- derjenige Zeitpunkt, an dem der Alarm detektiert wurde,
- eine Zeitdauer, während der dieser Alarm vorlag,
- eine Kennzeichnung einer Relevanz des Alarms,
- mindestens ein Signalwert, der zu dem Alarm geführt hat,
- ein vorgegebener Sollbereich für das Signal, das zu dem Alarm geführt hat, wobei der Alarm bevorzugt durch einen Signalwert außerhalb dieses Sollbereichs ausgelöst wird.

Erfindungsgemäß wird mindestens ein Alarm-Kriterium vorgegeben. Bevorzugt werden mindestens zwei unterschiedliche Alarm-Kriterien vorgegeben. Jedes Alarm-Kriterium definiert jeweils eine Alarm-Art. Dasselbe Alarm-Kriterium kann wiederholt erfüllt sein, nämlich zu unterschiedlichen Zeitpunkten. In diesem Falle sind nacheinander mehrere gleichartige Alarme detektiert. Insgesamt werden also mindestens zwei verschiedene Alarm-Arten definiert. Wenn ein vorgegebenes Alarm-Kriterium erfüllt und detektiert ist, so ist ein Alarm der zugeordneten Alarm-Art eingetreten und detektiert.

Die Signalverarbeitungseinheit vermag die Auswahl eines Alarms durch einen Benutzer zu erfassen. Nachdem die Signalverarbeitungseinheit die Auswahl eines Alarms erfasst hat, vermag sie die Ausgabeeinheit so anzusteuern, dass die angesteuerte Ausgabeeinheit folgendes anzeigt: In der Alarm-Gesamt-Abfolge und / oder in dem Alarm-Referenz-Abschnitt und / oder in der Alarm-Referenz-Abfolge wird jeder weitere Alarm, der zu der gleichen Alarm-Art wie der ausgewählte Alarm gehört, im Vergleich zu den übrigen dargestellten Alarmen hervorgehoben dargestellt.

In einer bevorzugten Ausgestaltung behält die Signalverarbeitungseinheit die Auswahl eines Alarms so lange bei, bis sie die Auswahl eines anderen Alarms erfasst hat. Diese Auswahl des Alarms wird bevorzugt insbesondere auch dann beibehalten, wenn das Referenz-Zeitfenster oder ein weiter unten beschriebener Referenz-Zeitpunkt aufgrund einer Benutzereingabe verändert wird, besonders bevorzugt auch dann, wenn der ausgewählte Alarm vor Verschiebung des Referenz-Zeitfensters im Referenz-Zeitfenster liegt und danach nicht mehr.

Erfindungsgemäß steuert die Signalverarbeitungseinheit die Ausgabeeinheit so an, dass die angesteuerte Ausgabeeinheit eine Signalverlaufs-Darstellung, einen Alarm-Referenz-Abschnitt und / oder eine Alarm-Referenz-Abfolge darstellt, die sich alle auf das Referenz-Zeitfenster beziehen. Dieses Referenz-Zeitfenster ist ein Abschnitt, also ein Teil, des Gesamt-Zeitraums. Die dargestellte Alarm-Gesamt-Abfolge bezieht sich auf den Gesamt-Zeitraum. In einer bevorzugten Ausgestaltung vermag die Signalverarbeitungseinheit eine Benutzereingabe zu erfassen, um das Referenz-Zeitfenster zu verändern, insbesondere um es zu verschieben oder um seine Länge zu verändern. Indem der Benutzer durch eine Benutzereingabe veranlasst, dass die zeitliche Länge des Referenz-Zeitfensters verändert wird, wird bevorzugt auch der oder jeder feinere Zeitmaßstab verändert, insbesondere wenn die räumliche Ausdehnung des Referenz-Zeitfensters gleich bleibt. Möglich ist auch, dass der Zeitmaßstab gleich bleibt und die räumliche Ausdehnung an die Veränderung der zeitlichen Länge angepasst wird. Bei einer bloßen Verschiebung des Referenz-Zeitfensters bleibt der oder jeder feinere Zeitmaßstab hingegen unverändert. Nachdem die Signalverarbeitungseinheit die geforderte Veränderung des Referenz-Zeitfensters erfasst hat, steuert die Signalverarbeitungseinheit die Ausgabeeinheit an. Die entsprechend angesteuerte Ausgabeeinheit passt den Alarm-Referenz-Abschnitt sowie die Signalverlaufs-Darstellung und / oder die Alarm-Referenz-Abfolge automatisch an das veränderte Referenz-Zeitfenster an.

Zumindest dann, wenn auch das veränderte Referenz-Zeitfenster vollständig in dem Gesamt-Zeitraum liegt, lässt die angesteuerte Ausgabeeinheit die dargestellte Alarm-Gesamt-Abfolge unverändert. Falls das veränderte Referenz-Zeitfenster nicht vollständig im Gesamt-Zeitraum liegt, so verändert die Signalverarbeitungseinheit hingegen den Gesamt-Zeitraum und / oder das Referenz-Zeitfenster so, dass danach wieder das Referenz-Zeitfenster vollständig im Gesamt-Zeitraum liegt, und passt die dargestellte Alarm-Gesamt-Abfolge entsprechend an. Oder die Signalverarbeitungseinheit veranlasst, dass eine Fehlermeldung ausgegeben wird.

In einer bevorzugten Ausgestaltung zeigt die angesteuerte Ausgabeeinheit zusätzlich einen Referenz-Zeitpunkt an, der im Referenz-Zeitfenster liegt. Dieser Referenz-Zeitpunkt wird in der Signalverlaufs-Darstellung und / oder in dem Alarm-Referenz-Abschnitt und / oder in der Alarm-Referenz-Abfolge dargestellt. Bevorzugt zeigt die angesteuerte Ausgabeeinheit zusätzlich den Wert mindestens eines Signals zu diesem Referenz-Zeitpunkt, besonders bevorzugt den jeweiligen Wert mindestens eines oder sogar jedes in der Signalverlaufs-Darstellung dargestellten Signals.

Die Signalverarbeitungseinheit vermag eine Benutzereingabe zu erfassen, mit welcher ein Benutzer den dargestellten Referenz-Zeitpunkt verändert, insbesondere verschiebt. Diese Benutzereingabe kann die numerische Eingabe eines Zeitpunkts umfassen oder auch den Schritt, ein auf einem Bildschirm dargestelltes Symbol für den Referenz-Zeitpunkt zu verschieben. Als Reaktion auf eine derartige Benutzereingabe vermag die Signalverarbeitungseinheit die Ausgabeeinheit anzusteuern. Die als Reaktion angesteuerte Ausgabeeinheit stellt in der Signalverlaufs-Darstellung und / oder in dem Alarm-Referenz-Abschnitt und / oder in der Alarm-Referenz-Abfolge den veränderten Referenz-Zeitpunkt dar und bevorzugt den oder jeden Signalwert am veränderten Referenz-Zeitpunkt.

Falls der veränderte Referenz-Zeitpunkt außerhalb desjenigen Referenz-Zeitfensters liegt, das vor der Veränderung des Referenz-Zeitpunkts verwendet wurde, so verändert die Signalverarbeitungseinheit bevorzugt zusätzlich das Referenz-Zeitfenster dergestalt, dass der entsprechend der Benutzereingabe veränderte Referenz-Zeitpunkt im veränderten Referenz-Zeitfenster liegt. In einer anderen Ausgestaltung legt sie den Referenz-Zeitpunkt an eine Grenze des unverändert belassenen Referenz-Zeitfensters. In einer weiteren Ausgestaltung veranlasst die Signalverarbeitungseinheit, dass eine Fehlermeldung ausgegeben wird. Der Benutzer kann daraufhin das Referenz-Zeitfenster oder den Referenz-Zeitpunkt verändern.

In einer Fortbildung dieser Ausgestaltung prüft die Signalverarbeitungseinheit, ob an dem veränderten Referenz-Zeitpunkt ein Alarm aufgetreten ist und detektiert worden ist. Falls an dem veränderten Referenz-Zeitpunkt ein Alarm aufgetreten und detektiert worden ist, so verwendet die Signalverarbeitungseinheit diesen Alarm als den ausgewählten Alarm. Nicht erforderlich ist es, diesen Alarm direkt auszuwählen.

In einer Abwandlung dieser Ausgestaltung vermag ein Benutzer zunächst einen Alarm auszuwählen. Die erfindungsgemäße Anordnung vermag diese Auswahl eines Alarms durch den Benutzer zu erfassen. Der Schritt, dass die Auswahl eines Alarms erfasst ist, löst den Schritt aus, dass der Zeitpunkt, an dem dieser Alarm aufgetreten ist, als der Referenz-Zeitpunkt verwendet wird. Falls der ausgewählte Alarm zuvor außerhalb des Referenz-Zeitfensters lag, wird das Referenz-Zeitfenster so verschoben, dass der ausgewählte Alarm nunmehr im Referenz-Zeitfenster liegt. Außerdem wird bevorzugt der jeweilige Wert mindestens eines Signals der Signalverlaufs-Darstellung an diesem Referenz-Zeitpunkt dargestellt. Diese Ausgestaltung ermöglicht es einem Benutzer, sich mit einer einzigen Interaktion einen Überblick über eine Situation zum Zeitpunkt des Alarms zu verschaffen.

Die Ausgestaltung, bei der sich ein Alarm auswählen lässt, lässt sich kombinieren mit der Ausgestaltung, bei der sich ein Referenz-Zeitpunkt auswählen lässt. Dadurch werden dem Benutzer zwei unterschiedliche Möglichkeiten der Interaktion gegeben.

Erfindungsgemäß stellt die angesteuerte Ausgabeeinheit eine Alarm-Gesamt-Abfolge dar, die sich auf einen Gesamt-Zeitraum bezieht, sowie einen Alarm-Referenz-Abschnitt und optional eine Alarm-Referenz-Abfolge, die sich auf ein Referenz-Zeitfenster beziehen, dar. Bevorzugt stellt die angesteuerte Ausgabeeinheit in der Alarm-Gesamt-Abfolge und / oder im Alarm-Referenz-Abschnitt und / oder in der Alarm-Referenz-Abfolge jeden Alarm mithilfe jeweils eines Symbols dar. Diese Ausgestaltung spart Platz ein verglichen mit einer textlichen Beschreibung des Alarms und ermöglicht es einem Benutzer rascher, die dargestellte Situation zu erfassen.

In einer Fortbildung dieser Ausgestaltung ist jedem vorgegebenen Alarm-Kriterium und somit jeder möglichen Alarm-Art jeweils ein vorgegebenes Symbol zugeordnet. Die angesteuerte Ausgabeeinheit stellt als Symbol für einen Alarm dasjenige Symbol dar, welches dem Alarm-Kriterium und somit der Alarm-Art dieses Alarms zugeordnet ist. Mehrere gleichartige Alarme unterscheiden sich durch den jeweiligen Zeitpunkt des Auftretens.

In einer Ausgestaltung ist jeder Alarm-Art ein anderes Symbol zugeordnet. Eine abweichende Ausgestaltung, welche in vielen Fällen übersichtlicher ist, ist diese: Jedem Alarm-Kriterium ist jeweils eine Relevanz zugeordnet. Jeder Relevanz ist jeweils ein Symbol zugeordnet, verschiedenen Relevanzen hingegen unterschiedliche Symbole. Unterschiedlichen Alarm-Kriterien gleicher Relevanz ist bei dieser bevorzugten Ausgestaltung daher das gleiche Symbol zugeordnet. Auf der Ausgabeeinheit werden die Alarme durch die Symbole der Alarm-Arten zeitrichtig angezeigt. Diese Ausgestaltung reduziert die Zahl erforderlicher Symbole - verglichen mit einer Ausgestaltung, bei der jedem Alarm-Kriterium und somit jeder Alarm-Art jeweils ein eigenes spezielles Symbol zugeordnet ist. Außerdem lassen sich relevante Alarme rascher erkennen. In einer Ausgestaltung zeigt die angesteuerte Ausgabeeinheit abhängig von einer Benutzereingabe entweder die Symbole für die Relevanzen oder die Symbole für die Alarm-Arten an.

Erfindungsgemäß stellt die angesteuerte Ausgabeeinheit eine Alarm-Gesamt-Abfolge dar, die sich auf einen Gesamt-Zeitraum bezieht, sowie einen Alarm-Referenz-Abschnitt und optional eine Alarm-Referenz-Abfolge, die sich auf ein Referenz-Zeitfenster beziehen, dar. Bevorzugt stellt die angesteuerte Ausgabeeinheit zusätzlich eine Alarm-Beschreibungs-Abfolge dar. Diese Alarm-Beschreibungs-Abfolge umfasst jeweils eine textliche Alarm-Beschreibung pro Alarm einer Sequenz von Alarmen. Diese Alarm-Sequenz gehört zu der zeitlichen Abfolge von Alarmen, die in der Alarm-Gesamt-Abfolge dargestellt wird, bevorzugt zu einer Sequenz der Alarm-Referenz-Abfolge. Die textliche Alarm-Beschreibung erstreckt sich in eine Listen-Richtung. Diese Listen-Richtung steht bevorzugt senkrecht auf der Zeitachsen-Darstellungs-Richtung, beispielsweise von oben nach unten. Die jeweilige Schreibrichtung jeder textlichen Alarm-Beschreibung in der Alarm-Beschreibungs-Abfolge steht senkrecht auf der Listen-Richtung und bei einer zweidimensionalen Darstellung daher parallel zu der Zeitachsen-Darstellungs-Richtung.

Bevorzugt umfasst die textliche Alarm-Beschreibung eines Alarms mindestens eine der folgenden Informationen:
- eine textliche Beschreibung einer Alarm-Art, zu der dieser Alarm gehört,
- ein Symbol für diese Alarm-Art,
- denjenigen Zeitpunkt, an dem der Alarm detektiert wurde,
- eine Zeitdauer, während der dieser Alarm vorlag,
- eine Kennzeichnung einer Relevanz des Alarms,
- mindestens einen Signalwert, der zu dem Alarm geführt hat.

Gemäß der gerade beschriebenen Ausgestaltung umfasst die dargestellte Alarm-Beschreibungs-Abfolge jeweils eine textliche Alarm-Beschreibung pro Alarm einer Sequenz von Alarmen. Bevorzugt vermag die Signalverarbeitungseinheit eine Benutzereingabe zu erfassen, gemäß der die Sequenz von Alarmen, deren Alarm-Beschreibungen in der Alarm-Beschreibungs-Abfolge angezeigt werden, verändert werden soll, d.h. dass eine andere Alarm-Sequenz angezeigt werden soll. Nach Erfassen einer solchen Veränderung vermag die Signalverarbeitungseinheit die Ausgabeeinheit so anzusteuern, dass die angesteuerte Ausgabeeinheit die Alarm-Beschreibungen für die Alarme der veränderten Sequenz darstellt, bevorzugt wiederum in die Listen-Richtung.

In einer Fortbildung der Ausgestaltung mit der Alarm-Beschreibungs-Abfolge vermag die Signalverarbeitungseinheit die Auswahl einer Alarm-Beschreibung zu erfassen, wobei die ausgewählte Alarm-Beschreibung in der Alarm-Beschreibungs-Abfolge dargestellt wird. Diese ausgewählte Alarm-Beschreibung gehört zu einem Alarm, der im Gesamt-Zeitraum detektiert worden ist, bevorzugt zu einem Alarm im Referenz-Zeitfenster. Bevorzugt verwendet die Signalverarbeitungseinheit den Alarm, auf den sich die ausgewählte Alarm-Beschreibung bezieht, als den ausgewählten Alarm. Insbesondere stellt sie jeden weiteren Alarm, der zu der gleichen Alarm-Art wie der ausgewählte Alarm gehört, im Vergleich zu den übrigen dargestellten Alarmen hervorgehoben dar.

In einer weiteren Fortbildung der Ausgestaltung mit der Alarm-Beschreibungs-Abfolge stellt die Signalverarbeitungseinheit die Alarm-Referenz-Abfolge mit Alarmen, die im Referenz-Zeitfenster detektiert worden sind, sowie einen Korrelationsanzeiger dar. Der Korrelationsanzeiger umfasst ein führendes Element und ein geführtes Element.

In einer ersten Alternative dieser Fortbildung bezieht das führende Element sich auf eine Alarm-Beschreibung in der Alarm-Beschreibungs-Abfolge. Das geführte Element bezieht sich auf denjenigen Alarm in der Alarm-Referenz-Abfolge und / oder im Alarm-Referenz-Abschnitt, auf den sich diese Alarm-Beschreibung bezieht. In einer zweiten Alternative dieser Fortbildung bezieht das führende Element sich auf einen Alarm in der Alarm-Referenz-Abfolge und / oder im Alarm-Referenz-Abschnitt und das geführte Element auf diejenige Alarm-Beschreibung in der Alarm-Beschreibungs-Abfolge, die sich auf diesen Alarm bezieht.

Diese Ausgestaltung erleichtert es, zu einem Alarm in der Alarm-Referenz-Abfolge die zugehörige Alarm-Beschreibung oder umgekehrt zu einer Alarm-Beschreibung den zugehörigen Alarm in der Alarm-Referenz-Abfolge zu finden. Wenn beispielsweise aufgrund einer Benutzereingabe das führende Element auf eine andere Alarm-Beschreibung oder auf einen anderen Alarm zeigt, so wird das geführte Element entsprechend mitgeführt.

Erfindungsgemäß stellt die angesteuerte Ausgabeeinheit eine Signalverlaufs-Darstellung und / oder eine Alarm-Referenz-Abfolge dar, die sich beide auf das Referenz-Zeitfenster beziehen. Die Signalverarbeitungseinheit prüft automatisch, ob ein vorgegebenes Alarm-Kriterium erfüllt ist. In einer bevorzugten Ausgestaltung steuert die Signalverarbeitungseinheit die Ausgabeeinheit so an, dass die Ausgabeeinheit folgendes darstellt: Falls ein in der Signalverlaufs-Darstellung dargestellter Signalverlauf im Referenz-Zeitfenster mindestens ein vorgegebenes Alarm-Kriterium erfüllt, so hebt die Ausgabeeinheit in der Darstellung denjenigen Abschnitt des dargestellten Signalverlaufs und / oder denjenigen Zeitabschnitt im Gesamt-Zeitraum und / oder im Referenz-Zeitfenster hervor, der dazu führt, dass dieses Alarm-Kriterium erfüllt ist. Beispielsweise stellt die Ausgabeeinheit denjenigen Abschnitt des Signalverlaufs hervorgehoben dar, der außerhalb eines vorgegebenen Sollbereichs für dieses Signal liegt, und oder denjenigen Abschnitt des Referenz-Zeitfensters, in dem die Werte des Signals außerhalb des Sollbereichs liegen. Dieser Sollbereich kann vorab vorgegeben sein oder aber zeitlich veränderlich sein und von der Signalverarbeitungseinheit berechnet worden sein.

Diese Ausgestaltung erleichtert es einem Benutzer, einen dargestellten Alarm genauer zu untersuchen, ohne dass notwendigerweise eine textliche Beschreibung auf der Ausgabeeinheit dargestellt werden muss. Diese Ausgestaltung erfordert - im Unterschied zu einer textlichen Beschreibung - in vielen Fällen keinen zusätzlichen Platz auf der Ausgabeeinheit.

Die erfindungsgemäße Anordnung umfasst eine Signalverarbeitungseinheit und eine Ausgabeeinheit. In einer Ausgestaltung ist diese Signalverarbeitungseinheit auf zwei Signalverarbeitungsgeräte aufgeteilt, die bevorzugt räumlich voneinander entfernt sind und die miteinander durch eine Datenverbindung verbunden sind. Das erste Signalverarbeitungsgerät ist dazu ausgestaltet, Messwerte von den Patienten-Sensoren zu empfangen, mindestens ein Signal zu erzeugen, zu prüfen, ob ein Alarm-Kriterium erfüllt ist, und Alarme zu detektieren. Das zweite Signalverarbeitungsgerät ist dazu ausgestaltet, vom ersten Signalbearbeitungsgerät die Informationen über die Signale und Alarmverläufe zu empfangen und die Ausgabeeinheit anzusteuern.

Bevorzugt ist das erste Signalverarbeitungsgerät ein Bestandteil eines medizinischen Geräts oder diesem medizinischen Gerät zugeordnet, und die detektierten Alarme beziehen sich auf einen Patienten, der zeitweise mit diesem medizinischen Gerät verbunden ist. Das zweite Signalverarbeitungsgerät ist räumlich von dem medizinischen Gerät und dem ersten Signalverarbeitungsgerät getrennt und steht wenigstens zeitweise mit dem ersten Signalverarbeitungsgerät in einer Datenverbindung. Möglich ist, dass das erste Signalverarbeitungsgerät zusätzlich eine Ausgabeeinheit des medizinischen Geräts ansteuert, bevorzugt so, dass die Ausgabeeinheit des medizinischen Geräts so wie oben beschrieben arbeitet. Möglich ist, dass das zweite Signalverarbeitungsgerät mit mehreren ersten Signalbearbeitungsgeräten verbunden ist, besonders bevorzugt solchen von unterschiedlichen medizinischen Geräten. Das zweite Signalverarbeitungsgerät und / oder die vom zweiten Signalbearbeitungsgerät angesteuerte Ausgabeeinheit sind beispielsweise in einer Zentrale angeordnet.

In einer Fortbildung dieser Ausgestaltung gehört die Anordnung zu einem System mit mindestens zwei medizinischen Geräten, die über ein Datennetzwerk wenigstens zeitweise miteinander verbunden sind. Mindestens zwei dieser medizinischen Geräte umfassen jeweils ein erstes Signalverarbeitungsgerät, welches so wie gerade beschrieben ausgestaltet ist. Jedes erste Signalverarbeitungsgerät bewirkt, dass Nachrichten über die Alarme und deren Zeitpunkte an das zweite Signalverarbeitungsgerät übermittelt werden. Beispielsweise hat jedes erste Signalverarbeitungsgerät wenigstens zeitweise Schreibzugriff auf denselben zentralen Datenspeicher und schreibt Informationen über die Alarme, die es detektiert hat, in diesen zentralen Datenspeicher.

Das zweite Signalverarbeitungsgerät steht mit diesen beiden ersten Signalverarbeitungsgeräten in jeweils einer Datenverbindung, beispielsweise indem das zweite Signalverarbeitungsgerät wenigstens zeitweise Lesezugriff auf den zentralen Datenspeicher aufweist und Informationen über Alarme einliest. Das zweite Signalverarbeitungsgerät steuert die Ausgabeeinheit so an, dass die Ausgabeeinheit wahlweise eingelesene Alarme und optional weitere Patientendaten von dem einen medizinischen Gerät oder von dem anderen medizinischen Gerät anzeigt. Möglich ist auch, dass die angesteuerte Ausgabeeinheit gleichzeitig Alarme von beiden medizinischen Geräten anzeigt.

Das zweite Signalverarbeitungsgerät und die Ausgabeeinheit fungieren somit als ein zentrales System, um mehrere erste medizinische Geräte zu überwachen.

Im Folgenden wird die Erfindung anhand mehrerer Ausführungsbeispiele beschrieben. Hierbei zeigt:
- Figur 1: schematisch einen Patienten, der wenigstens zeitweise künstlich beatmet wird, ein Beatmungsgerät und die verwendeten Patienten-Sensoren;
- Figur 2: eine anfängliche Unterteilung des Bildschirms des medizinischen Geräts;
- Figur 3: die Alarm-Übersichts-Darstellung im unteren Bereich des Bildschirms in vergrößerter Form vor Auswahl eines Alarms;
- Figur 4, Figur 5: eine Reaktion auf die Auswahl eines im Referenz-Zeitfenster liegenden Alarms in der Alarm-Referenz-Abfolge und eine Reaktion auf diese Auswahl;
- Figur 6: die Alarm-Übersichts-Darstellung von Figur 3 nach Auswahl eines Alarms;
- Figur 7, Figur 8, Figur 9: wie das Referenz-Zeitfenster verschoben wird und ein vor dem Referenz-Zeitfenster liegender Alarm ausgewählt wird;
- Figur 10, Figur 11,: wie die Alarm-Beschreibungen eingeblendet werden;
- Figur 12: in einer vergrößerten Darstellung mehrere Alarm-Beschreibungen;
- Figur 13: wie ein Alarm ausgewählt wird, wenn der zeitliche Verlauf desjenigen Signals, auf das sich der Alarm bezieht, nicht dargestellt wird;
- Figur 14: eine Reaktion auf die Auswahl eines im Referenz-Zeitfenster liegenden Alarms in der Alarm-Beschreibungs-Abfolge;
- Figur 15, Figur 16, Figur 17: eine abweichende Ausgestaltung der Alarm-Beschreibungs-Abfolge;
- Figur 18, Figur 19: eine Reaktion auf die Auswahl eines vor dem Referenz-Zeitfenster liegenden Alarms in der Alarm-Beschreibungs-Abfolge;
- Figur 20, Figur 21, Figur 22: wie ein Korrelationsanzeiger verwendet wird, um einen Alarm in der Alarm-Referenz-Abfolge anzuzeigen;
- Figur 23, Figur 24: wie ein noch früher detektierter Alarm angezeigt wird;
- Figur 25, Figur 26: eine andere Art der Benutzer-Interaktion, um das Referenz- Zeitfenster zu verschieben und um sich früher detektierte Alarme anzeigen zu lassen;
- Figur 27, Figur 28: eine alternative Ausgestaltung, um das Referenz-Zeitfenster zu verschieben und um früher detektierte Alarme anzeigen zu lassen;
- Figur 29, Figur 30: eine andere Art der Benutzer-Interaktion, um einen Alarm auszuwählen;
- Figur 31, Figur 32, Figur 33, Figur 34: eine weitere Art der Benutzer-Interaktion, um den Referenz- Zeitpunkt zu verschieben;
- Figur 35, Figur 36: wie Erläuterungen zu einem Alarm angezeigt werden;
- Figur 37, Figur 38, Figur 39: wie ein weiteres Signal ausgewählt wird und wie dessen zeitlicher Verlauf zusätzlich dargestellt wird;
- Figur 40: eine weitere Ausführungsform mit einem kleineren Bildschirm: Situation vor Auswahl eines Alarms;
- Figur 41: Ausführungsform nach Figur 40: aktueller Alarm wird angezeigt;
- Figur 42: Ausführungsform nach Figur 40: Alarm-Beschreibungs-Abfolge wird angezeigt;
- Figur 43: Ausführungsform nach Figur 40: Waveform-Snippet-Ansicht wird angezeigt;
- Figur 44: Ausführungsform nach Figur 40: geglättete Signalverläufe werden angezeigt;
- Figur 45: Ausführungsform nach Figur 40: Signalverläufe werden numerisch angezeigt;
- Figur 46: Ausführungsform nach Figur 40: ein Filter für Alarme lässt sich setzen;
- Figur 47: ein beispielhaftes System mit zwei medizinischen Geräten und einer zentralen Signalverarbeitungseinheit.

Im Ausführungsbeispiel wird die Erfindung für ein Beatmungsgerät mit einem Bildschirm und einer Signalverarbeitungseinheit eingesetzt.

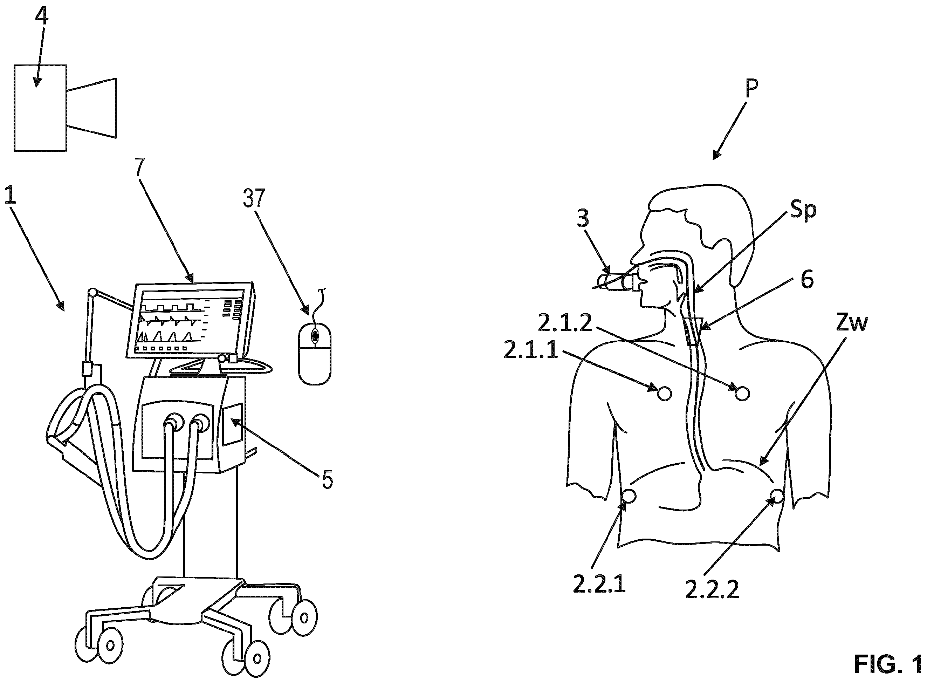

Figur 1 zeigt einen Patienten P mit einer Speiseröhre Sp und einem Zwerchfell Zw, wobei der Patient P von einem Beatmungsgerät 1 künstlich beatmet wird und mit einem Verbindungsstück 3 vor seinem Mund verbunden ist.

Auf der Haut des Patienten P sind ein erster Satz 2.1.1 und 2.1.2 von Messelektroden nahe dem Herzen des Patienten P sowie ein zweiter Satz 2.2.1 und 2.2.2 von Messelektroden nahe dem Zwerchfell Zw positioniert, außerdem eine nicht gezeigte Elektrode für Masse. Aus den Messwerten der Messelektroden 2.1.1 bis 2.2.2 und denen der Masse-Elektrode lassen sich ein elektrisches respiratorisches Signal und / oder ein elektrisches kardiogenes Signal herleiten, welche die Aktivität der Atmungsmuskulatur bzw. die Aktivität der Herzmuskulatur des Patienten P beschreiben.

Optional befindet sich in der Speiseröhre Sp und nahe des Zwerchfells Zw ein pneumatischer Sensor 6, z.B. eine Sonde oder ein Ballon. Aus den Messwerten dieses pneumatischen Sensors 6 lässt sich ein pneumatisches Signal herleiten, welches den Druck Pₑₛ (pressure in esophagus) in der Speiseröhre Sp beschreibt und mit dem Druck im Atemweg korreliert. Aus Messwerten eines weiteren bevorzugt pneumatischen Sensors, der beispielsweise im Beatmungsgerät 1 angeordnet ist, lässt sich der Atemwegsdruck P_{aw} (pressure in airway) an dem Verbindungsstück 3 herleiten. Optional misst ein optischer Sensor 4 die Geometrie des Körpers des Patienten P. Aus Messwerten des optischen Sensors 4, also aus der gemessenen Körper-Geometrie, lässt sich ein Maß für den zeitlich veränderlichen Füllstand der Lunge des Patienten P herleiten.

Figur 1 zeigt ein Beatmungsgerät 1 umfassend ein Verbindungsstück 3 und eine Ausgabeeinheit mit einem berührungssensitiven Bildschirm 7, der Informationen in visueller Form an einen Benutzer auszugeben vermag. Dieses Beatmungsgerät 1 führt die künstliche Beatmung des Patienten P durch. Außerdem wird eine optionale zusätzliche Eingabeeinheit mit einer DV-Maus 37 gezeigt.

Eine datenverarbeitende Signalverarbeitungseinheit 5 des Beatmungsgeräts 1 empfängt Messwerte von den Sensoren 2.1.1 bis 2.2.2, 3, 4, 6, berechnet unter Verwendung von diesen Messwerten patientenbezogene Signale und veranlasst, dass ausgewählte Signale auf dem Bildschirm 7 dargestellt werden. Die Signalverarbeitungseinheit 5 steuert einen Prozessor für den Bildschirm 7 an und veranlasst dadurch, dass auf dem Bildschirm 7 die zeitlichen Verläufe von verschiedenen Signalen und weitere Informationen dargestellt werden. Der Bildschirm 7 und dieser Prozessor gehören zu einer Ausgabeeinheit des Ausführungsbeispiels.

Beispiele für derartige patientenbezogene Signale sind die folgenden Signale:
- VT ("ventilation", Tidalvolumen, das ist die Menge von Atemluft, die während eines Atemzugs bei einem Einatmen in die Lunge des Patienten P fließt, in [ml],
- MV ("minute volume", die Menge der pro Zeiteinheit in die Lunge zugeführten Atemluft, in [Liter/min]) ,
- RR ("Respiratory Rate", die Atemfrequenz des Patienten P, die bei ausschließlich künstlicher Beatmung am Beatmungsgerät 1 vorgegeben und / oder gemessen wird und dann, wenn der Patient P selber atmet, bevorzugt gemessen wird, indem gezählt wird, wie oft der Atemfluss seine Richtung ändert),
- HR ("Heart Rate", Herzschlagfrequenz, beispielsweise gemessen als Anzahl der R-Spitzen eines elektrischen kardiogenen Signals oder EMG-Signals pro Minute, in [1/min]), und
- SpO2, der Sauerstoffgehalt im Blut, wird pulsoymetrisch gemessen.

Die Signalverarbeitungseinheit 5 empfängt Messwerte von Sensoren, beispielsweise von den in Figur 1 gezeigten Sensoren 2.1.1 bis 2.2.2, 3, 4, 6, und erzeugt aus Messwerten patientenbezogene Signale. Das Signal VT (Menge von Atemluft) wird berechnet, indem die Signalverarbeitungseinheit 5 über mehrere Messwerte integriert, welche den Fluss von Atemluft zu verschiedenen Zeitpunkten während eines Atemzugs beschreiben. Das Signal MV (Menge der zugeführten Atemluft) wird aus dem Signal VT berechnet, beispielsweise durch eine geeignete Mittelung oder aus einem Signalabschnitt des Signals VT von 1 min Länge.

Der Bildschirm 7 kann ein Bestandteil des Beatmungsgeräts 1 sein oder auch räumlich von dem Beatmungsgerät 1 getrennt sein und beispielsweise zu einem Smartphone oder sonstigen tragbaren Gerät gehören. Auch die Signalverarbeitungseinheit 5 kann räumlich von dem Beatmungsgerät 1 getrennt sein und z.B. zu dem tragbaren Gerät gehören.

Ein Benutzer kann Eingaben vornehmen und dadurch die Darstellung auf dem Bildschirm 7 verändern, was weiter unten beschrieben wird. Bevorzugt ist der Bildschirm 7 als ein berührungssensitiver Bildschirm (Touchscreen) ausgestaltet, und der Benutzer kann ein auf dem Bildschirm 7 dargestelltes Element berühren und verschieben, z.B. durch eine Bewegung mit einem Finger über den Bildschirm 7. Möglich ist auch, dass das Beatmungsgerät 1 oder das räumlich entfernte tragbare Gerät eine zusätzliche Eingabeeinheit umfasst, beispielsweise eine Maus 37 und / oder eine Tastatur oder eine Einheit, welche Spracheingaben erkennt.

Die Signalverarbeitungseinheit 5 vermag Alarme zu detektieren. Jeder detektierbare Alarm bezieht sich auf mindestens ein Signal, welches die Signalverarbeitungseinheit 5 durch Auswertung von Messwerten erzeugt hat. Ein Alarm liegt vor und wird automatisch detektiert, wenn dieses Signal an mindestens einem Abtast-Zeitpunkt und / oder für einen Zeitraum, der größer als ein vorgegebener Mindest-Zeitraum ist, ein vordefiniertes Alarm-Kriterium erfüllt.

Bevorzugt ist für jedes patientenbezogene Signal, das sich abhängig von Messwerten der am Patienten P positionierten Sensoren 2.1.1 bis 2.2.2, 3, 4, 6 erzeugen lässt, jeweils ein Sollbereich vorgegeben, in dem die Signalwerte liegen sollen. Dieser Sollbereich kann zeitlich konstant sein oder wird während des Einsatzes abhängig von Messwerten berechnet und kann daher zeitlich variabel sein. Ein Alarm-Kriterium für dieses Signal ist erfüllt, wenn an mindestens n1 Abtast-Zeitpunkten der Wert eines Signals unterhalb der unteren Schranke des Sollbereichs liegt. Ein weiteres Alarm-Kriterium ist erfüllt, wenn an mindestens n2 Abtast-Zeitpunkten der Wert eines Signals oberhalb der oberen Schranke des Sollbereichs liegt. Die Anzahlen n1 und n2 sind vorgegeben und können gleich sein oder sich voneinander unterscheiden. Ein Alarm-Kriterium kann auch dann erfüllt sein, wenn die zeitliche Veränderung oder Veränderungsrate eines Signals oberhalb einer vorgegebenen Veränderungs-Schranke liegt.

Die Signalverarbeitungseinheit 5 überprüft laufend, z.B. mit einer vorgegebenen Abtastfrequenz, ob mindestens ein patientenbezogenes Signal ein vorgegebenes Alarm-Kriterium erfüllt. Bevorzugt überprüft sie für jedes Signal, ob ein für dieses Signal vorgegebenes Alarm-Kriterium erfüllt ist. Falls dies der Fall ist, so hat die Signalverarbeitungseinheit 5 einen Alarm einer bestimmten Alarm-Art detektiert. Das als erfüllt detektierte Alarm-Kriterium legt fest, von welcher Alarm-Art der detektierte Alarm ist. Die Signalverarbeitungseinheit 5 detektiert die Alarm-Art und den Zeitpunkt oder den frühesten Zeitpunkt, an dem dieser Alarm aufgetreten ist. Selbstverständlich kann ein Alarm der gleichen Alarm-Art mehrmals hintereinander auftreten. Jeder Alarm ist durch die Alarm-Art und einen Auftretens-Zeitpunkt gekennzeichnet.

Die Signalverarbeitungseinheit 5 vergleicht insbesondere die patientenbezogenen Signale mit vorgegebenen Grenzwerten, beispielsweise mit den Grenzen eines Sollbereichs, und erzeugt einen patientenbezogenen Alarm, wenn ein Signalwert oberhalb eines oberen Grenzwerts oder unterhalb eines unteren Grenzwerts liegt.

Ein Beispiel für einen Alarm aufgrund eines abweichenden patientenbezogenen Signals ist "Pressure high" - der Atemwegsdruck P_{aw} (Differenzdruck zum Umgebungsdruck) liegt oberhalb eines vorgegebenen oberen Grenzwerts, beträgt beispielsweise 27 mbar. Ein weiteres Beispiel ist "MV low" - die Menge der in die Lunge zugeführten Atemluft liegt unterhalb eines unteren Grenzwerts, beträgt z.B. 3,65 Liter / min. Ein weiterer Alarm liegt vor, wenn die Atemfrequenz oberhalb einer oberen Schranke liegt ("RR high").

Die Signalverarbeitungseinheit 5 vermag auch Systemzustände des Beatmungsgeräts 1 zu überwachen und einen gerätetechnischen Alarm zu erzeugen, beispielsweise den Alarm "Batterie-Ladezustand gering". Dieser Alarm wird ausgelöst, wenn der Ladezustand der Batterie des Beatmungsgeräts 1 so gering ist, dass diese Batterie einen zeitweisen Ausfall eines stationären Spannungsversorgungsnetzes oder die Trennung des Beatmungsgeräts 1 vom Spannungsversorgungsnetz nicht für eine ausreichend lange Zeit überbrücken könnte. Ein weiterer gerätetechnischer Alarm wird z.B. dann generiert, wenn ein Sensor keinen validen Messwert liefern kann.

Die Signalverarbeitungseinheit 5 vermag Eingaben eines Benutzers zu erfassen und zu verarbeiten. Die Signalverarbeitungseinheit 5 steuert den Bildschirm 7 abhängig von erfassten Benutzereingaben an und veranlasst den Bildschirm 7, als Reaktion auf die Benutzereingabe verschiedene Darstellungen anzuzeigen, was im Folgenden beschrieben wird.

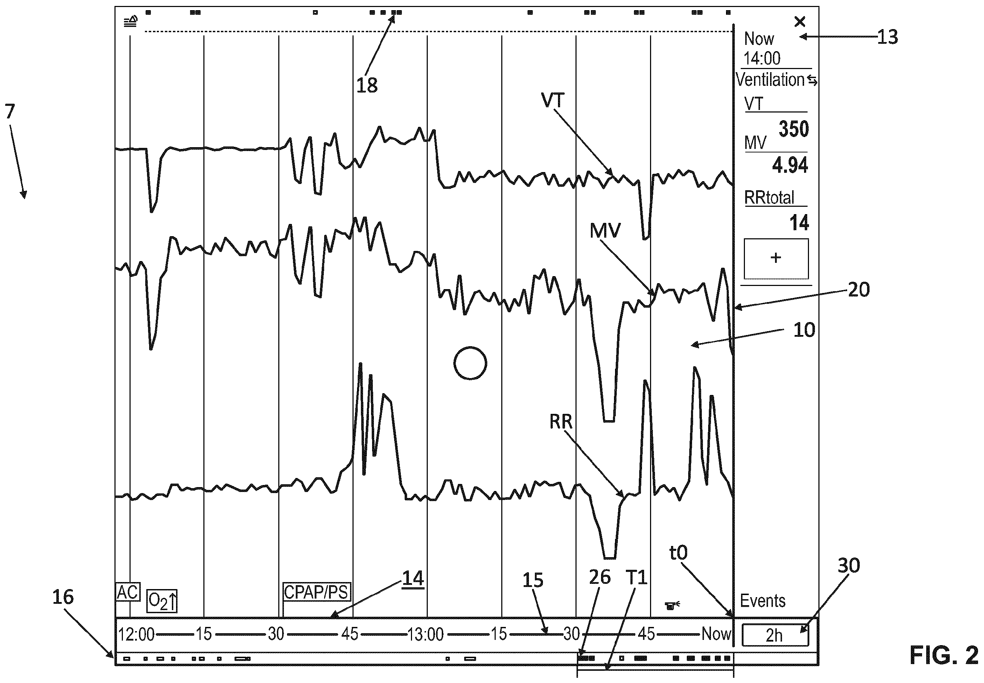

Figur 2 zeigt eine beispielhafte anfängliche Aufteilung eines Teilbereichs des Bildschirms 7. Für die Darstellung im Rest des Bildschirms 7 wird die Erfindung bevorzugt nicht verwendet. Dargestellt werden beispielhaft folgende Bereiche des Bildschirms 7:
- In einem zentralen Signalverlaufs-Bereich 10 des Bildschirms 7 werden in der gezeigten Situation die zeitlichen Verläufe von drei Signalen VT, MV und RR dargestellt. Der Benutzer kann festlegen, von welchen Signalen die zeitlichen Verläufe angezeigt werden.
- In einem Signalwerte-Bereich 13 rechts von dem zentralen Signalverlaufs-Bereich 10 werden ein Referenz-Zeitpunkt t0 (hier: 14:00 Uhr) sowie die Werte der drei Signale VT (350 ml), MV (4,94 Liter/min) und RR (14 / min) zu diesem Referenz-Zeitpunkt t0 dargestellt. In der gezeigten Situation ist der aktuelle Zeitpunkt (Now) der Referenz-Zeitpunkt t0, weswegen die aktuelle Uhrzeit dargestellt wird, und verändert sich mit fortschreitender Zeit.
- In einem unteren Bereich des Bildschirms 7 wird eine Alarm-Übersichts-Darstellung 14 dargestellt. Diese Alarm-Übersichts-Darstellung 14 umfasst eine Referenz-Zeitachse 15 und eine Alarm-Gesamt-Abfolge 16 von Alarmen sowie einen Alarm-Referenz-Abschnitt 26, was im Folgenden näher beschrieben wird.

Die drei zeitlichen Verläufe von drei Signalen VT, MV, RR, die in dem Signalverlaufs-Bereich 10 dargestellt werden, beziehen sich auf ein Referenz-Zeitfenster T1, das in diesem Fall von 12:00 Uhr bis 14:00 Uhr reicht. Die Referenz-Zeitachse 15 in der Alarm-Übersichts-Darstellung 14 bezieht sich auf dieses Referenz-Zeitfenster T1. Die drei dargestellten zeitlichen Signalverläufe VT, MV, RR beziehen sich ebenfalls auf die Referenz-Zeitachse 15 und zeigen den jeweiligen Verlauf des Signals im Referenz-Zeitfenster T1.

Eine Referenz-Zeitpunkt-Linie 20 auf dem Bildschirm 7 steht senkrecht auf der Referenz-Zeitachse 15 und zeigt den veränderlichen und veränderbaren Referenz-Zeitpunkt t0 an, in der in Figur 2 gezeigten Situation ist dies der aktuellen Zeitpunkt 14:00 Uhr. Diese Referenz-Zeitpunkt-Linie 20 bezieht sich ebenfalls auf die Referenz-Zeitachse 15. Anfänglich ist der Referenz-Zeitpunkt t0 der aktuelle Zeitpunkt, hier also 14:00 Uhr. Ein Benutzer kann einen früheren Zeitpunkt als veränderten Referenz-Zeitpunkt t0 vorgeben, was weiter unten beschrieben wird.

In der Alarm-Übersichts-Darstellung 14 werden unterhalb der Referenz-Zeitachse 15 mehrere verschiedenartige Alarme dargestellt. Jeder Alarm bezieht sich auf ein Signal, im gezeigten Beispiel auf das Signal MV oder auf das Signal VT oder auf das Signal RR, und ist detektiert, wenn ein vorgegebenes Alarm-Kriterium erfüllt ist. Jedes vorgegebene Alarm-Kriterium legt jeweils eine Alarm-Art fest, beispielsweise die Alarm-Arten "Signal MV zu niedrig" ("MV low") oder "Signal RR zu hoch" ("RR high").

In einer Alarm-Gesamt-Abfolge 16, die sich unterhalb der Referenz-Zeitachse 15 befindet, wird eine zeitliche Abfolge von Alarmen dargestellt. Diese Alarm-Gesamt-Abfolge 16 bezieht sich auf einen Gesamt-Zeitraum T während der Therapie des Patienten P, wobei der Gesamt-Zeitraum T größer ist als das durch die Referenz-Zeitachse 15 dargestellte Referenz-Zeitfenster T1. Das Referenz-Zeitfenster T1 ist also ein Abschnitt des Gesamt-Zeitraums T. Der Zeitmaßstab der Referenz-Zeitachse 15 ist feiner als der Zeitmaßstab der Alarm-Gesamt-Abfolge 16. Die Alarm-Gesamt-Abfolge 16 verwendet nicht die Referenz-Zeitachse 15, sondern eine Gesamt-Zeitachse, die in einer Ausgestaltung nicht dargestellt und nicht durch numerische Zeitangaben beschrieben wird, um auf dem Bildschirm 7 Platz einzusparen. Diese Gesamt-Zeitachse zeigt dieselbe Zeitspanne mit weniger Platz an als die Referenz-Zeitachse 15, und daher ist die Gesamt-Zeitachse für den Gesamt-Zeitraum T bevorzugt genauso lang wie die Referenz-Zeitachse 15 für das Referenz-Zeitfenster T1, obwohl der Gesamt-Zeitraum T länger ist als das Referenz-Zeitfenster T1. Zwangsläufig verwendet die Gesamt-Zeitachse einen gröberen Zeitmaßstab als die Referenz-Zeitachse 15.

In einer Ausgestaltung wird die Alarm-Gesamt-Abfolge 16 stets angezeigt. Sie bietet eine Übersicht über den gesamten Zeitraum der Therapie und ermöglicht es einem Benutzer, direkt ein anderes Referenz-Zeitfenster T1 auszuwählen. In einer anderen Ausgestaltung wird die Alarm-Gesamt-Abfolge 16 abhängig von einer Benutzereingabe eingeblendet oder ausgeblendet.

Ein Alarm-Referenz-Abschnitt 26 dieser Alarm-Gesamt-Abfolge 16 zeigt an, wann welche Alarm-Arten in dem Referenz-Zeitfenster T1 aufgetreten sind und wie das Referenz-Zeitfenster T1 im Zeitraum T positioniert ist. Die Alarm-Übersichts-Darstellung 14 zeigt also durch den Alarm-Referenz-Abschnitt 26 und die Alarm-Gesamt-Abfolge 16, welchen Abschnitt des Gesamt-Zeitraums T das Referenz-Zeitfenster T1 aktuell einnimmt, also eine Positionierungs-Darstellung. Dieser Abschnitt ist veränderbar. Für den Alarm-Referenz-Abschnitt 26 wird im Ausführungsbeispiel die Gesamt-Zeitachse verwendet.

In der gezeigten Realisierung ist der Alarm-Referenz-Abschnitt 26 ein Abschnitt der Alarm-Gesamt-Abfolge 16, was auf dem Bildschirm 7 Platz einspart. Möglich ist auch, dass der Alarm-Referenz-Abschnitt 26 räumlich getrennt von der Alarm-Gesamt-Abfolge 16 dargestellt wird. Jeder im Referenz-Zeitfenster T1 aufgetretene Alarm wird in dieser abweichenden Realisierung also zweimal dargestellt, nämlich einmal in der Alarm-Gesamt-Abfolge 16 und einmal in dem Alarm-Referenz-Abschnitt 26.

In einem Zeitdauer-Fenster 30 wird die zeitliche Länge des Referenz-Zeitfensters T1 angezeigt, hier 2h. Eine anfängliche zeitliche Länge wird vorgegeben. Ein Benutzer kann die zeitliche Länge des Referenz-Zeitfensters T1 verändern und auch auf diese Weise das Referenz-Zeitfenster T1 verändern. Beispielsweise berührt der Benutzer das Zeitdauer-Fenster 30 und kann dann z.B. durch einen Schieberegler und / oder mithilfe von dann eingeblendeten Tasten "+" und "-" und / oder durch Eingabe eines Zahlwerts eine neue zeitliche Länge vorgeben.

Die Alarm-Arten dieses Alarm-Referenz-Abschnitts 26 und somit die Alarm-Arten, die in dem Referenz-Zeitfenster T1 detektiert wurden, werden zusätzlich in einer Alarm-Referenz-Abfolge 18 oberhalb des Signalverlaufs-Bereichs 10 dargestellt.

Jeder Alarm, der in dem Referenz-Zeitfenster T1 detektiert wurde, wird in der Alarm-Gesamt-Abfolge 16 und in der Alarm-Referenz-Abfolge 18 durch das Symbol für die entsprechende Alarm-Art dargestellt. Die Zeitpunkte der in der Alarm-Referenz-Abfolge 18 dargestellten Alarme beziehen sich auf das Referenz-Zeitfenster T1, das unter Verwendung der Referenz-Zeitachse 15 dargestellt wird. Die Referenz-Zeitachse 15 wird also auch für die Alarm-Referenz-Abfolge 18 verwendet. Die Alarm-Referenz-Abfolge 18 bezieht sich ebenfalls auf die Referenz-Zeitachse 15 und verwendet den gleichen feineren Zeitmaßstab wie die Signalverlaufs-Darstellung 10.

In einer Ausgestaltung wird für jede Alarm-Art jeweils ein eigenes Symbol vorgegeben, und jeder Alarm wird in der Alarm-Gesamt-Abfolge 16, der Alarm-Referenz-Abfolge 18 und dem Alarm-Referenz-Abschnitt 26 mithilfe des Symbols für die Alarm-Art dargestellt.

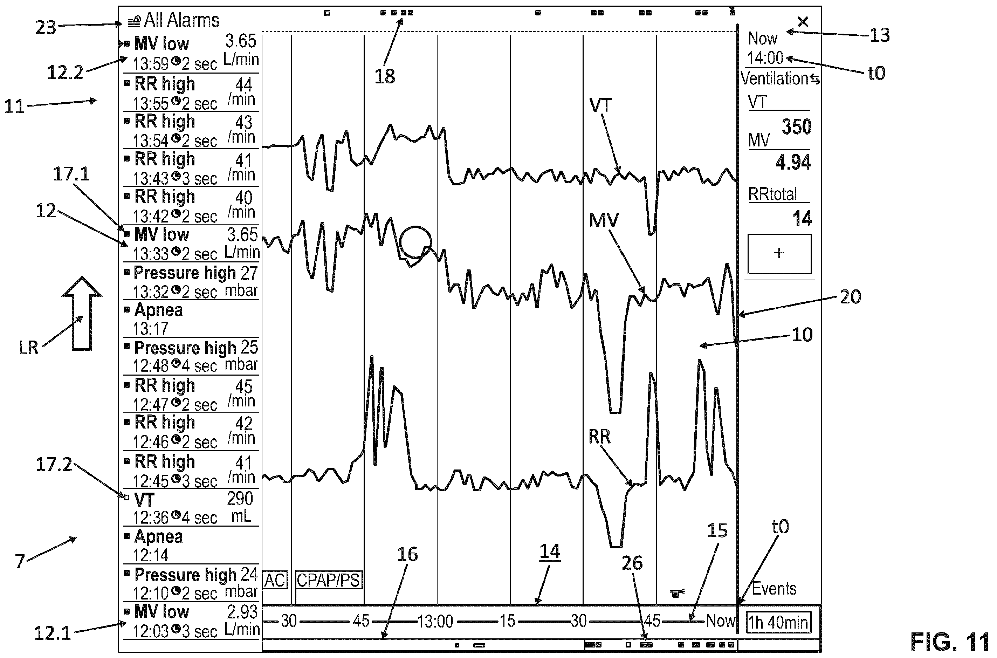

Jedoch stehen in vielen Anwendungen nicht genügend Symbole zur Verfügung, die sich in unterscheidbarer Weise darstellen lassen. Im Ausführungsbeispiel werden daher mehrere mögliche Relevanzen für einen Alarm vorgegeben, beispielsweise "niedrig", "mittel" und "hoch". Jeder Alarm-Art ist eine Relevanz zugeordnet, und jeder Relevanz ist ein Symbol zugeordnet, beispielsweise ein grüner Kreis für "niedrig", ein gelber Kreis für "mittel" und ein roter Kreis für "hoch" (also eine Ampeldarstellung). In den Abfolgen 16, 18 und 26 wird jeder Alarm mithilfe eines Kreises oder sonstigen Symbols dargestellt, wobei die Farbe und / oder Form dieses Symbols von der Relevanz der Alarm-Art abhängt. Der Alarm-Art "MV low" ist die Relevanz "hoch" zugeordnet, welche mit einem roten Kreis 17.1 dargestellt wird, der Alarm-Art die Relevanz "mittel", welche mit einem gelben Kreis 17.2 dargestellt wird, vgl. Figur 11.

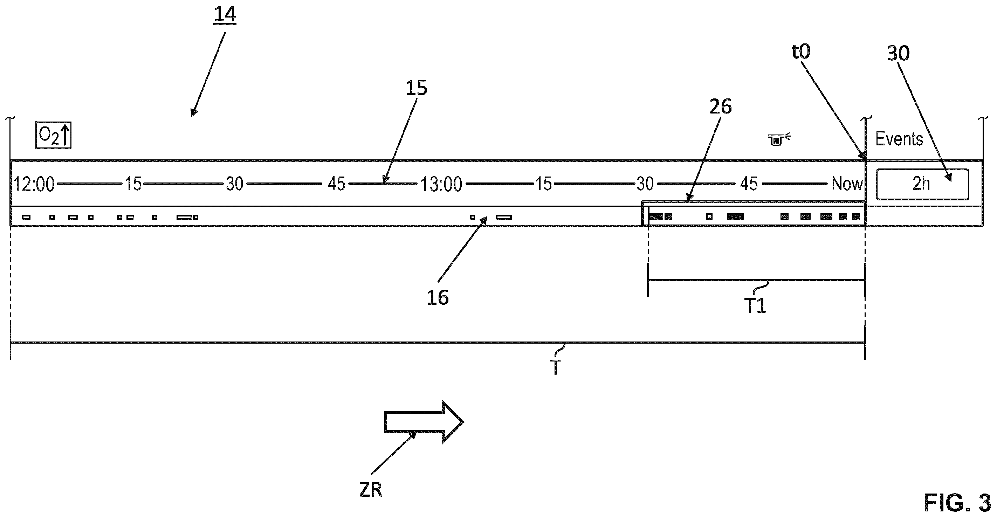

Figur 3 zeigt die Alarm-Übersichts-Darstellung 14 von Figur 2 in vergrößerter Form. Zu sehen sind:
- die Alarm-Gesamt-Abfolge 16, die den Gesamt-Zeitraum T abdeckt,
- der Alarm-Referenz-Abschnitt 26, die das Referenz-Zeitfenster T1 abdeckt, wobei zur Verdeutlichung ein rechteckiger schwarz dargestellter Rahmen um die Alarm-Referenz-Abschnitt 26 gelegt ist,
- wie der Alarm-Referenz-Abschnitt 26 zeitlich in der Alarm-Gesamt-Abfolge 16 und somit das Referenz-Zeitfenster T1 im Gesamt-Zeitraum T angeordnet ist, also die Positionierungs-Darstellung des Ausführungsbeispiels,
- die Referenz-Zeitachse 15, die sich auf das Referenz-Zeitfenster T1 bezieht und die für den Alarm-Referenz-Abschnitt 26 verwendet wird, aber nicht für die Alarm-Gesamt-Abfolge 16,
- das Zeitdauer-Fenster 30, welche die zeitliche Dauer des Referenz-Zeitfensters T1 zeigt, und
- der Referenz-Zeitpunkt t0 auf der Referenz-Zeitachse 15.

Weiterhin wird in Figur 3 die Zeitachsen-Darstellungs-Richtung ZR dargestellt. In diese Zeitachsen-Darstellungs-Richtung ZR erstrecken sich die Referenz-Zeitachse 15, die Alarm-Gesamt-Abfolge 16 und der Alarm-Referenz-Abschnitt 26. Die Zeitachsen-Darstellungs-Richtung ZR zeigt von älteren zu neueren Zeitpunkten. Die x-Achse der Signalverläufe, die in dem Signalverlaufs-Bereich 10 dargestellt werden, ist parallel zu dieser Zeitachsen-Darstellungs-Richtung ZR. Im gezeigten Beispiel zeigt die Zeitachsen-Darstellungs-Richtung ZR nach rechts, eine andere Ausrichtung ist ebenfalls möglich.

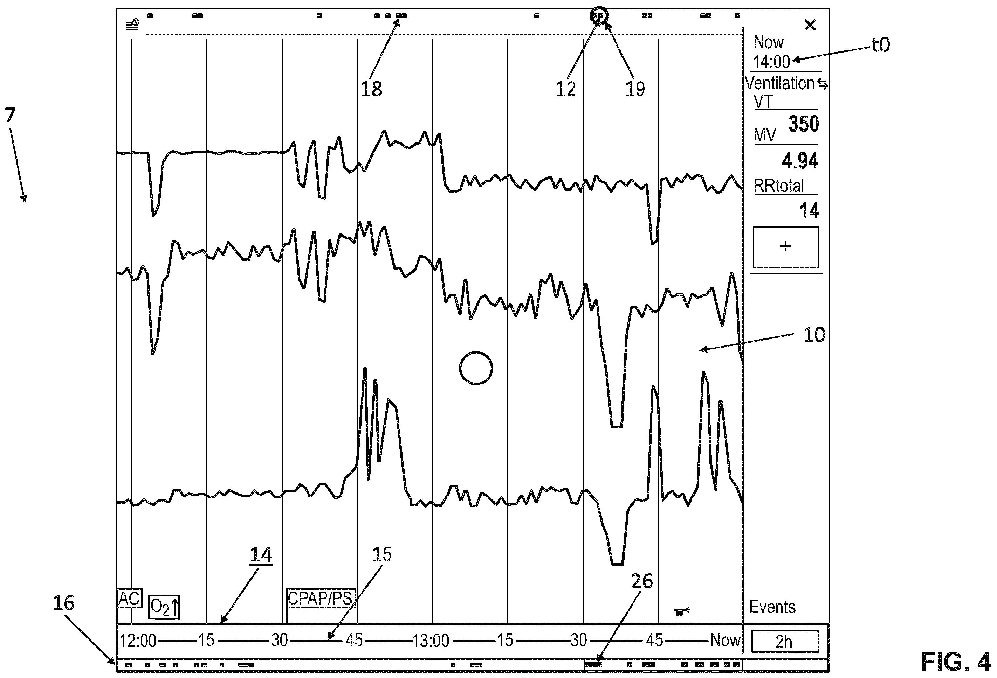

Ein Benutzer wählt einen dargestellten Alarm 12 aus. Dies kann der Benutzer auf unterschiedliche Weisen machen. Eine Möglichkeit zeigt Figur 4: Der Benutzer wählt einen Alarm 12 aus, der in der Alarm-Referenz-Abfolge 18 dargestellt ist, beispielsweise indem er die Darstellung für diesen Alarm 12 mit einem Finger berührt. Der Kreis 19 veranschaulicht in Figur 4 und in den folgenden Figuren die jeweilige Auswahl und Interaktion durch einen Benutzer. Nachfolgend werden weitere Möglichkeiten angegeben, wie ein Benutzer einen dargestellten Alarm 12 auswählen kann.

Die Auswahl des Alarms 12 in der Alarm-Referenz-Abfolge 18 veranschaulicht Figur 4. Dieser ausgewählte Alarm 12 gehört zu der Alarm-Art "MV low".

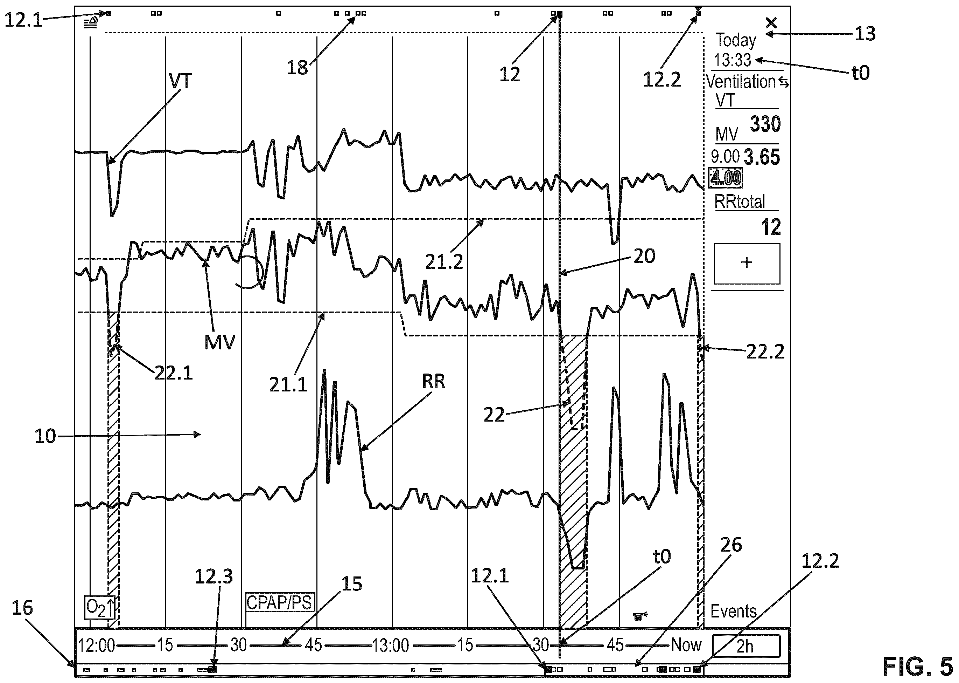

Figur 5 zeigt, welche Reaktionen diese Auswahl des Alarms 12 auslöst:
- Die Referenz-Zeitpunkt-Linie 20 springt zu dem Zeitpunkt 13:33 Uhr des ausgewählten Alarms 12. Dieser Zeitpunkt ist nunmehr der Referenz-Zeitpunkt t0.
- In dem Signalwerte-Bereich 13 werden der Zeitpunkt des ausgewählten Alarms 12 (der neue Referenz-Zeitpunkt t0, also 13:33 Uhr) sowie die jeweiligen Signalwerte der drei Signale MV, VT und RR zu diesem Zeitpunkt t0 (330 ml, 3,65 Liter/min bzw. 12 / min) dargestellt.
- In der Alarm-Referenz-Abfolge 18 sowie im Alarm-Referenz-Abschnitt 26 der Alarm-Gesamt-Abfolge 16 werden jeweils eine Abfolge der im Referenz-Zeitfenster T1 detektierten Alarme dargestellt. Als Reaktion auf die Auswahl des Alarms 12 werden diejenigen weiteren Alarme, die zu derselben Alarm-Art wieder ausgewählte Alarm 12 gehören, hervorgehoben dargestellt - hervorgehoben im Vergleich zu den übrigen dargestellten Alarmen. In diesem Beispiel gehören der Alarm 12.1 zum Zeitpunkt 12:03 Uhr sowie der Alarm 12.2 zum Zeitpunkt 13:59 Uhr zu derselben Alarm-Art "MV low" wie der ausgewählte Alarm 12. In Figur 5 werden beispielhaft alle Alarme, die zu derselben Alarm-Art "MV low" gehören, schwarz und die übrigen weiß mit schwarzem Rand dargestellt.
- Die Alarme 12, 12.1, 12.2 der Alarm-Art "MV low", die im Referenz-Zeitfenster T1 detektiert wurden, werden außerdem in der Alarm-Gesamt-Abfolge 16 angezeigt und zusätzlich ein weiterer Alarm 12.3, der außerhalb des Referenz-Zeitfensters T1 detektiert wurde.
- Ein Sollbereich für das Signal MV ist vorgegeben oder wird zur Laufzeit von der Signalverarbeitungseinheit 5 berechnet. Die untere Schranke 21.1 und die obere Schranke 21.2 dieses Sollbereichs werden dargestellt. Wie zu sehen ist, können die untere Schranke 21.1 und / oder die obere Schranke 21.2 zeitlich veränderlich sein.
- Diejenigen Abschnitte des Signals MV, die unterhalb der unteren Schranke 21.1 liegen und daher zu einem Alarm der Alarm-Art "MV low" führen, werden hervorgehoben gekennzeichnet. Im gezeigten Beispiel sind dies der Abschnitt 22 für den Alarm 12, der Abschnitt 22.1 für den Alarm 12.1 und der Abschnitt 22.2 für den Alarm 12.2. Beispielsweise werden die drei Abschnitte 22, 22.1 und 22.2 im Signalverlaufs-Bereich 10 durch eine andere Hintergrundfarbe hervorgehoben dargestellt. Dargestellt wird jeweils, wie lange der vom Sollzustand abweichende Zustand gedauert hat. Der Zeitpunkt des ausgewählten Alarms 12 und damit der ausgewählte Referenz-Zeitpunkt t0 ist im gezeigten Beispiel der erste Zeitpunkt des Abschnitts 22, an dem das Signal MV unterhalb der unteren Schranke 21.1 fällt.

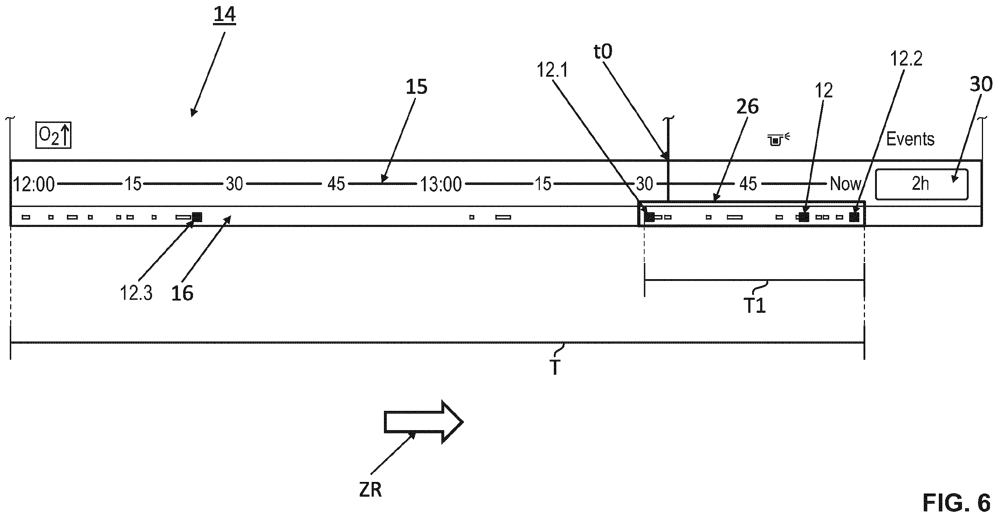

Figur 6 zeigt in einer Detaildarstellung von Figur 5, wie sich die Alarm-Übersichts-Darstellung 14 von Figur 3 verändert, nachdem der Alarm 12 ausgewählt wurde. In der Alarm-Gesamt-Abfolge 16 und damit auch in dem Alarm-Referenz-Abschnitt 26 sind nur noch die Alarme 12.1, 12.2 und 12.3 der Alarm-Art "MV low" hervorgehoben (hier: schwarz) gekennzeichnet und die übrigen nicht hervorgehoben (hier: weiß mit schwarzem Rahmen) dargestellt. Diese Ausgestaltung ermöglicht es, alle Alarme der Alarm-Art "MV low" hervorgehoben darzustellen, ohne ein spezielles Symbol für die Alarm-Art "MV low" zu verwenden, nämlich indem nur die Alarme "MV low" hervorgehoben werden. Zu sehen ist, wo im Alarm-Referenz-Abschnitt 26 der ausgewählte Alarm 12 angeordnet ist. Die Alarm-Übersichts-Darstellung 14 zeigt auch, wo der ausgewählte Alarm 12 zeitlich im Gesamt-Zeitraum T angeordnet ist. Weiterhin ist der Alarm 12.3 zu sehen, der außerhalb des Referenz-Zeitfensters T1 liegt.

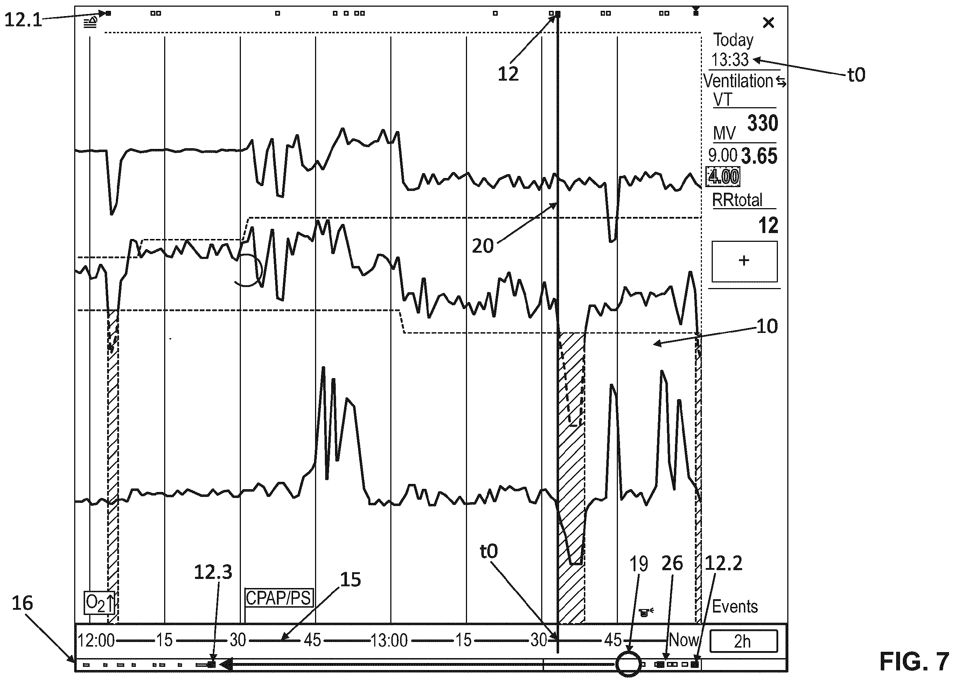

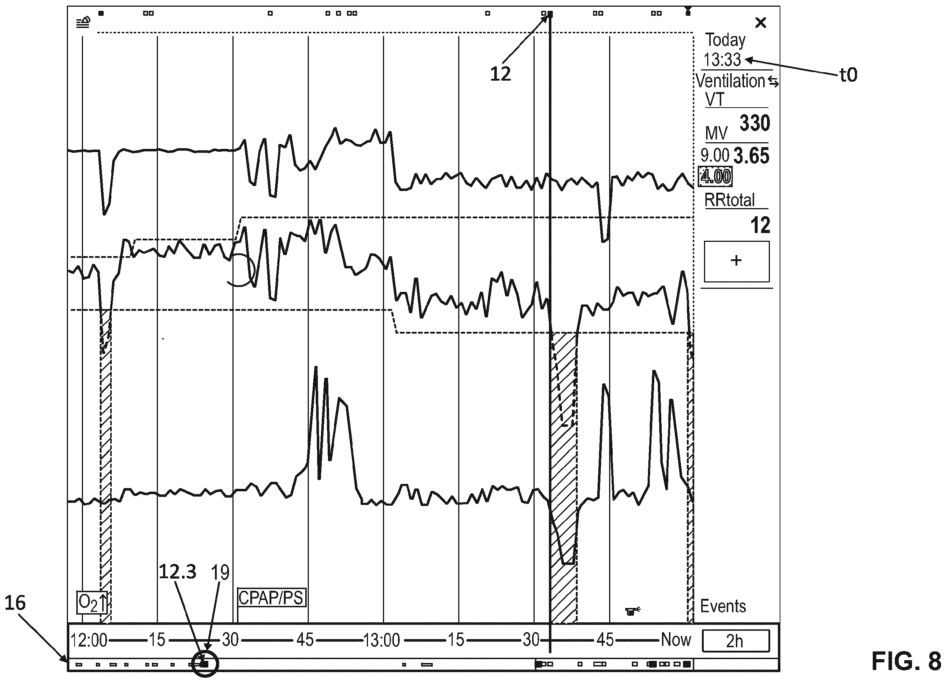

Figur 7 und Figur 8 veranschaulichen, wie der Benutzer ein zeitlich früheres Referenz-Zeitfenster T1 vorgibt und einen gleichartigen Alarm im früheren Referenz-Zeitfenster T1 auswählt. Der Gesamt-Zeitraum T bleibt unverändert. Das vorgegebene frühere Referenz-Zeitfenster T1 soll den Zeitpunkt umfassen, an dem der gleichartige Alarm 12.3 detektiert wurde. Der Benutzer berührt den Alarm-Referenz-Abschnitt 26 und zieht ihn nach links über den Zeitpunkt des Alarms 12.3, vgl. den Kreis 19 und den nach links zeigenden Pfeil in Figur 7. Der Benutzer wählt den Alarm 12.3 aus, beispielsweise indem er die Darstellung in der Alarm-Gesamt-Abfolge 16 mit einem Finger berührt, vgl. Figur 8.

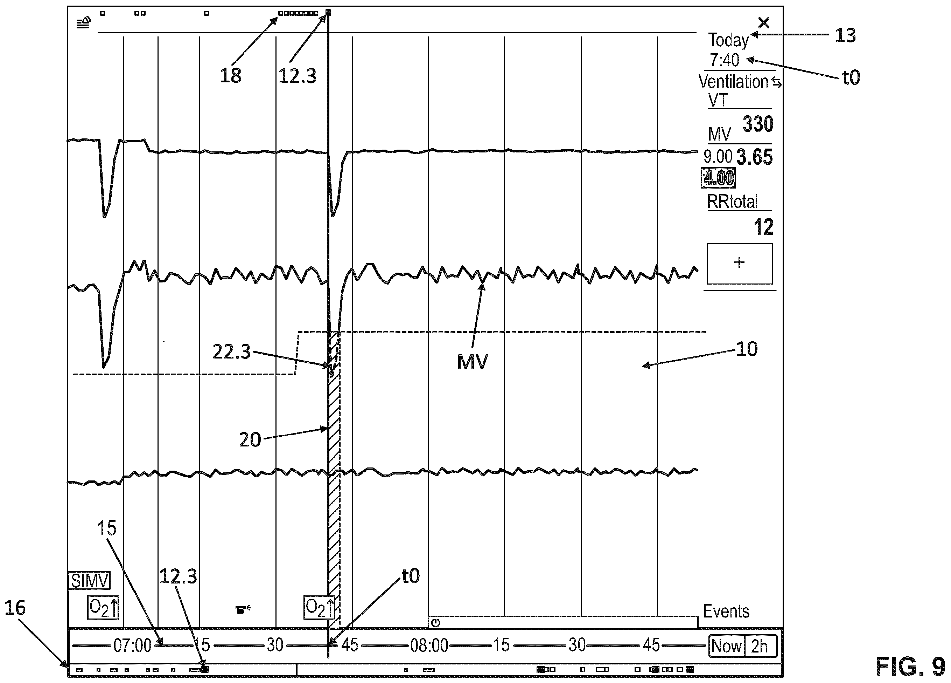

Die Reaktion auf diese Benutzereingabe zeigt Figur 9:
- Der Referenz-Zeitpunkt t0 ist nunmehr derjenige Zeitpunkt, an dem der Alarm 12.3 auftrat, nämlich 07:40 Uhr.
- Der Abschnitt 22.3 des Signals MV, der zu dem Alarm 12.3 geführt hat, wird hervorgehoben dargestellt.
- Die Referenz-Zeitpunkt-Linie 20 zeigt nunmehr als den Referenz-Zeitpunkt t0 den Zeitpunkt des Alarms 12.3, also 07:40 Uhr.
- In dem Signalwerte-Bereich 13 werden der neue Referenz-Zeitpunkt t0 und die Werte der drei Signale VT, MV und RR zu diesem Referenz-Zeitpunkt t0 angezeigt.
- Auf der Referenz-Zeitachse 15 wird das frühere Referenz-Zeitfenster T1 dargestellt.
- Der Signalverlaufs-Bereich 10 zeigt nunmehr die drei Signalverläufe VT, MV, RR im früheren Referenz-Zeitfenster T1.
- Die Alarm-Gesamt-Abfolge 16 bleibt unverändert.

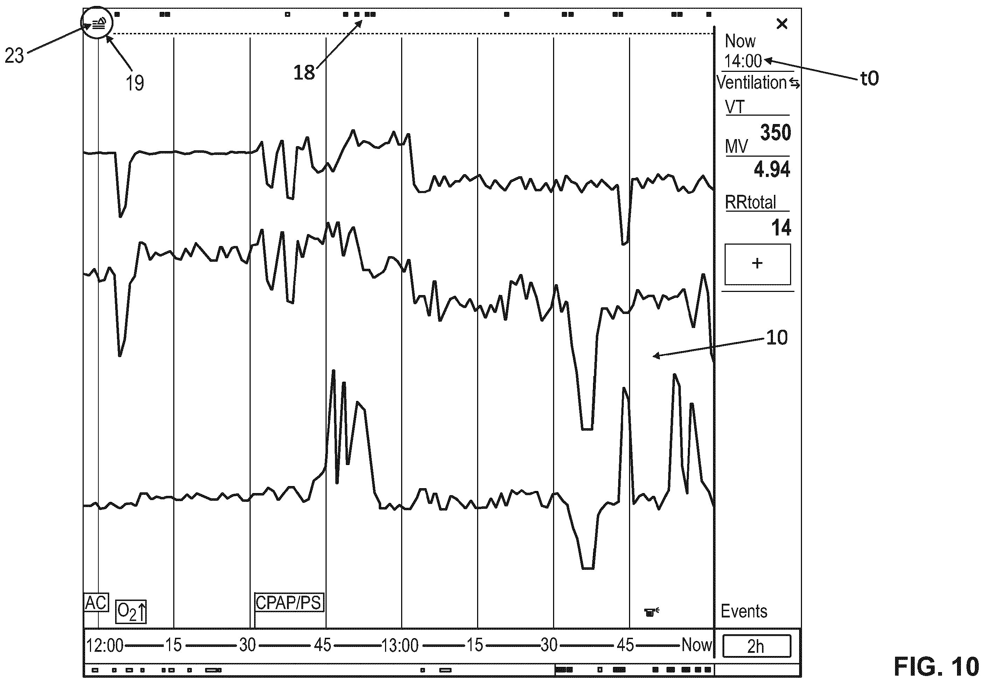

In den bislang gezeigten Beispielen hat der Benutzer einen Alarm durch Auswahl eines Symbols für die Alarm-Art und der zeitlichen Anordnung in der Alarm-Gesamt-Abfolge 16 oder in dem Alarm-Referenz-Abschnitt 26 ausgewählt. Im Folgenden wird eine andere Art gezeigt, um einen Alarm auszuwählen. Ausgangspunkt ist wiederum die anfängliche Situation, die in Figur 2 gezeigt wird. Der Benutzer klickt auf das Symbol 23 oder nimmt auf eine andere Weise eine entsprechende Benutzereingabe vor. Dies wird in Figur 10 durch den Kreis 19 um das Symbol 23 angedeutet.

Wie Figur 11 zeigt, wird als Reaktion hierauf zusätzlich eine Alarm-Beschreibungs-Abfolge 11 dargestellt, und zwar links neben dem Signalverlaufs-Bereich 10. Im gezeigten Beispiel zeigt diese Alarm-Beschreibungs-Abfolge 11 textliche Beschreibungen einer Sequenz von maximal N zeitlich unmittelbar aufeinander folgenden Alarmen, wobei die Anzahl N bevorzugt von der vertikalen Abmessung des Bildschirms 7 und von der bevorzugt veränderbaren Schriftgröße abhängt. Die Alarm-Beschreibungs-Abfolge 11 zeigt diese N Alarm-Beschreibungen in einer Reihenfolge von oben nach unten, wobei die Alarm-Beschreibung des zeitlich jüngsten Alarms der Sequenz oben gezeigt wird. Jede Alarm-Beschreibung nimmt in der Alarm-Beschreibungs-Abfolge 11 den gleichen vertikalen Platz ein - vorausgesetzt die Alarm-Beschreibungen werden in einer horizontalen Schreibrichtung SR dargestellt.

Die Alarm-Beschreibungs-Abfolge 11 ist eine zeitlich geordnete Liste von textlichen Alarm-Beschreibungen, wobei diese Liste sich auf eine Sequenz von maximal N Alarmen bezieht und wobei diese Alarme im Gesamt-Zeitraum T detektiert worden sind. Im gezeigten Beispiel wurden einige Alarme im Referenz-Zeitfenster T1 detektiert, beispielsweise der Alarm 12 zum Zeitpunkt 13:33 Uhr und der Alarm 12.2 zum Zeitpunkt 13:59 Uhr.

Die Liste erstreckt sich in die Listen-Richtung LR. Diese Listen-Richtung LR steht bevorzugt senkrecht auf der Zeitachsen-Darstellungs-Richtung ZR. Im gezeigten Beispiel ist die Listen-Richtung LR senkrecht. Je jünger ein Alarm ist, desto weiter oben steht dessen Alarm-Beschreibung in dieser geordneten Liste 11. Die Schreibrichtung SR einer einzelnen Alarm-Beschreibung steht senkrecht auf der Listen-Richtung LR und ist damit - bei zweidimensionaler Darstellung - parallel zur Zeitachsen-Darstellungs-Richtung ZR. Auch eine perspektivische, also räumliche, Darstellung ist möglich.

Eine Konsequenz des Schrittes, die Alarm-Beschreibungs-Abfolge 11 anzuzeigen, ist die, dass im Ausführungsbeispiel das Referenz-Zeitfenster T1 zeitlich verkürzt wird. Der Zeitmaßstab und somit der Darstellungsmaßstab bleiben unverändert, aber es steht weniger Platz für das Referenz-Zeitfenster T1 zur Verfügung. Der feinere Zeitmaßstab bleibt unverändert. Nachdem die Alarm-Beschreibungs-Abfolge 11 angezeigt ist, erstreckt sich das verkürzte Referenz-Zeitfenster T1 von 12:20 Uhr bis 14:00 Uhr, ist also um etwa 20 Minuten kürzer. Entsprechend verkürzt sich der Anteil, den der Alarm-Referenz-Abschnitt 26 in der Alarm-Gesamt-Abfolge 16 einnimmt. Die Positionierungs-Darstellung wird also automatisch verändert.

Die Alarme, die in der Alarm-Beschreibungs-Abfolge 11 angezeigt werden, sind im ursprünglichen Referenz-Zeitfenster T1 detektiert worden, welches von 12:00 Uhr bis 14:00 Uhr dauert. In einer Ausgestaltung ist es möglich, dass ein in der Alarm-Beschreibungs-Abfolge 11 angezeigter Alarm nicht mehr in dem verkürzten Referenz-Zeitfenster T1, welches von 12:20 Uhr bis 14:00 Uhr dauert, liegt, beispielsweise der Alarm 12.1 zum Zeitpunkt 12:03 Uhr.

Die gerade beschriebene Ausgestaltung ist sinnvoll, falls die textlichen Beschreibungen in einer Sprache dargestellt werden, die eine horizontale Schreibrichtung vorsieht, beispielsweise bei Englisch oder Deutsch von links nach rechts oder bei Hebräisch oder Arabisch von rechts nach links. Falls die textlichen Beschreibungen in einer Sprache mit einer vertikalen Schreibrichtung dargestellt werden, beispielsweise traditionelles Chinesisch oder Japanisch, so wird die Darstellung bevorzugt entsprechend angepasst. Beispielsweise verlaufen die Zeitachsen-Darstellungs-Richtung ZR und die Schreibrichtung SR einer textlichen Alarm-Beschreibung vertikal, und die Listen-Richtung LR ist horizontal. Bevorzugt lässt sich konfigurieren, in welcher Sprache textliche Ausgaben erzeugt werden sollen, und dadurch werden die Schreibrichtung und damit die Zeitachsen-Darstellungs-Richtung ZR und die Listen-Richtung LR festgelegt.

Möglich ist auch, auf dem Bildschirm 7 eine perspektivische Darstellung anzuzeigen, wobei die Zeitachsen-Darstellungs-Richtung ZR, die Listen-Richtung LR und die Schreibrichtung SR ein dreidimensionales kartesisches Koordinatensystem festlegen, welches perspektivisch dargestellt wird.

In dem gezeigten Beispiel erstreckt sich die Alarm-Beschreibungs-Abfolge 11 in die Listen-Richtung LR, wobei eine Alarm-Beschreibung eines Alarms der Sequenz umso weiter oben dargestellt wird, je jünger der Alarm ist.

Für jeden dargestellten Alarm werden jeweils folgende Informationen dargestellt:
- eine textliche Beschreibung der Alarm-Art, z.B. "MV low" oder "RR high",
- das Symbol für die Relevanz dieser Alarm-Art,
- der jeweiligen Zeitpunkt des Alarms - genauer: der erste Zeitpunkt, an dem dieser Alarm auftrat,
- optional die jeweilige Zeitdauer des Alarms, bevorzugt in [sec] und
- optional der jeweiligen Wert oder der maximal von einem Sollbereich abweichende Wert des betreffenden Signals zum Zeitpunkt oder Zeitraum des Alarms.

Mehrere Alarme der gleichen Alarm-Art können in dem dargestellten Zeitbereich auftreten, in dem in Figur 11 gezeigten Beispiel drei Mal ein Alarm der Art "MV low" und sieben Mal ein Alarm der Art "RR high".

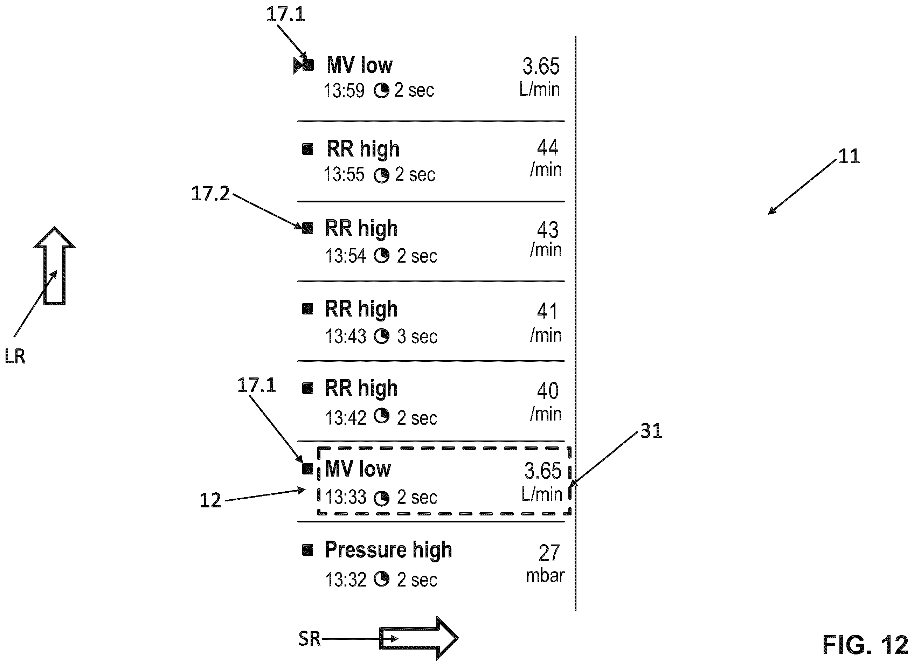

Figur 12 zeigt in einer vergrößerten Darstellung mehrere Alarm-Beschreibungen. Die beispielhafte Alarm-Beschreibung 31 für den Alarm 12 umfasst folgende Informationen:
- die textliche Beschreibung "MV low" der Alarm-Art "MV low" des Alarms 12,
- der Zeitpunkt 13:33 Uhr,
- die Zeitdauer 2 sec,
- der Signalwert 3,65 Liter/min zum Zeitpunkt des Alarms 12 und
- das Symbol 17.1 für die Relevanz "hoch" der Alarm-Art "MV low".

In Figur 12 werden weiterhin die Listen-Richtung LR der Alarm-Beschreibungs-Abfolge 11 sowie die Schreibrichtung SR der textlichen Alarm-Beschreibung dargestellt. Die Schreibrichtung SR steht senkrecht auf der Listen-Richtung LR.

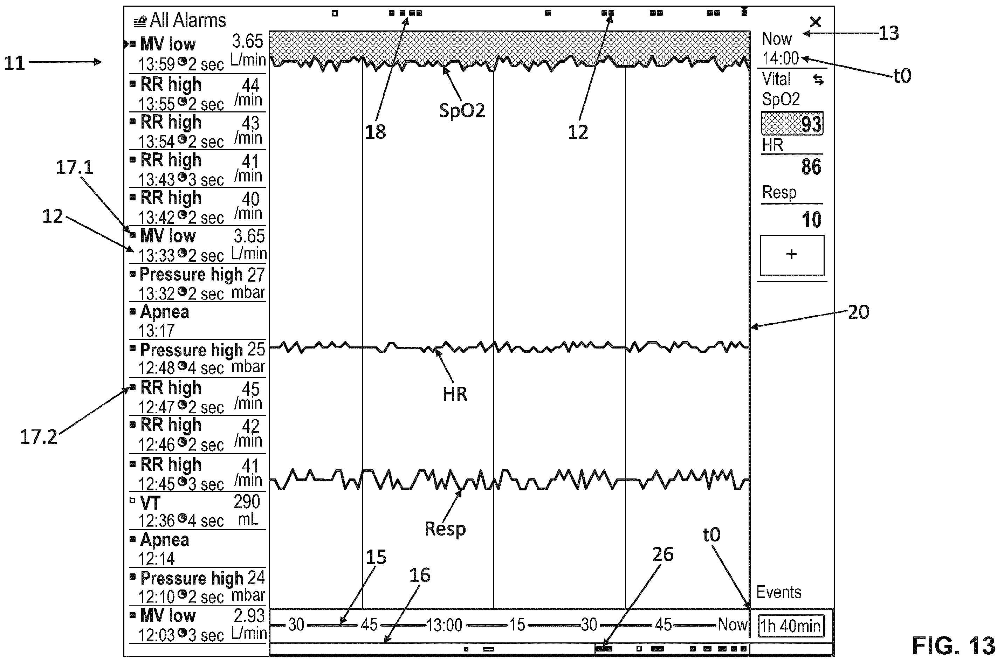

Figur 13 zeigt einen anderen Ausgangspunkt für die Auswahl eines Alarms der Alarm-Art "MV low". In dem Signalverlaufs-Bereich 10 werden Verläufe der drei Signale SpO2, HR und Resp dargestellt, aber nicht der Verlauf des Signals MV. Der Benutzer kann in der Alarm-Referenz-Abfolge 18 den Alarm 12 auswählen oder wie im Folgenden beschrieben vorgehen.

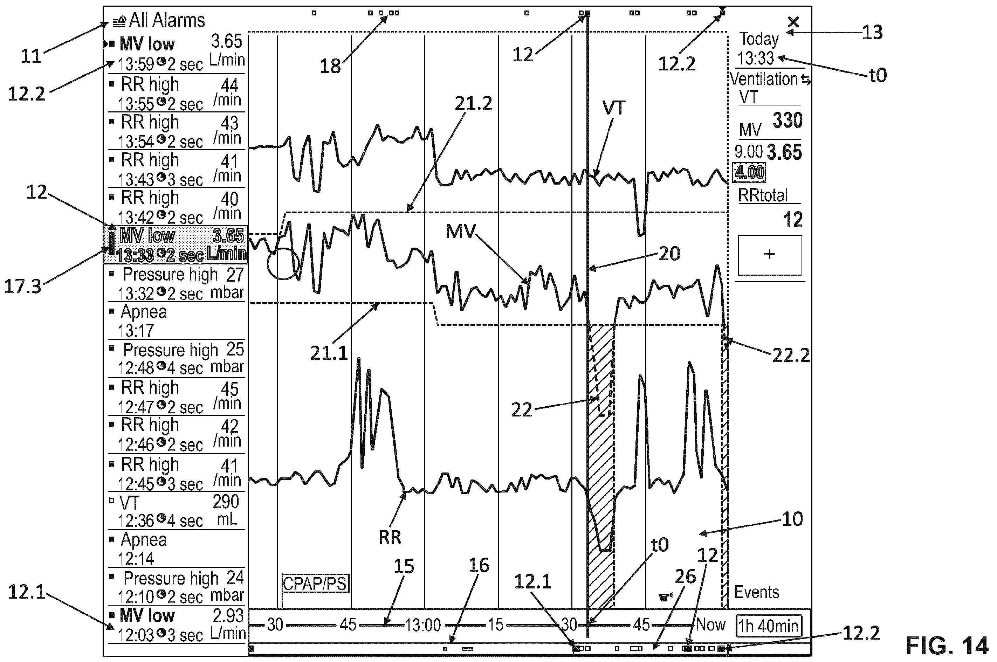

Der Benutzer wählt in der Alarm-Beschreibungs-Abfolge 11 eine dargestellte Alarm-Beschreibung aus, beispielsweise diejenige des Alarms 12. Figur 14 zeigt beispielhaft, welche Reaktionen die Auswahl des Alarms 12 hat:
- In dem Signalverlaufs-Bereich 10 werden die Verläufe der Signale VT, MV und RR im Referenz-Zeitfenster T1 angezeigt.
- Die Referenz-Zeitpunkt-Linie 20 springt zu dem Zeitpunkt 13:33 Uhr des ausgewählten Alarms 12, wobei dieser Zeitpunkt nunmehr als Referenz-Zeitpunkt t0 fungiert.
- In dem Signalwerte-Bereich 13 werden der Zeitpunkt t0 des ausgewählten Alarms 12 (13:33 Uhr) sowie die Signalwerte der drei Signale MV, VT und RR zu diesem Referenz-Zeitpunkt t0 (330 ml, 3,65 Liter/min bzw. 12 / min) dargestellt.
- In der Alarm-Beschreibungs-Abfolge 11 werden alle Alarme 12, 12.1, 12.2 der ausgewählten Alarm-Art "MV low" im Vergleich zu den übrigen Alarmen hervorgehoben gekennzeichnet, beispielsweise indem die übrigen Alarme mager und nur die Alarme 12, 12.1, 12.2 weiterhin fett dargestellt werden.
- In der Alarm-Referenz-Abfolge 18 werden in der Abfolge der Alarme im Zeitfenster, die durch die Symbole für die jeweiligen Alarm-Arten dargestellt werden, hier also werden die Alarme der Alarm-Art "MV low" im Vergleich zu den anderen Alarmen hervorgehoben dargestellt.
- Der ausgewählte Alarm 12 wird auch in der Alarm-Beschreibungs-Abfolge 11 gegenüber den Alarmen 12.1, 12.2 derselben Alarm-Art "MV low" hervorgehoben gekennzeichnet, beispielsweise durch eine andere Hintergrundfarbe.
- Die Alarm-Art "MV low", in diesem Fall die Relevanz für die Alarm-Art, des Alarms 12 wird mit einem anderen Symbol 17.3 dargestellt, beispielsweise mit einem Rechteck anstelle mit einem Kreis.
- Die Alarm-Gesamt-Abfolge 16 bleibt unverändert.

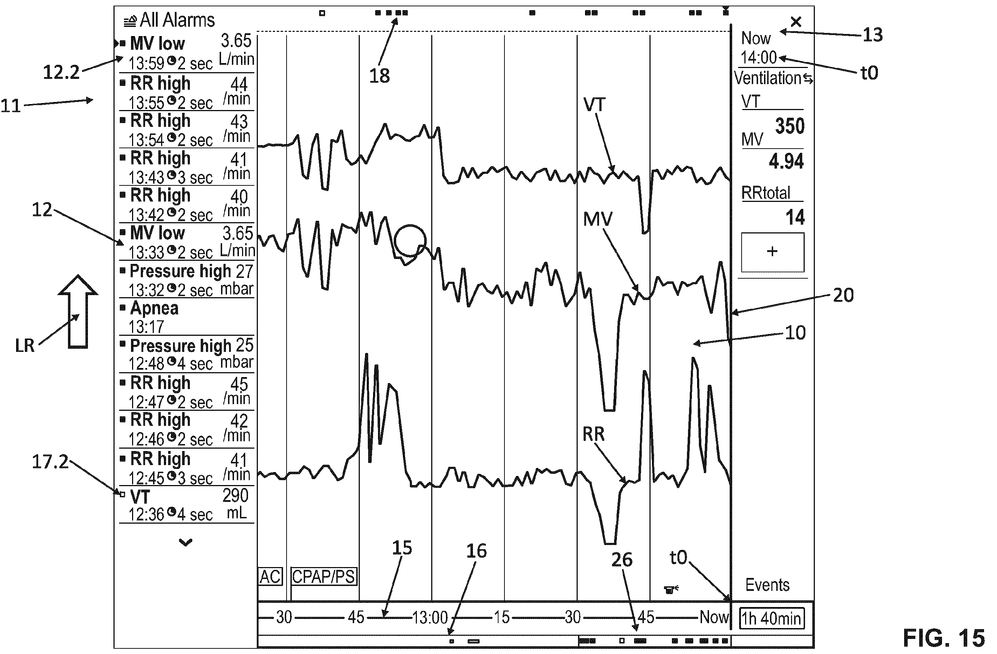

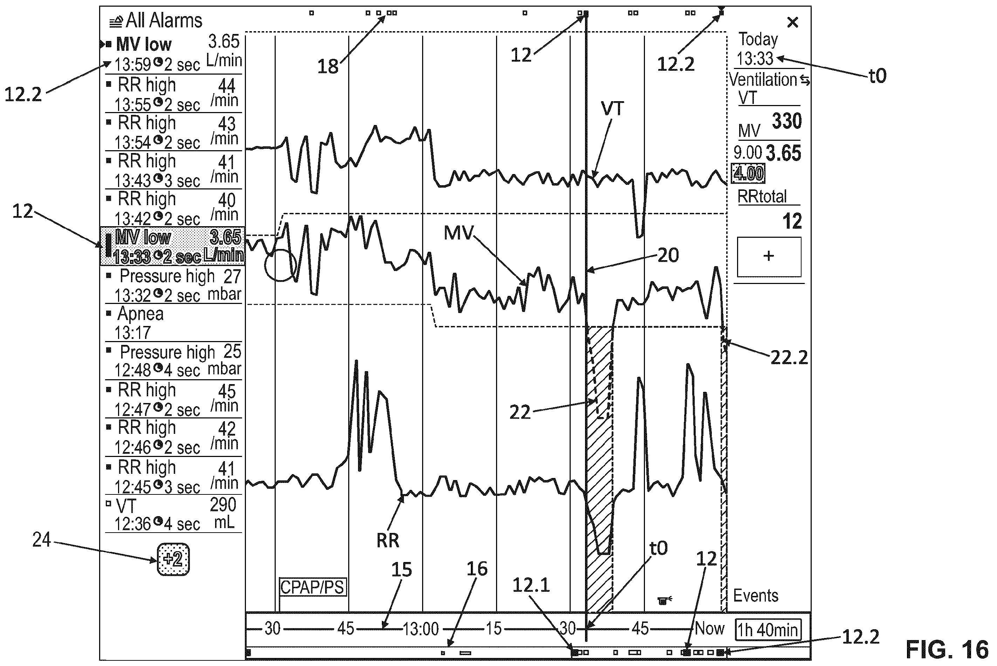

In der abweichenden Ausgestaltung von Figur 15 bis Figur 17 zeigt die Alarm-Beschreibungs-Abfolge 11 nur solche Alarme, die im verkürzten Referenz-Zeitfenster T1 liegen. Figur 15 zeigt die Situation vor Auswahl eines Alarms in der Alarm-Beschreibungs-Abfolge 11. Figur 16 zeigt die Reaktion, nachdem der Benutzer den Alarm 12 ausgewählt hat.

Ein Platz 24 unterhalb der Alarm-Beschreibungs-Abfolge 11 ist frei geblieben, weil die Alarm-Beschreibungs-Abfolge 11 entsprechend kurz ist. In diesem Platz 24 wird die Anzahl von Alarmen im Gesamt-Zeitraum T angegeben, die zeitlich vor dem Referenz-Zeitfenster T1 liegen und die daher nicht in der Alarm-Beschreibungs-Abfolge 11 dargestellt werden und von der gleichen Alarm-Art wie der ausgewählte Alarm 12 sind, in diesem Falle zwei Alarme (+2), vgl. Figur 16.

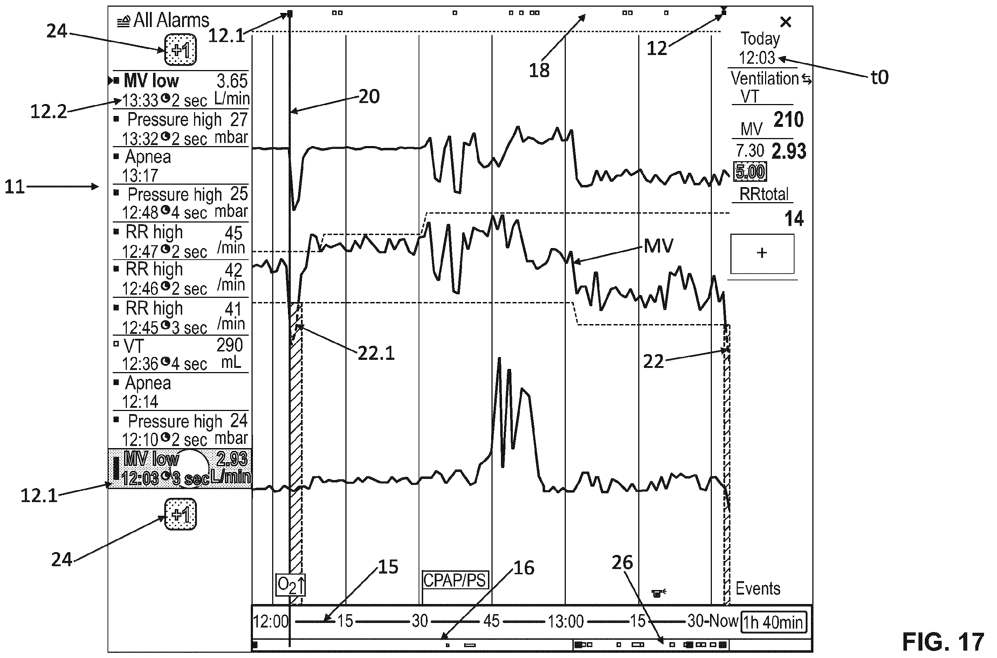

Der Benutzer kann auf die Darstellung dieser Anzahl klicken, beispielsweise mit einem Finger. Dadurch werden zeitlich frühere Alarme dargestellt, siehe Figur 17. Das Referenz-Zeitfenster T1 verändert sich, in diesem Falle auf den Zeitraum von 11:50 Uhr bis 13:35 Uhr, was in der Referenz-Zeitachse 15 sichtbar wird. Der feinere Zeitmaßstab bleibt unverändert. Der Referenz-Zeitpunkt t0 ist nunmehr 12:03 Uhr. Die Alarm-Referenz-Abfolge 18 wird entsprechend verschoben dargestellt. Weiterhin wird in zwei Plätzen 24 dargestellt, dass jeweils ein früherer und ein späterer Alarm, der im Gesamt-Zeitraum T vor bzw. nach dem Referenz-Zeitfenster T1 detektiert wurde und von der gleichen Alarm-Art wie der ausgewählte Alarm 12 ist (+1), nicht in der geänderten Alarm-Beschreibungs-Abfolge 11 dargestellt werden, vgl. Figur 17.

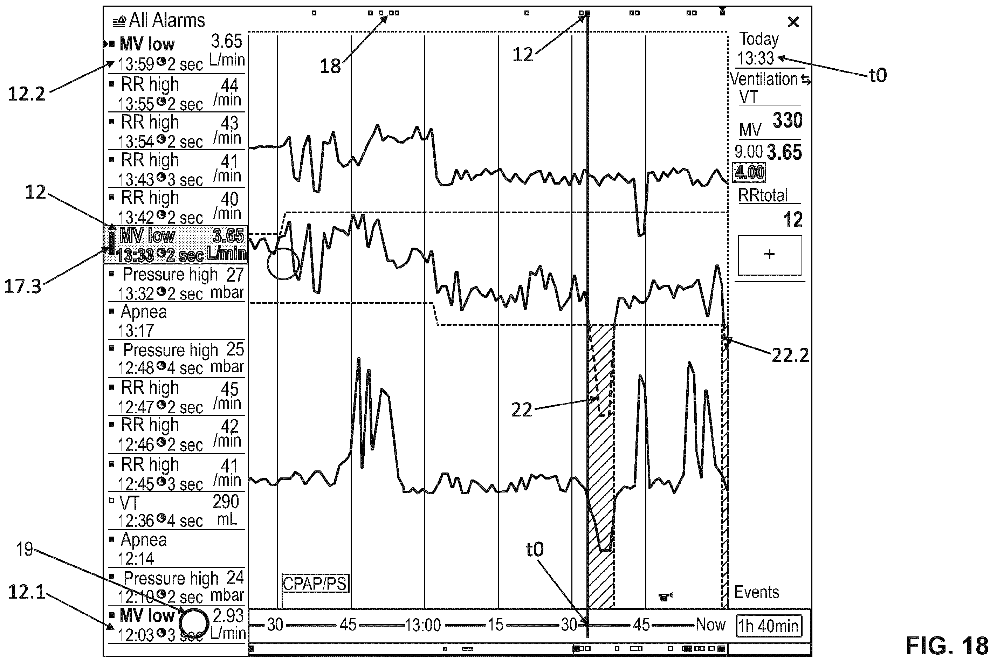

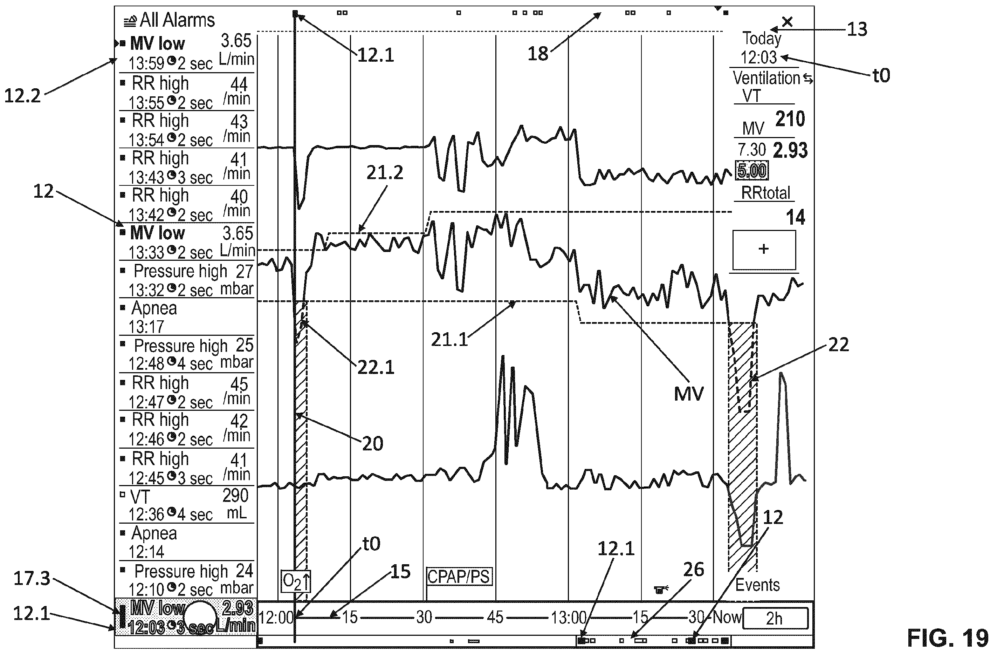

Im Beispiel von Figur 18 wählt der Benutzer den Alarm 12.1 aus, der zum Zeitpunkt 12:03 Uhr detektiert wurde. Dieser Alarm 12.1 liegt außerhalb des aktuellen Referenz-Zeitfensters T1. Figur 19 zeigt die Reaktion auf diese Auswahl:
- Auf der Referenz-Zeitachse 15 wird ein anderes Referenz-Zeitfenster T1 gezeigt, nämlich der Zeitraum von 11:55 Uhr bis 13:35 Uhr. Der ausgewählte Alarm 12.1 liegt in diesem veränderten Referenz-Zeitfenster T1.
- Die dargestellten Alarme in der Alarm-Referenz-Abfolge 18 und im Alarm-Referenz-Abschnitt 26 beziehen sich auf dieses veränderte Referenz-Zeitfenster T1.
- Der Referenz-Zeitpunkt t0 ist nunmehr der Zeitpunkt 12:03 Uhr des ausgewählten Alarms 12.1. Im Signalwerte-Bereich 13 werden dieser Referenz-Zeitpunkt t0 sowie die Signalwerte zu diesem Referenz-Zeitpunkt t0 angezeigt.
- Die Referenz-Zeitpunkt-Linie 20 springt auf den geänderten Referenz-Zeitpunkt t0.
- Der ausgewählte Alarm 12.1 wird hervorgehoben dargestellt.
- Der Gesamt-Zeitraum T und die Alarm-Gesamt-Abfolge 16 bleiben unverändert.

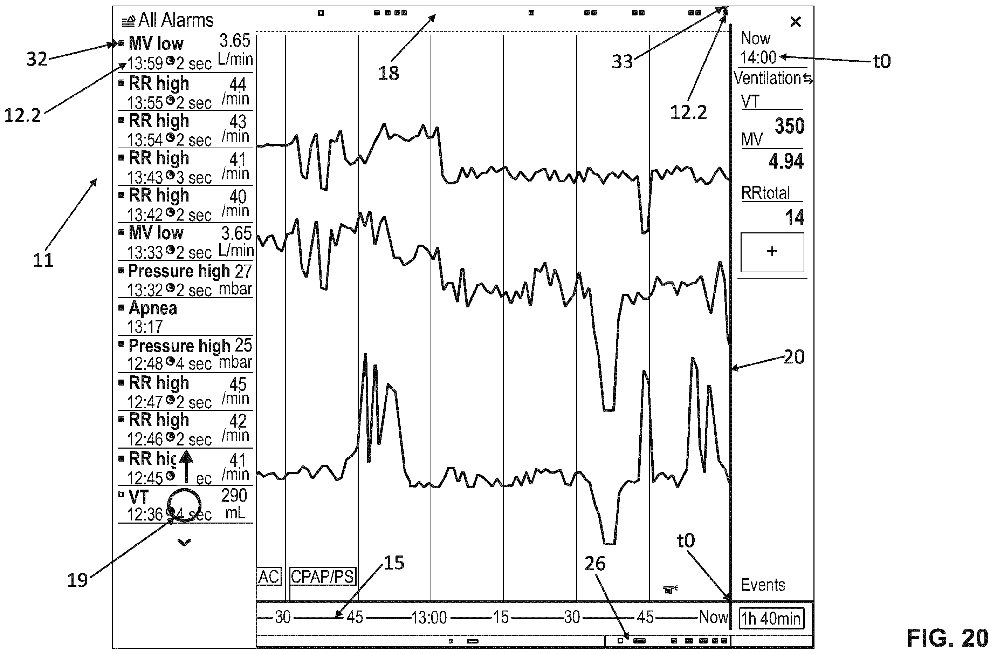

Figur 20 bis Figur 22 illustrieren, wie ein Korrelationsanzeiger verwendet wird. Dieser Korrelationsanzeiger erleichtert es einem Benutzer, einen bestimmten Alarm und den Zeitpunkt, an dem dieser Alarm aufgetreten ist, in der Alarm-Referenz-Abfolge 18 aufzufinden.

Der Korrelationsanzeiger umfasst ein führendes Element 32 und ein geführtes Element 33. Im gezeigten Beispiel hat das führende Element 32 die Form eines nach rechts zeigenden Dreiecks und das geführte Element 33 die Form eines nach unten oder nach oben zeigenden Dreiecks. Das führende Element 32 zeigt stets auf die oberste Alarm-Beschreibung in der Alarm-Beschreibungs-Abfolge 11. Diese Alarm-Beschreibung bezieht sich auf einen Alarm, der in dem oder außerhalb des Referenz-Zeitfensters T1 liegen kann und der aktuell ausgewählt oder nicht ausgewählt sein kann. Das geführte Element 33 zeigt auf das Symbol für diesen obersten Alarm in der Alarm-Referenz-Abfolge 18. Das geführte Element 33 wird dem führenden Element 32 nachgeführt.

Im Beispiel von Figur 20 zeigt das führende Element 32 auf die oberste Alarm-Beschreibung, das ist die für den Alarm 12.2 (Alarm-Art "MV low", Zeitpunkt 13:59 Uhr). Dies ist auch die in Figur 11 gezeigte Situation. Das geführte Element 33 zeigt auf das Symbol in der Alarm-Referenz-Abfolge 18 für diesen Alarm 12.2. Der Benutzer verändert die Sequenz von Alarmen, deren Alarm-Beschreibungen in der Alarm-Beschreibungs-Abfolge 11 angezeigt werden. Dies wird durch den Kreis 19 und durch den nach oben zeigenden Pfeil angedeutet.

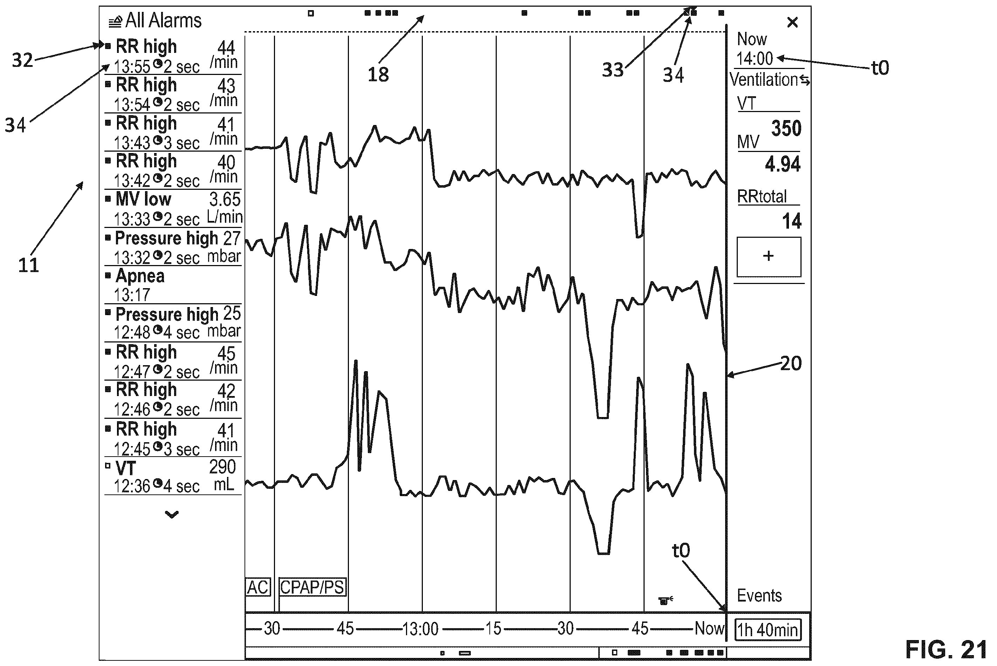

Nach dieser Benutzereingabe ist der Alarm 34 (Alarm-Art "RR high", Zeitpunkt 13:55 Uhr) der oberste Alarm in der Alarm-Beschreibungs-Abfolge 11, vgl. Figur 21. Das führende Element 32 zeigt daher auf die Alarm-Beschreibung für den Alarm 34. Das geführte Element 33 zeigt auf das Symbol für diesen Alarm 34 in der Alarm-Referenz-Abfolge 18.

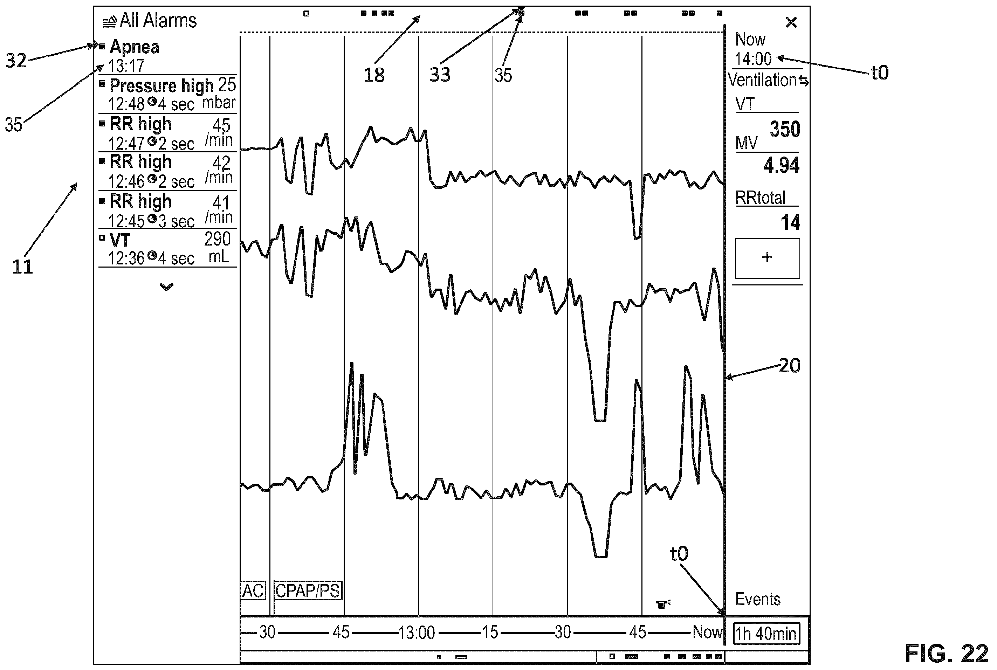

Figur 22 zeigt die Auswirkung einer weiteren Benutzereingabe. Das führende Element 32 zeigt auf die Alarm-Beschreibung für den Alarm 35 (Alarm-Art "Apnea", Zeitpunkt 13:17 Uhr). Das geführte Element 33 zeigt auf das Symbol für diesen Alarm 35 in der Alarm-Referenz-Abfolge 18.

Im Beispiel von Figur 20 bis Figur 22 zeigt das führende Element 32 stets auf die oberste Alarm-Beschreibung in der Alarm-Beschreibungs-Abfolge 11. Möglich ist, dass der Benutzer das führende Element 32 auf eine andere Alarm-Beschreibung in der Alarm-Beschreibungs-Abfolge 11 verschieben kann. Das geführte Element 33 zeigt dann auf den entsprechenden Alarm in der Alarm-Referenz-Abfolge 18. Möglich ist auch, dass das führende Element 32 auf einen Alarm in der Alarm-Referenz-Abfolge 18 zeigt und vom Benutzer bewegt werden kann. Das geführte Element 33 zeigt auf die Alarm-Beschreibung für diesen Alarm in der Alarm-Beschreibungs-Abfolge 11.

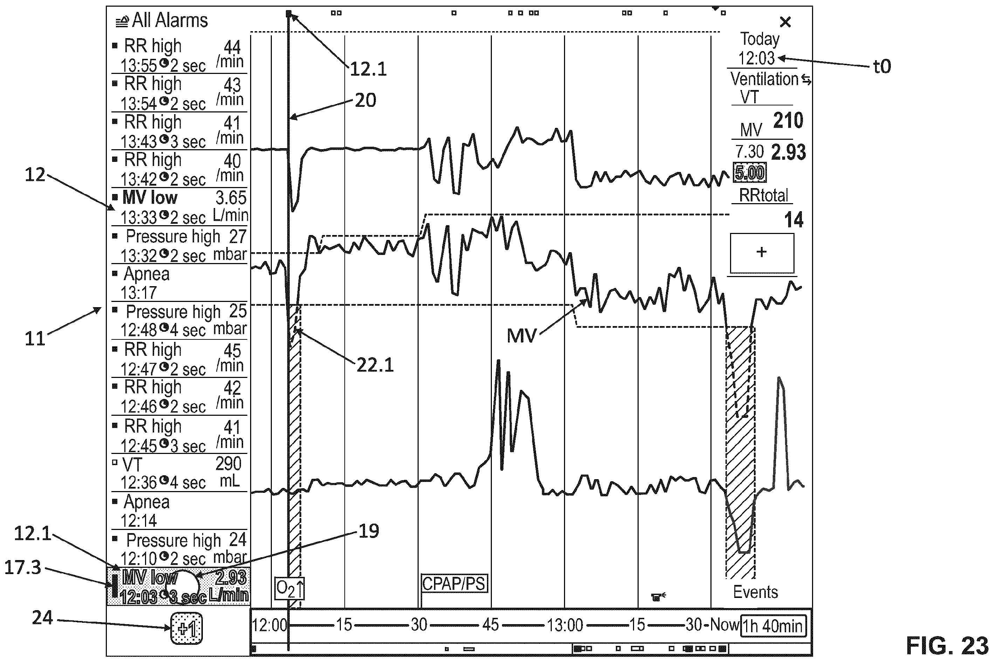

Ein Benutzer kann sich anzeigen lassen, welche Alarme, die vor dem aktuellen Referenz-Zeitfenster T1 aufgetreten sind, zu der gleichen Alarm-Art "MV low" gehören wie der aktuell ausgewählte Alarm 12.1. Dem Benutzer wird die Möglichkeit gegeben, das Referenz-Zeitfenster T1 zu verschieben. Figur 23 veranschaulicht, dass der Benutzer den aktuell ausgewählten Alarm 12.1 in der Alarm-Beschreibungs-Abfolge 11 nach oben zieht. Dadurch wird ein Platz 24 in der Alarm-Beschreibungs-Abfolge 11 frei, in diesem Falle an unterster Stelle. In diesem Platz 24 wird die Anzahl derjenigen Alarme angezeigt, die im Gesamt-Zeitraum T vor dem Referenz-Zeitfenster T1 detektiert wurden, ebenfalls zu der Alarm-Art "MV low" gehören und aktuell nicht in der Alarm-Beschreibungs-Abfolge 11 angezeigt werden, in diesem Falle "+1". Die Position des Platzes 24 ganz links unten zeigt, dass dieser zusätzliche Alarm zeitlich vor den im Alarm-Beschreibungs-Abfolge 11 angezeigten Alarmen detektiert wurde.

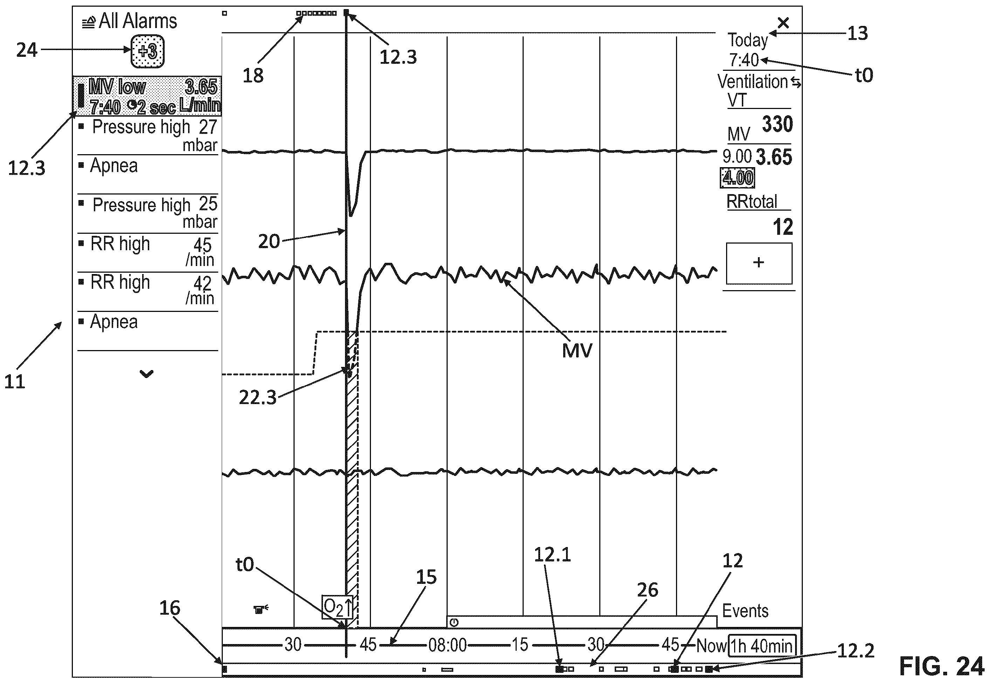

Im gezeigten Beispiel klickt der Benutzer auf die im Platz 24 gezeigte Anzeige, vgl. Figur 23. Die Anzeige "+1" im Platz 24 bezieht sich in diesem Beispiel auf den Alarm 12.3. Figur 24 zeigt die Reaktionen:
- Die Alarm-Beschreibungs-Abfolge 11 wird abgeändert.
- In der abgeänderten Alarm-Beschreibungs-Abfolge 11 wird der Alarm 12.3 hervorgehoben dargestellt.
- Im Platz 24 wird angezeigt, dass zeitlich nach dem nunmehr hervorgehoben dargestellten Alarm 12.3 drei weitere Alarme ("+3") der gleichen Alarm-Art "MV low" detektiert wurden. Deshalb befindet sich der Platz 24 nunmehr oberhalb der dargestellten Alarme.
- Die Referenz-Zeitpunkt-Linie 20 springt auf den veränderten Referenz-Zeitpunkt t0, das ist der Zeitpunkt 07:40 Uhr des Alarms 12.3.
- Die Referenz-Zeitachse 15 zeigt ein verändertes Referenz-Zeitfenster T1, nämlich dasjenige, in dem der Alarm 12.3 liegt. Die Signalverläufe im Signalverlaufs-Bereich 10, die Alarm-Referenz-Abfolge 18 und der Alarm-Referenz-Abschnitt 26 beziehen sich auf dieses veränderte Referenz-Zeitfenster T1 und werden entsprechend verändert.
- Im Signalwerte-Bereich 13 werden der veränderte Referenz-Zeitpunkt t0 = 07:40 Uhr und die Signalwerte zu diesem Referenz-Zeitpunkt t0 dargestellt.
- Der Abschnitt 22.3 für den Alarm 12.3 wird hervorgehoben dargestellt.
- Der Gesamt-Zeitraum T und die Alarm-Gesamt-Abfolge 16 bleiben unverändert.

Wenn der Benutzer auf die Anzahl-Anzeige "+3" im Platz 24 klickt, so wird wieder die Darstellung von Figur 23 gezeigt.

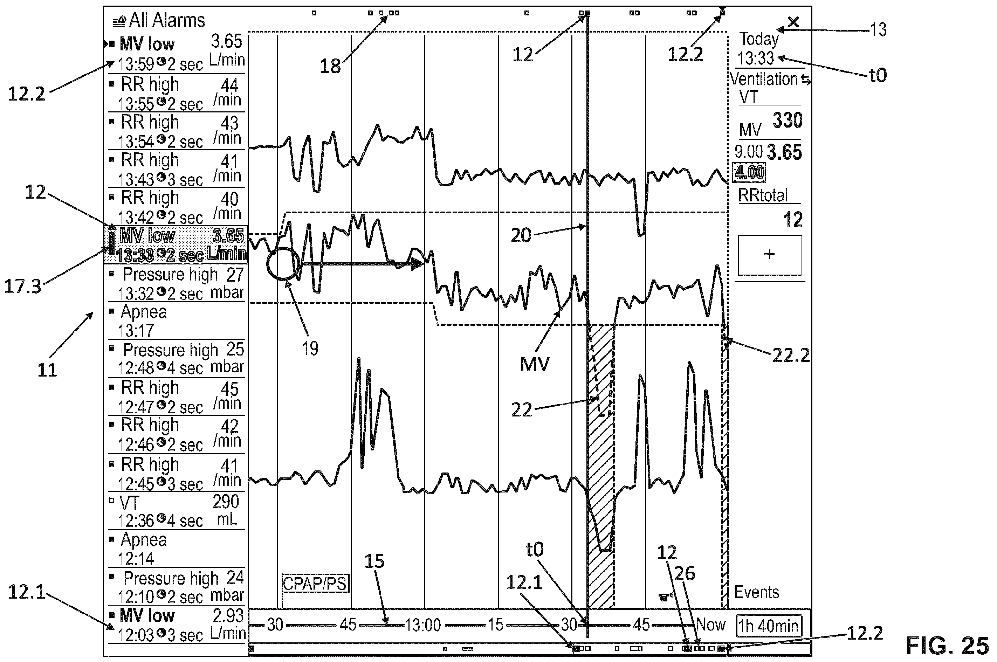

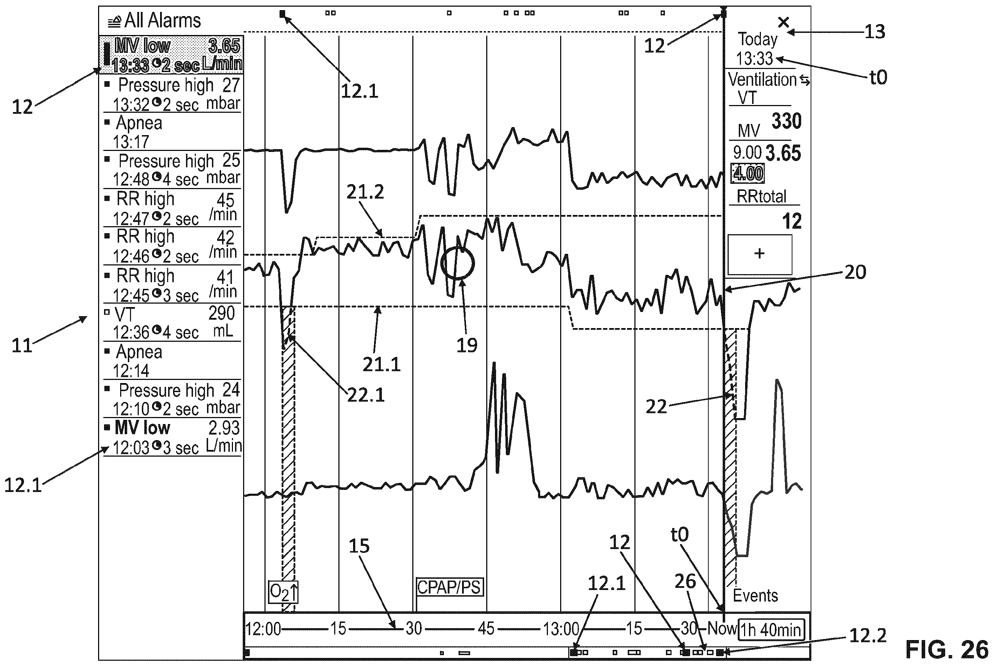

Figur 25 und Figur 26 zeigen eine andere Art, um das dargestellte Referenz-Zeitfenster T1 zu verschieben. Ausgangspunkt ist wiederum die Situation, welche in Figur 14 gezeigt wird. Der Benutzer berührt die Darstellung des Signalverlaufs MV im Signalverlaufs-Bereich 10 und zieht diese nach rechts, was eine Verschiebung des Referenz-Zeitfensters T1 nach links, also zu früheren Zeitpunkten, bewirkt. Der feinere Zeitmaßstab bleibt wiederum unverändert. Diese Verschiebung wird in Figur 25 durch den Kreis 19 und den Pfeil angedeutet.

Figur 26 veranschaulicht die Reaktion auf diese Verschiebung des Referenz-Zeitfensters:
- Auf der Referenz-Zeitachse 15 wird das verschobene Referenz-Zeitfenster T1 angezeigt.
- Die Alarm-Referenz-Abfolge 18 und der Alarm-Referenz-Abschnitt 26 werden so abgeändert, dass sie sich nach der Abänderung auf das verschobene Referenz-Zeitfenster T1 beziehen.
- In der Alarm-Beschreibungs-Abfolge 11 werden Alarme aus dem verschobenen Referenz-Zeitfenster T1 angezeigt.
- Die Auswahl des Alarms 12 und die Festlegung des Referenz-Zeitpunkts t0 bleiben erhalten.
- Die Referenz-Zeitpunkt-Linie 20 wird entsprechend der anderen Position des Referenz-Zeitpunkts t0 auf der Referenz-Zeitachse 15 verschoben.

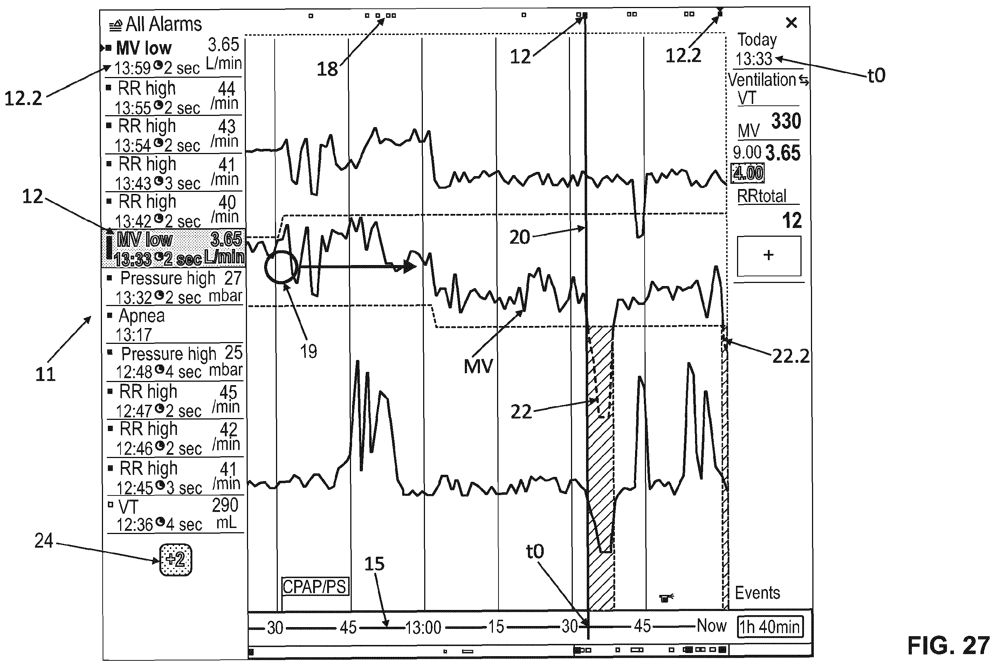

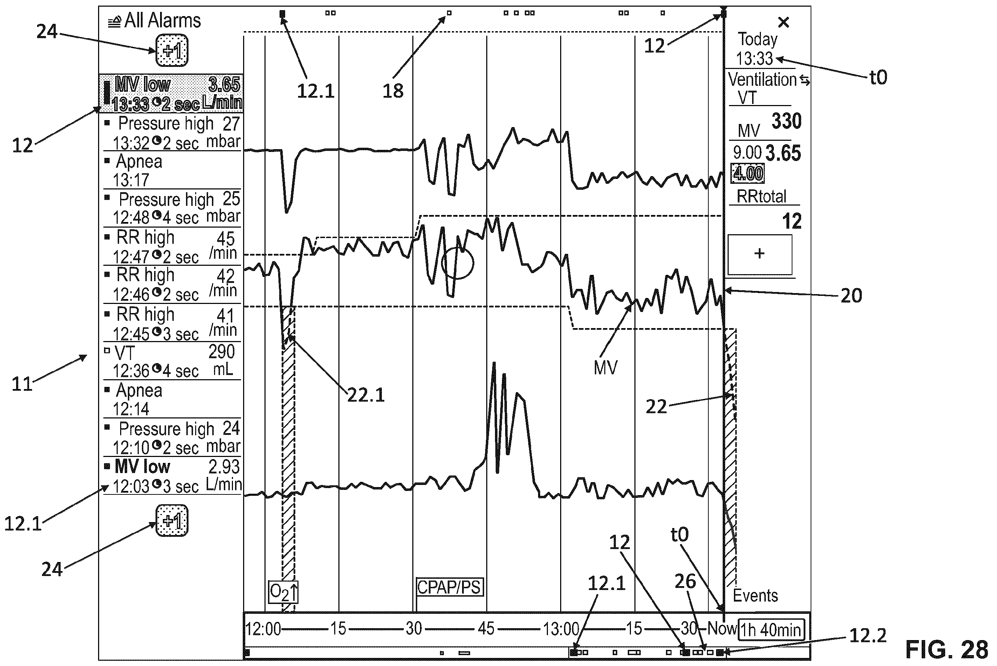

Figur 27 und Figur 28 zeigen eine alternative Ausgestaltung. In dieser alternativen Ausgestaltung werden in der Alarm-Beschreibungs-Abfolge 11 nur diejenigen Alarme angezeigt, die im verkürzten Referenz-Zeitfenster T1 liegen. Im Platz 24 wird die Anzahl von weiteren Alarmen der gleichen Alarm-Art angezeigt.

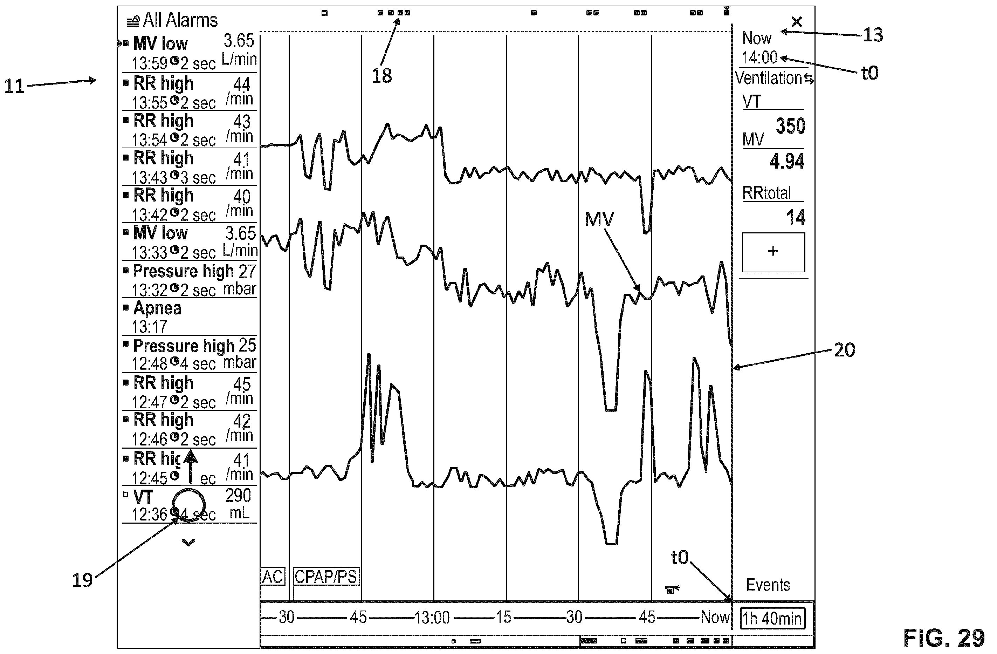

Figur 29 zeigt eine andere Vorgehensweise, wie ein Benutzer die Alarm-Sequenz, deren Alarm-Beschreibungen in der Alarm-Beschreibungs-Abfolge 11 angezeigt wird, zu verändern. Dies ermöglicht es dem Benutzer, eine Alarm-Art auszuwählen, in diesem Falle die Alarm-Art "RR high". Ausgangspunkt ist die Situation, die in Figur 11 gezeigt wird. Der Benutzer sucht in der Alarm-Beschreibungs-Abfolge 11 einen Alarm aus, hier den Alarm der Alarm-Art "VT" zum Zeitpunkt 12:36 Uhr, und zieht diesen ausgesuchten Alarm in der Alarm-Beschreibungs-Abfolge 11 nach oben. Dies wird in Figur 29 durch den Kreis 19 und den nach oben zeigenden Pfeil angedeutet.

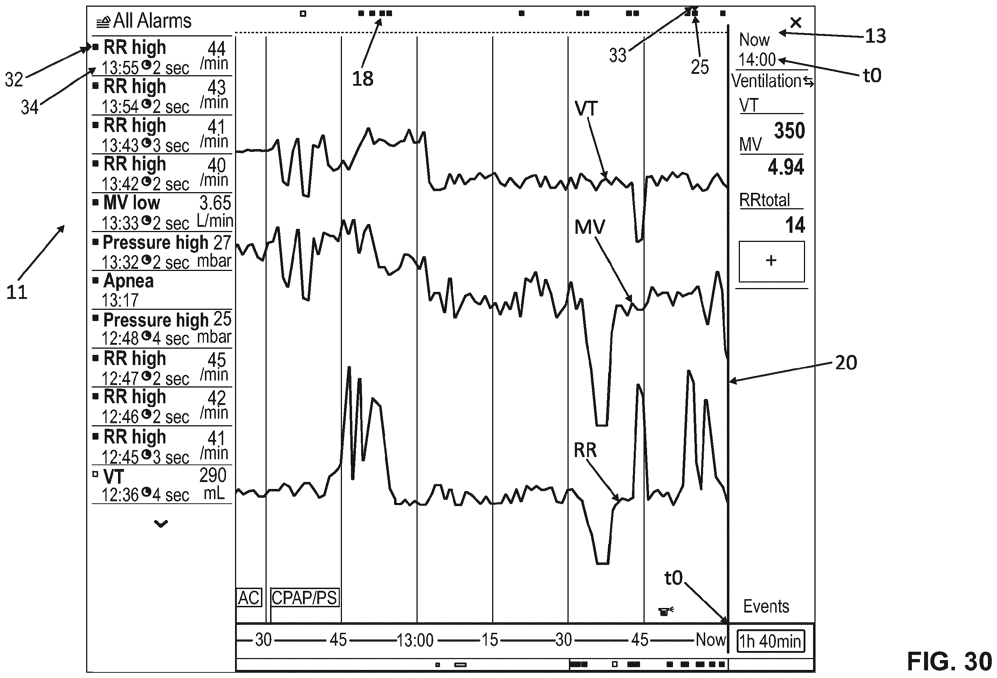

Figur 30 zeigt die entstandene Situation nach dem Verschieben. In der Alarm-Beschreibungs-Abfolge 11 wird als oberster Alarm der Alarm 34 der Alarm-Art "RR high" zum Zeitpunkt 13:55 Uhr gezeigt.

In den bisherigen Anwendungen war der Referenz-Zeitpunkt t0 der aktuelle Zeitpunkt (hier: 14:00 Uhr) oder der Zeitpunkt eines Alarms. Der Benutzer kann auch einen beliebigen anderen Referenz-Zeitpunkt t0 temporär vorgeben. Bevorzugt bleibt eine zuvor getroffene Auswahl eines Alarms erhalten. Weiterhin bleibt bevorzugt die Sequenz von Alarmen erhalten, deren Alarm-Beschreibungen in der Alarm-Beschreibungs-Abfolge 11 dargestellt werden.

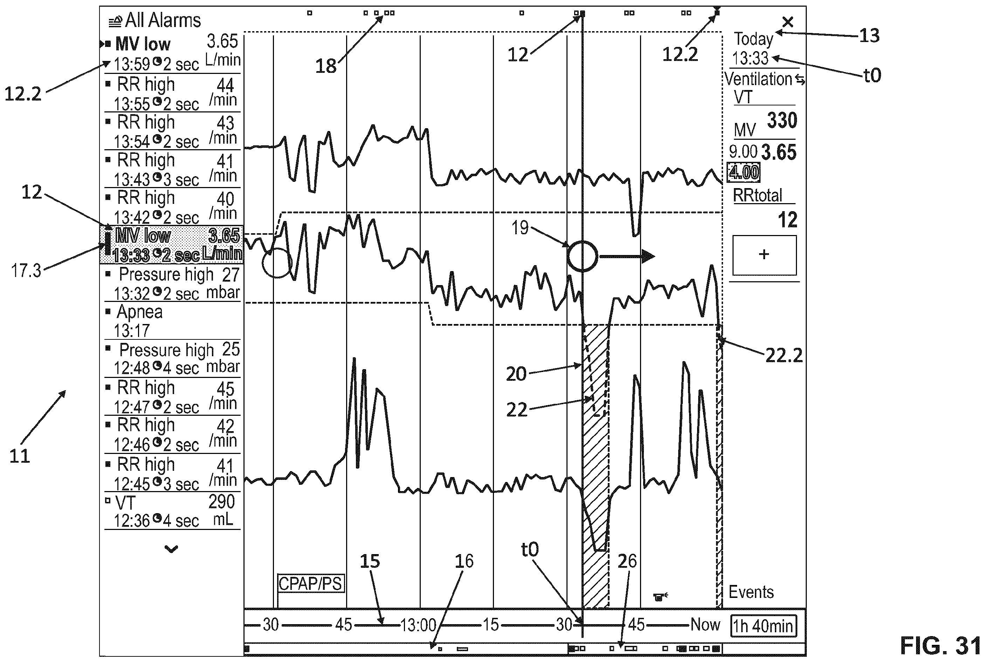

Die Festlegung eines temporären Referenz-Zeitpunkts t0 veranschaulicht Figur 31. Die bevorzugt vertikale Referenz-Zeitpunkt-Linie 20 fungiert als ein Cursor. Der Benutzer berührt diese Referenz-Zeitpunkt-Linie 20 und schiebt sie nach rechts und hält sie auf einen gewünschten Zeitpunkt, was in Figur 31 durch den Kreis 19 und den nach rechts zeigenden Pfeil angedeutet wird.

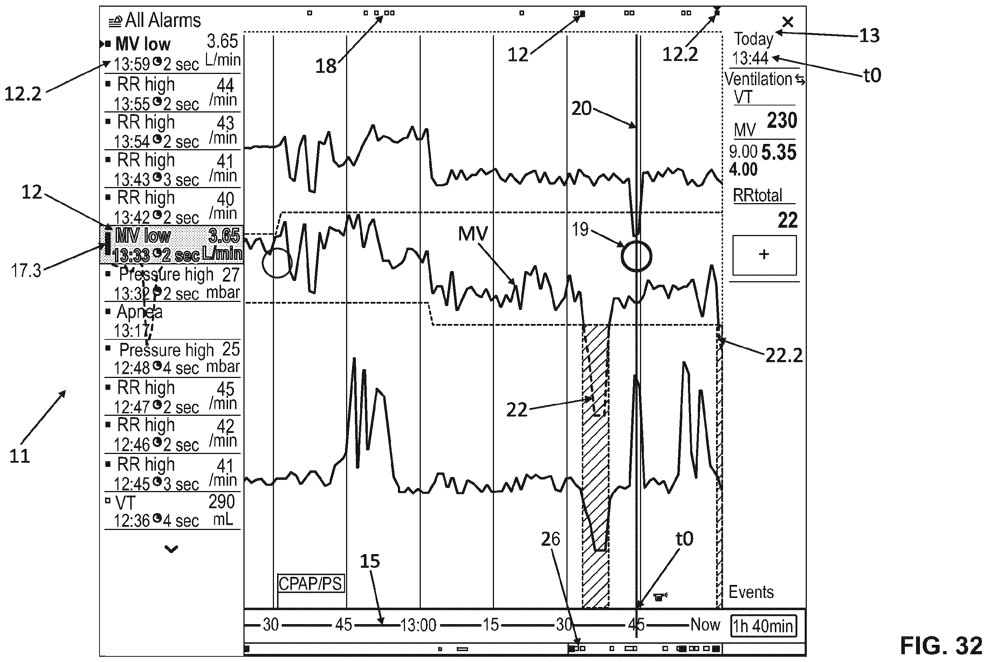

Als temporären Referenz-Zeitpunkt t0 hat der Benutzer durch das Verschieben und Halten den Zeitpunkt 13:44 Uhr vorgegeben, der nicht notwendigerweise der Zeitpunkt eines Alarms ist. Figur 32 illustriert mit dem Kreis 19, wo der Benutzer die Referenz-Zeitpunkt-Linie 20 hält. Außerdem zeigt Figur 32 die Reaktion: Im Signalwerte-Bereich 13 werden der geänderte Referenz-Zeitpunkt t0 sowie die Signalwerte zu diesem Referenz-Zeitpunkt t0 dargestellt. Das Referenz-Zeitfenster T1 sowie die Auswahl der Alarm-Art "MV low" und die Auswahl des Alarms 12 bleiben erhalten.

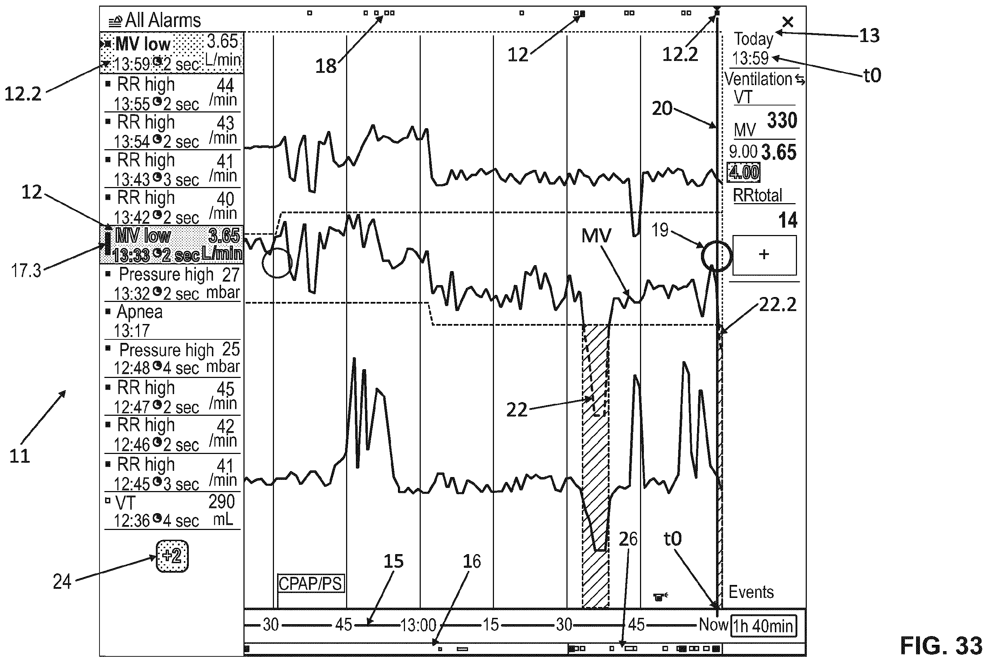

Möglich ist auch, als Referenz-Zeitpunkt t0 den Zeitpunkt eines Alarms festzulegen und dadurch diesen Alarm auszuwählen. Figur 33 zeigt ein Beispiel. Der Benutzer hat die Referenz-Zeitpunkt-Linie 20 weiter verschoben und als Referenz-Zeitpunkt t0 den Zeitpunkt 13:59 Uhr ausgewählt, das ist der Zeitpunkt des Alarms 12.2. Bevorzugt hält der Benutzer die Linie 20 für den Referenz-Zeitpunkt t0 auf dem Zeitpunkt des Alarms 12.2. Dieses Halten wird durch den Kreis 19 in Figur 33 angedeutet.

Folgende Reaktionen werden ausgelöst:
- In der Alarm-Beschreibungs-Abfolge 11 wird zusätzlich der ausgewählte Alarm 12.2 hervorgehoben dargestellt. Die zuvor getroffene Auswahl des Alarms 12 bleibt erhalten.
- In dem Signalwerte-Bereich 13 werden wiederum der Referenz-Zeitpunkt t0 (hier: 13:59 Uhr) sowie die Signalwerte zu diesem Referenz-Zeitpunkt t0 dargestellt.
- Das Referenz-Zeitfenster T1, die Referenz-Zeitachse 15 sowie die Darstellungen in der Alarm-Referenz-Abfolge 18 und dem Alarm-Referenz-Abschnitt 26 bleiben unverändert.

In einer Ausgestaltung bleibt dieser Referenz-Zeitpunkt t0 nur solange ausgewählt, wie der Benutzer die Referenz-Zeitpunkt-Linie 20 an der entsprechenden Stelle hält. Der Benutzer kann selbstverständlich die Referenz-Zeitpunkt-Linie 20 verschieben und halten, und die Darstellung wird entsprechend angepasst. Sobald der Benutzer nicht mehr die Referenz-Zeitpunkt-Linie 20 hält, sondern loslässt, so wird der Zeitpunkt des zuletzt ausgewählten Alarms wieder zum Referenz-Zeitpunkt t0. Dadurch wird wieder die in Figur 31 gezeigte Situation mit dem ausgewählten Alarm 12 hergestellt.

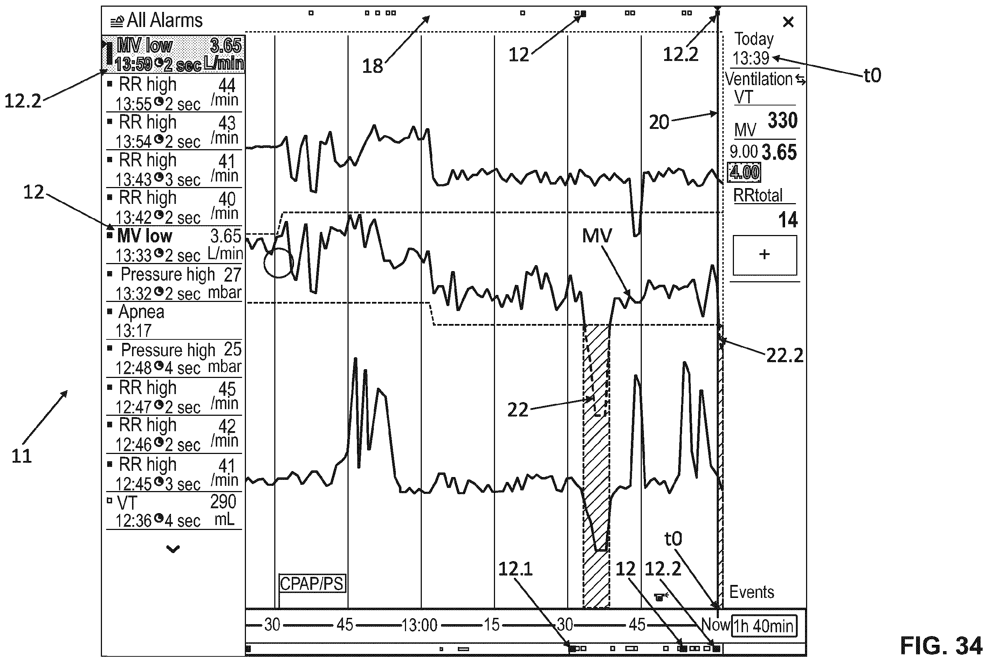

Der Benutzer kann auch die Referenz-Zeitpunkt-Linie 20 auf den Zeitpunkt des Alarms 12.2 verschieben und dort loslassen. Figur 34 zeigt die ausgelösten Reaktionen:
- Der Zeitpunkt des Alarms 12.2 ist der Referenz-Zeitpunkt t0.
- Anstelle des Alarms 12 wird der Alarm 12.2 ausgewählt und in der Alarm-Beschreibungs-Abfolge 11 als hervorgehoben gekennzeichnet.
- Der zuvor ausgewählte Alarm 12 wird in der Alarm-Beschreibungs-Abfolge 11 nicht hervorgehoben gekennzeichnet, ist aber von der gleichen Alarm-Art wie der nunmehr ausgewählte Alarm 12.2 und wird daher anders als die andersartigen Alarme dargestellt, nämlich mit fetter Schrift.

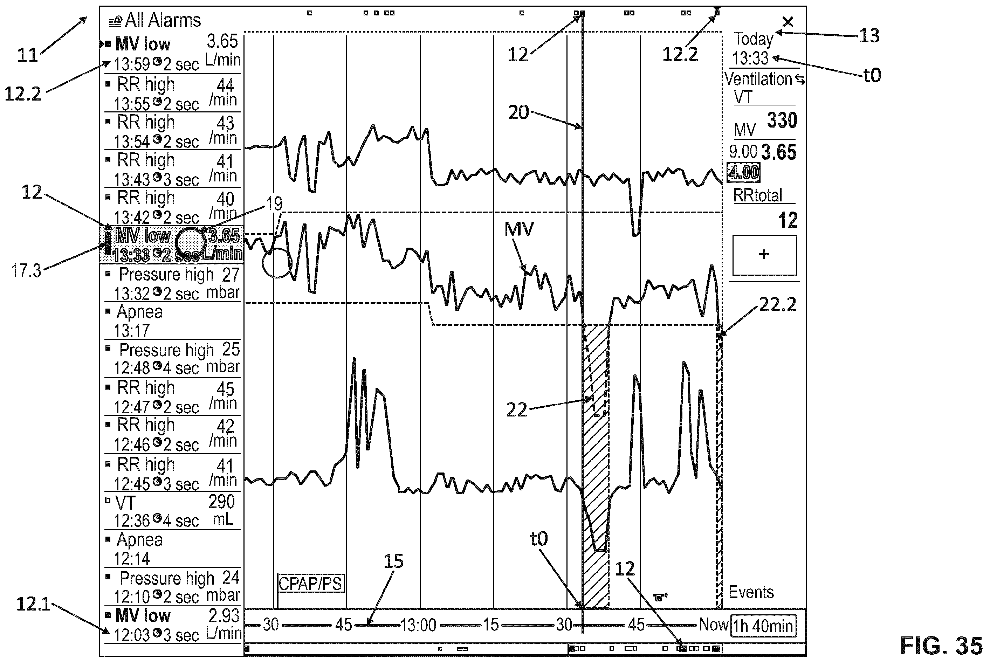

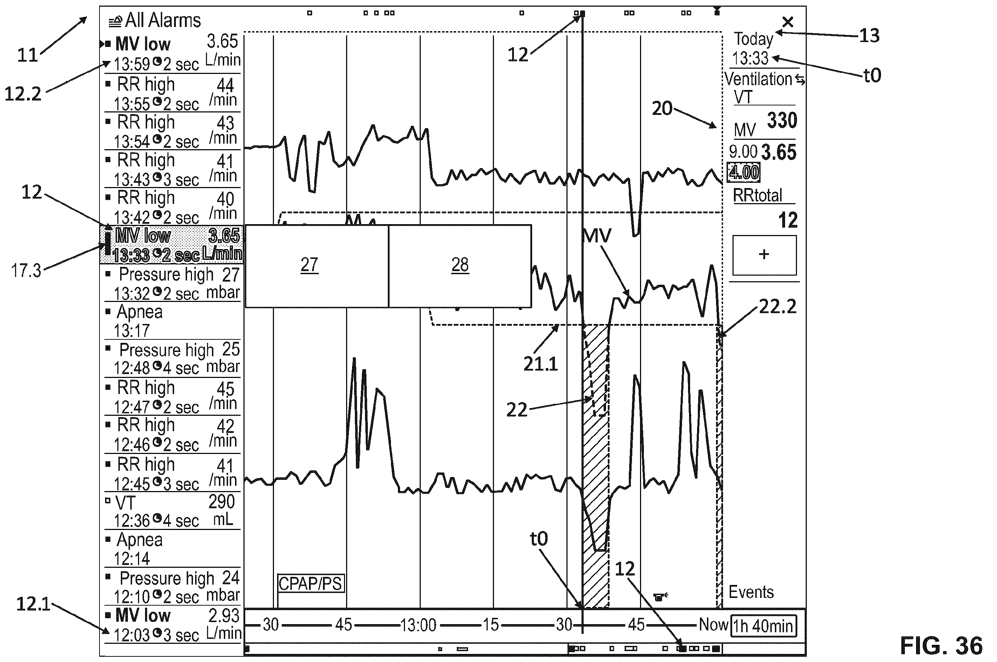

Der Benutzer kann sich Erläuterungen zu einem Alarm anzeigen lassen. Dies zeigen Figur 35 und Figur 36. Die in Figur 35 gezeigte Situation ist die gleiche, die auch in Figur 14 gezeigt wird. Der Benutzer hat den Alarm 12 ausgewählt, und dieser Alarm 12 wird hervorgehoben gekennzeichnet.

Der Benutzer wählt die Hervorhebung des Alarms 12 in der Alarm-Beschreibungs-Abfolge 11 aus, was in Figur 35 durch den Kreis 19 veranschaulicht wird. Als Reaktion werden zwei Textfenster 27 und 28 neben dem ausgewählten Alarm 12 angezeigt. Im Textfenster 27 wird eine Ursache oder Erläuterung für den Alarm 12 angezeigt - hier, dass der Wert die vorgegebene untere Schranke 21.1 unterschritten hat. Im Textfenster 28 werden mögliche Abhilfen angezeigt, um die Ursache zu beseitigen.

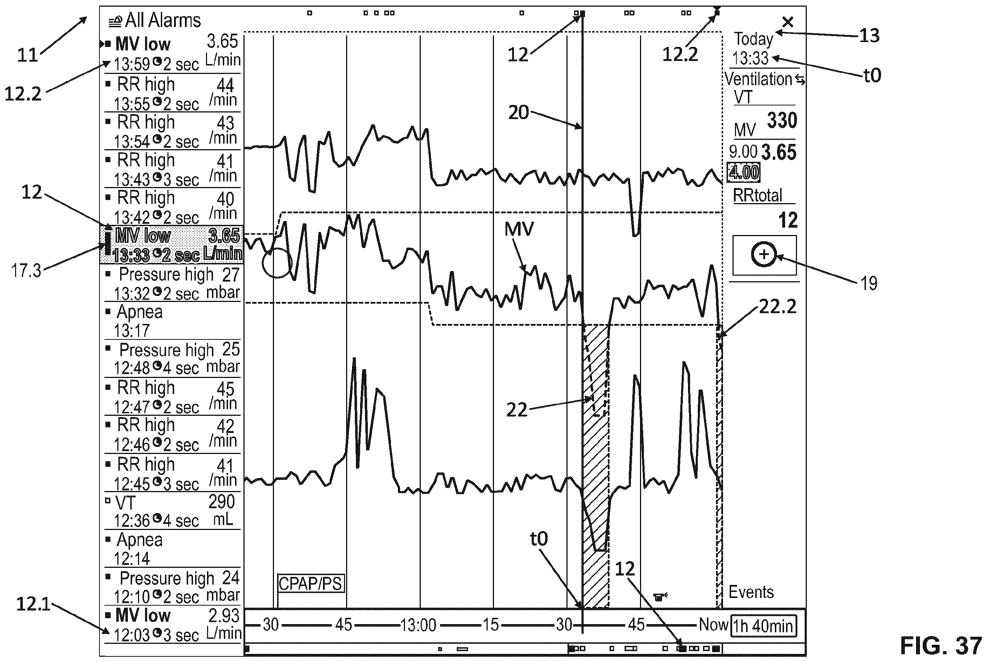

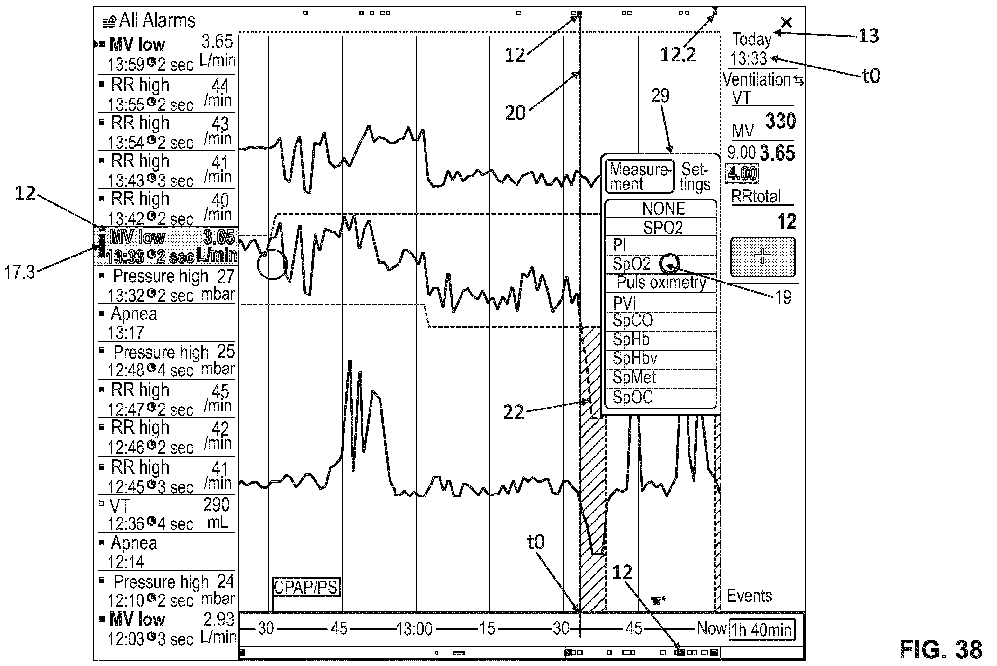

Der Benutzer kann sich die zeitlichen Verläufe von korrespondierenden oder sonstigen Signalen oder auch Größen, die sich am Beatmungsgerät 1 einstellen lassen, anzeigen lassen. Dies veranschaulichen Figur 37 und Figur 38.

Ausgangspunkt ist wiederum die Situation von Figur 14. Der Benutzer wählt ein Symbol ("+") in dem Signalwerte-Bereich 13 aus, was in Figur 37 durch den Kreis 19 dargestellt wird. Als Reaktion wird ein Auswahlmenü 29 angezeigt, welches zwei Reiter ("tabs") sowie die Namen von unterschiedlichen Signalen zur Auswahl anbietet, vgl. Figur 38. Der erste Reiter "Measurement" ermöglicht es, ein patientenbezogenes Signal auszuwählen, welches zusätzlich angezeigt werden soll. Der zweite Reiter "Settings" ermöglicht es, sich anzeigen zu lassen, welchen Wert eine zuvor ausgewählte und am Beatmungsgeräts 1 einstellbare Größe aktuell hat. Im Ausführungsbeispiel kann der Benutzer diesen Wert aber nicht in der gezeigten Benutzeroberfläche ändern, sondern nur auf andere Weise, bevorzugt direkt am Beatmungsgerät 1.

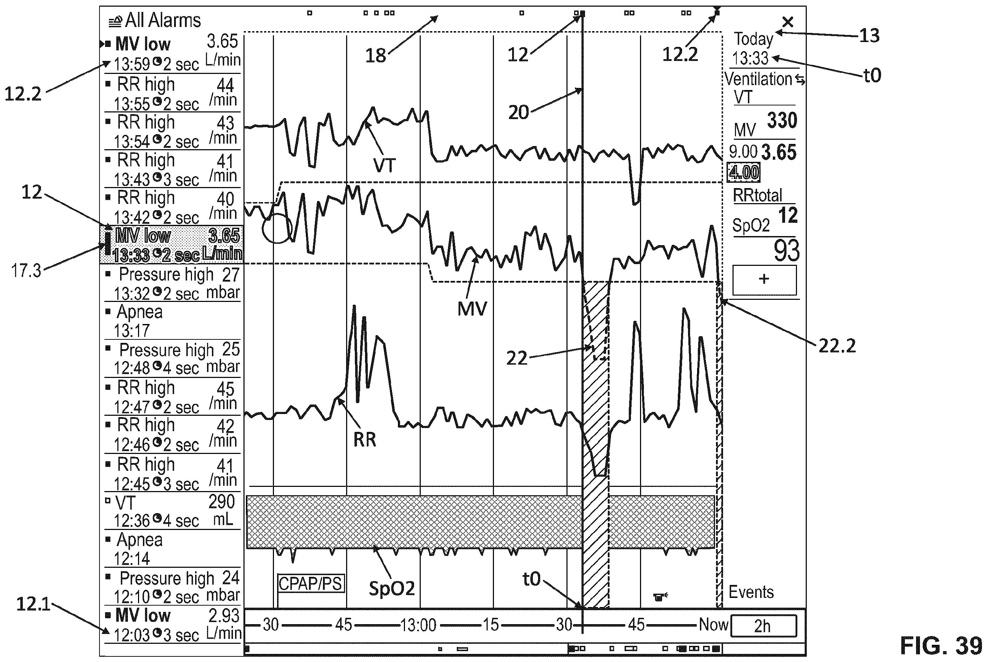

Im gezeigten Beispiel ist der erste Reiter aktiviert. Der Benutzer wählt beispielsweise das Signal SpO2 aus, was in Figur 38 durch den Kreis 19 dargestellt wird. Die Reaktionen auf diese Auswahl zeigt Figur 39:
- Zusätzlich zu den zeitlichen Verläufen der Signale VT, MV und RR wird der zeitliche Verlauf des Signals SpO2 angezeigt.
- Im Signalwerte-Bereich 13 wird zusätzlich der Wert 93 des Signals zum Referenz-Zeitpunkt t0, hier 13:33 Uhr, angezeigt.

Wiederum bleiben die Auswahl des Alarms 12, des Referenz-Zeitfensters T1 und des Referenz-Zeitpunkts t0 unverändert.

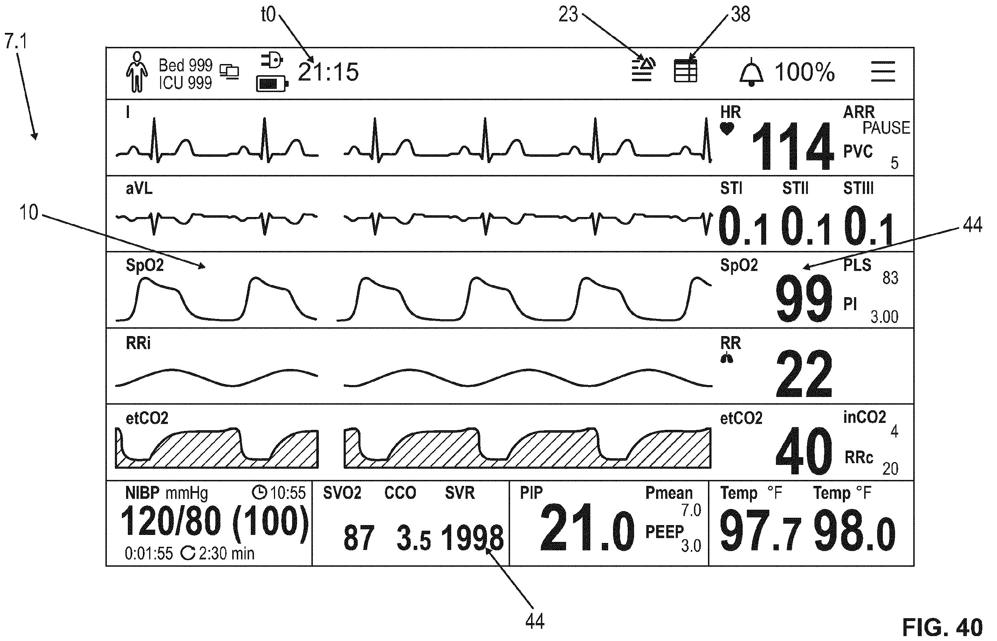

Figur 40 zeigt eine andere Ausgestaltung, die sich auf einem kleineren Bildschirm 7.1 realisieren lässt, und zwar in einer Situation, bevor ein Alarm ausgewählt wurde. Gleiche Bezugszeichen haben die gleiche Bedeutung wie oben. Als das aktuelle Referenz-Zeitfenster T1 werden die letzten 30 Minuten bis zum aktuellen Zeitpunkt t0 verwendet. Dargestellt werden zwei Bedienelemente, die sich durch Berührung aktivieren lassen:
- ein Bedienelement 23, um Alarm-Beschreibungen einzublenden oder ausblenden, und
- ein Bedienelement 38, welches einen im Folgenden beschriebenen Direkteinstieg ermöglicht.

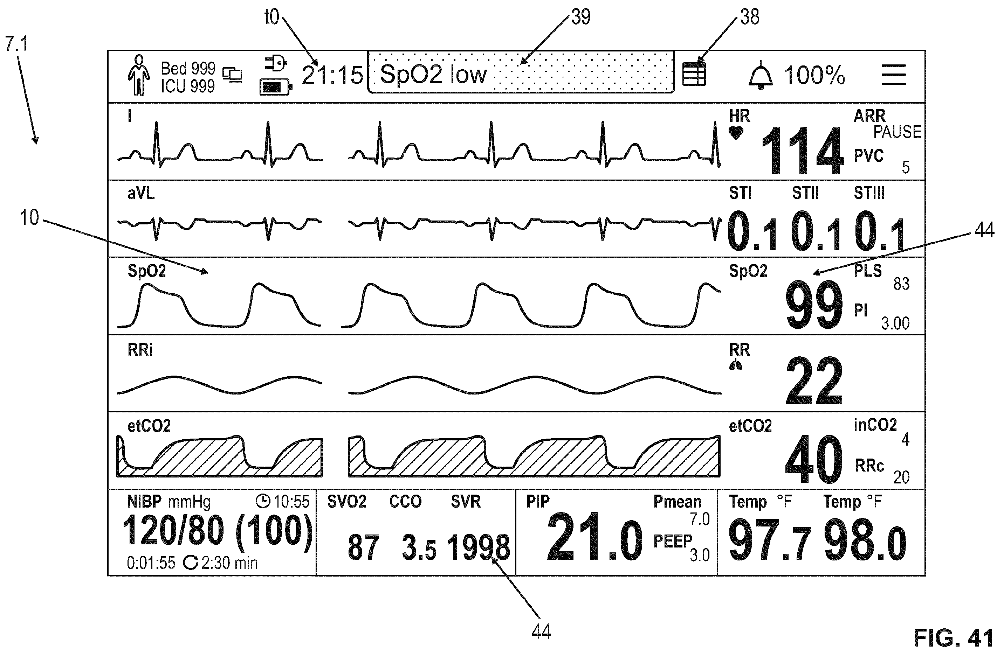

In der Situation von Figur 41 wird in dem Feld 39 ein Alarm angezeigt, der zum Referenz-Zeitpunkt t0 gleich 21:15 Uhr auftritt, nämlich der Alarm 40 der Alarm-Art "SpO2 low". In einem Bereich 44 werden die aktuellen Signalwerte der angezeigten Signalverläufe angezeigt.

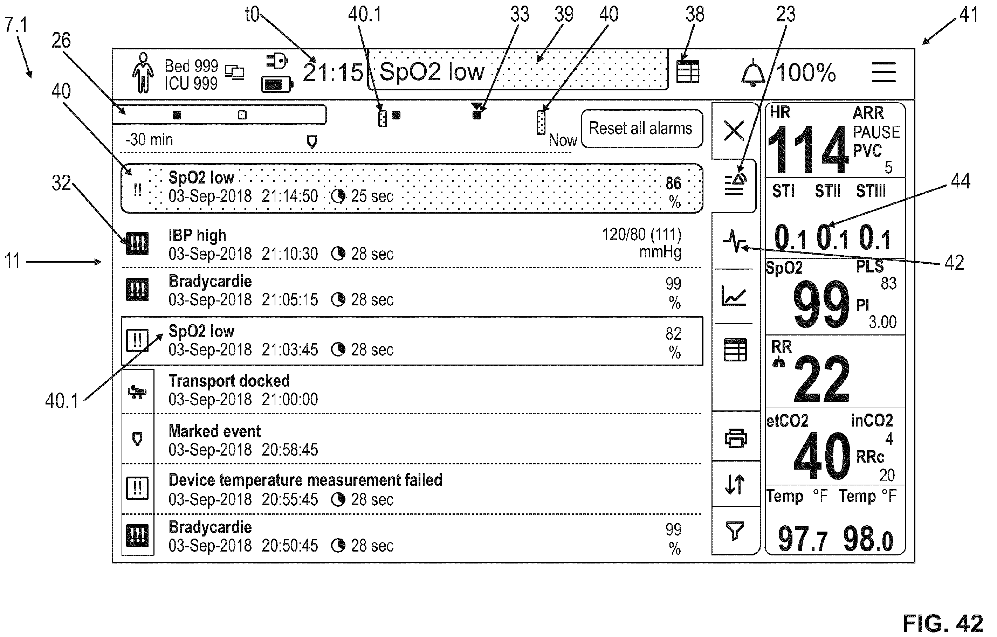

Der Benutzer betätigt das Bedienelement 38 oder das Feld 39. Figur 42 zeigt die Reaktionen auf diese Eingabe. In einer nunmehr eingeblendeten Spalte 41 auf dem Bildschirm 7.1 werden mehrere Bedienelemente angezeigt. Der Alarm-Referenz-Abschnitt 26 wird nunmehr angezeigt. Außerdem wird eine Alarm-Beschreibungs-Abfolge 11 in vertikaler Richtung angezeigt, der jüngste Alarm 40 an oberster Stelle. Um Platz auf dem Bildschirm 7.1 einzusparen, wird die Alarm-Beschreibungs-Abfolge 11 auf dem Platz angezeigt, der in der Situation von Figur 40 durch den Signalverlaufs-Bereich 10 eingenommen wurde. Der jüngste Alarm 40 trat um 21:14:50 Uhr auf.

Ein Alarm 40.1 der gleichen Alarm-Art "SpO2 low" trat um 21.03.45 Uhr auf. Der Benutzer hat diesen Alarm 40.1 ausgewählt, was durch die Umrandung angedeutet wird. Außerdem werden weitere Alarme gezeigt, z.B. "Bradycardie" um 21:05:15 Uhr, eine Ereignismarkierung, nämlich "Marked Event" um 20:58:45 Uhr, und eine besondere Handlung, die an dem Patienten P vorgenommen wird, beispielsweise ein Transport ("Transport docked", der um 21:00:00 Uhr beendet ist). Die Ereignismarkierung wurde von dem Benutzer manuell gesetzt, um damit eine besondere Situation festzuhalten. Der Benutzer kann später diese besondere Situation analysieren. In einer Ausgestaltung kann der Benutzer einen textlichen Kommentar zu dieser besonderen Situation eingeben (nicht dargestellt). Außerdem werden wiederum ein führendes Element 32 und ein geführtes Element 33 des Korrelationsanzeigers angezeigt, vgl. auch Figur 20 bis Figur 22.

Ein Benutzer kann den auf dem Bildschirm 7.1 dargestellten Ausschnitt der Alarm-Beschreibungs-Abfolge 11 nach oben und nach unten rollen ("scrollen"). Falls hierbei ein im aktuellen Alarm-Referenz-Abschnitt 26 nicht gezeigter Alarm an die Stelle des führenden Elements 32 gelangt, so werden das Referenz-Zeitfenster T1 und der Alarm-Referenz-Abschnitt 26 so angepasst, dass dieser Alarm nunmehr im Referenz-Zeitfenster T1 liegt. In einer Ausgestaltung bleibt auch hierbei der feinere Zeitmaßstab unverändert.

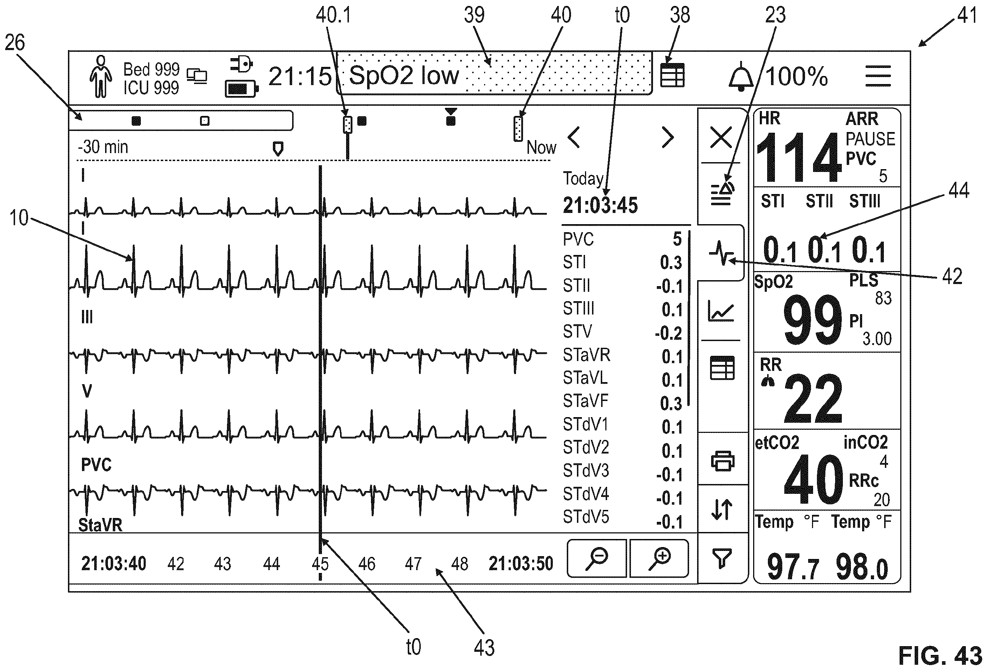

Im Beispiel von Figur 43 hat der Benutzer die Beschreibung des Alarms 40.1 in der Alarm-Beschreibungs-Abfolge 11 ausgewählt. Dadurch springt der Referenz-Zeitpunkt t0 auf den Zeitpunkt dieses Alarms 40.1, nämlich auf 21:03:45 Uhr. Außerdem hat der Benutzer das Bedienelement 42 in der Spalte 41 betätigt. Als Reaktion hierauf wird eine sogenannte Waveform-Snippet-Ansicht in dem Signalverlaufs-Bereich dargestellt. Unterhalb dieses Signalverlaufs-Bereichs wird eine Zeitachse 43 dargestellt. Der Referenz-Zeitpunkt t0 liegt in der Mitte dieser Zeitachse 43. Der Benutzer kann die aktuelle zeitliche Auflösung der Waveform-Snippet-Ansicht verändern, indem er die Bedienelemente mit "+" und "-" in den beiden dargestellten Lupen rechts neben der Zeitachse 43 betätigt. Über die Bedienelemente "<" und ">" rechts neben dem Alarm-Referenz-Abschnitt 26 kann der Benutzer zeitlich frühere und zeitlich spätere Alarme der gleichen Alarm-Art auswählen und dadurch sowohl den Referenz-Zeitpunkt t0 als auch das Referenz-Zeitfenster T1 verschieben.

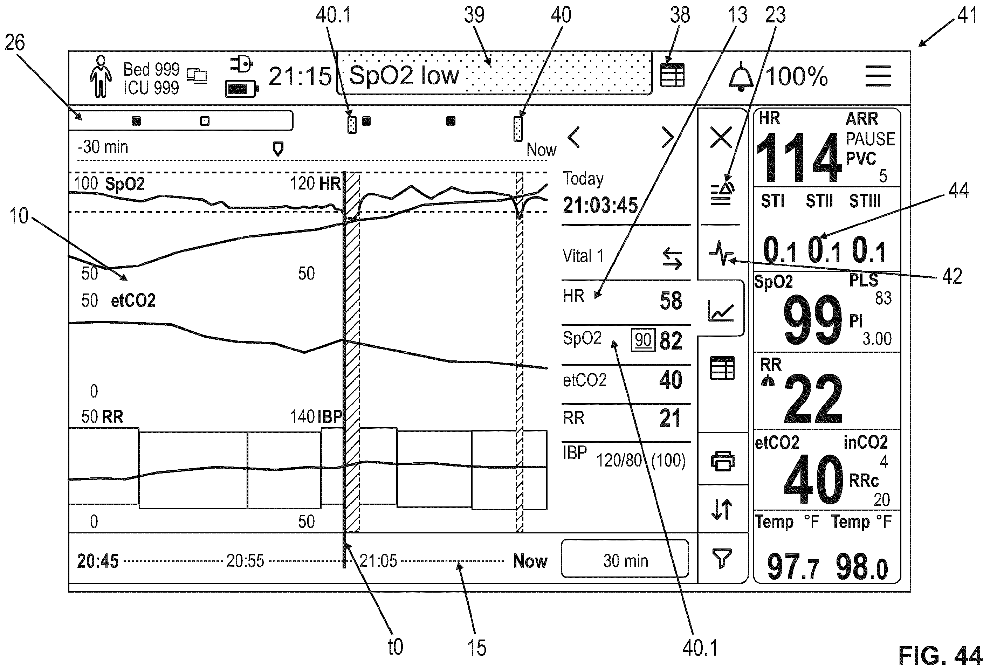

Figur 44 zeigt, wie die Verläufe von unterschiedlichen Signalen als Trendverläufe dargestellt werden, wobei die Signale in einer Ausgestaltung zuvor rechnerisch geglättet werden. Die Darstellung der Trendverläufe hängt von der gewählten zeitlichen Auflösung, der Abtastfrequenz und der Anzahl der darzustellenden Bildpunkte (Pixel) ab. Die Signalverläufe beziehen sich auf das Referenz-Zeitfenster T1, das eine halbe Stunde zurückreicht. Unter dem zentralen Signalverlaufs-Bereich 10 wird die Referenz-Zeitachse 15 für das Referenz-Zeitfenster T1 angezeigt.

Außerdem werden in der Situation, die in Figur 44 gezeigt wird, in einem Bereich 13 die Werte der gezeigten Signale zum Referenz-Zeitpunkt t0 gleich 21:03:45 Uhr angezeigt. Der Wert für SpO2 zum Referenz-Zeitpunkt t0 war zu niedrig (Alarm). Bei der Darstellung des Alarms 40.1 in dem Signalwerte-Bereich 13 wird daher neben dem Signalwert SpO2 = 82 zum Referenz-Zeitpunkt t0 auch die untere Schranke des Sollbereichs für SpO2, hier der Wert SpO2 = 90, angezeigt, wobei die Zahl für die untere Schranke unterstrichen dargestellt ist.

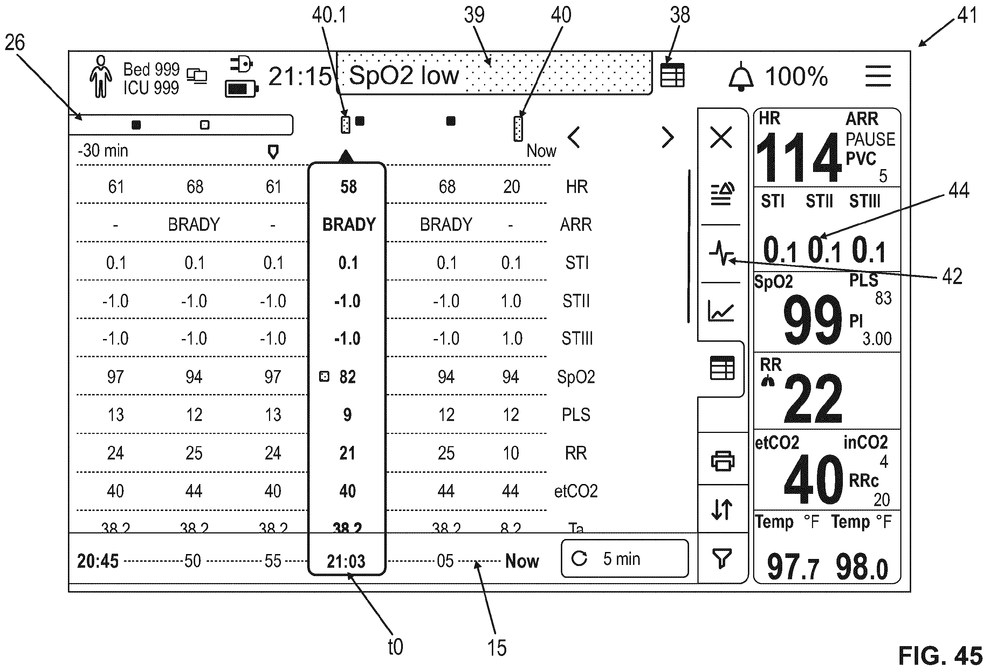

In der Darstellung, die in Figur 45 gezeigt wird, werden die Signalverläufe durch Zahlwerte angezeigt. Die Darstellung bezieht sich wiederum auf das Referenz-Zeitfenster T1, welches die letzten 30 Minuten vor dem aktuellen Zeitpunkt ("Now") umfasst. Der Referenz-Zeitpunkt t0 ist wiederum gleich 21:03:45 Uhr. Weitere Signalwerte werden numerisch in Zeitschritten von 5 Minuten angezeigt. Dieses Intervall lässt sich verändern, indem der Benutzer die Schaltfläche mit der Beschriftung "5 min" berührt.

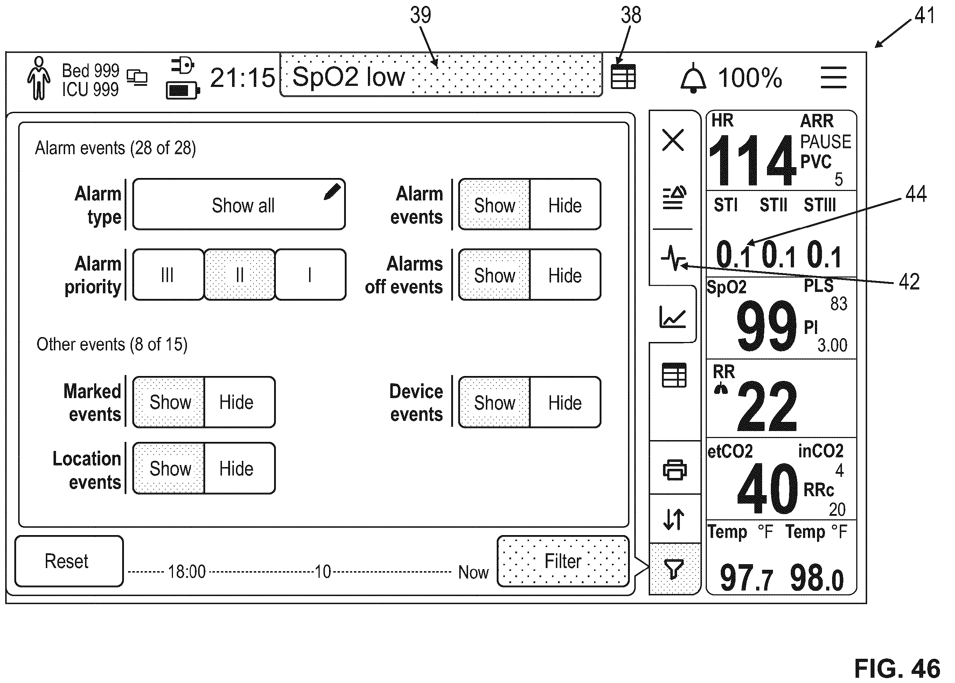

Figur 46 veranschaulicht, wie der Benutzer filtern kann, welche Alarme dargestellt werden. Beispielsweise kann der Benutzer folgende Filter setzen und wieder aufheben:
- Nur Alarme einer bestimmten Alarm-Art werden angezeigt, beispielsweise nur die Alarme der Alarm-Art "SpO2 low".
- Nur Alarme einer bestimmten Priorität werden angezeigt, beispielsweise nur Alarme, deren Alarm-Art die Priorität "mittel" oder höher oder auch nur "mittel" zugeordnet ist.
- Alarme werden wahlweise angezeigt oder nicht angezeigt.
- Die Überwachung von Alarmen wird eingeschaltet oder ausgeschaltet.
- Wahlweise werden nur patientenbezogene Alarme oder zusätzlich geräteseitige Alarme angezeigt.
- Ereignismarkierungen, die der Benutzer manuell hinzufügt, werden wahlweise angezeigt oder nicht angezeigt. Ein Beispiel für eine solche Ereignismarkierung ist das "Marked event" um 20:58:45 Uhr in Figur 42.
- Besondere Handlungen am Patienten P werden wahlweise angezeigt oder nicht angezeigt. Ein Beispiel ist der Patiententransport ("Transport docked"), der um 21:00:00 Uhr beendet ist, vgl. Figur 42.

Der gesetzte Filter wirkt sich sowohl darauf aus, welche Alarme im Alarm-Referenz-Abschnitt 26 gezeigt werden, als auch darauf aus, welche Alarme in der Alarm-Beschreibungs-Abfolge 11 beschrieben werden. Dargestellt wird außerdem, wie viele Alarme aktuell aufgetreten sind (28 Stück) und welcher Alarm aktuell im Feld 39 angezeigt wird (der zeitlich neueste Alarm 40 der Alarm-Art "SpO2 low").

Die bislang beschriebene Ausgestaltung bezieht sich auf ein medizinisches Gerät 1, welches eine eigene Signalverarbeitungseinheit 5 und eine eigene Ausgabeeinheit 7 umfasst. Die Signalverarbeitungseinheit 5 bewirkt, dass die Informationen über die Alarme so wie oben beschrieben auf dieser Ausgabeeinheit 7 dargestellt werden. Im Folgenden wird beispielhaft ein Datennetzwerk von mehreren medizinischen Geräten beschrieben.

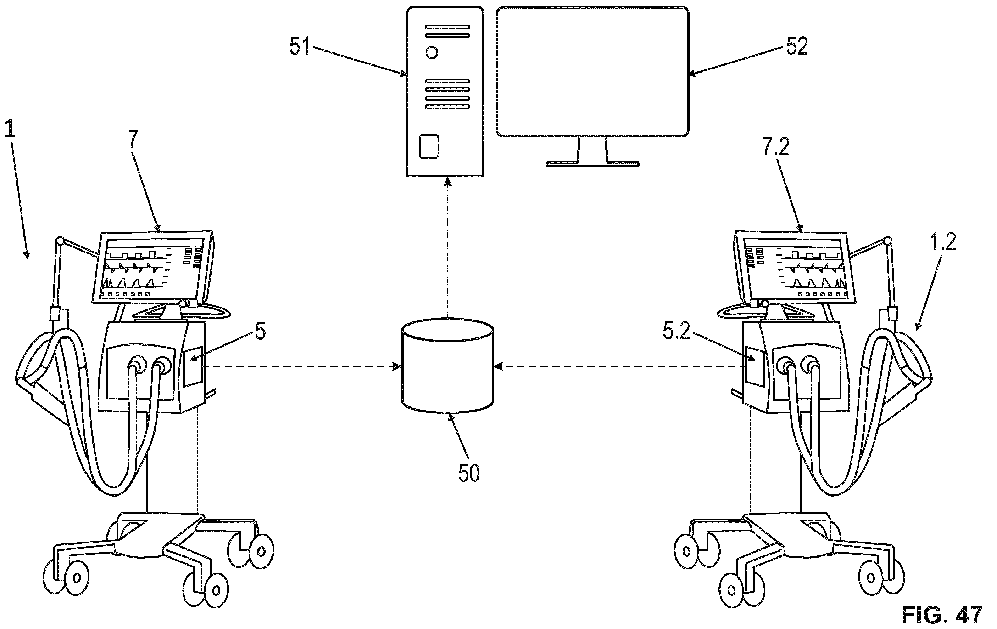

Figur 47 zeigt beispielhaft ein System, welches die erfindungsgemäße Anordnung umfasst. Dieses System umfasst
- das Beatmungsgerät 1 mit der Ausgabeeinheit 7 und der Signalverarbeitungseinheit 5 von Figur 1,
- ein weiteres Beatmungsgerät 1.2 mit einer weiteren Ausgabeeinheit 7.2 und einer weiteren Signalverarbeitungseinheit 5.2, wobei das weitere Beatmungsgerät 1.2 so wie das Beatmungsgerät 1 von Figur 1 aufgebaut sein kann,
- Patienten-Sensoren, die Messwerte an das Beatmungsgerät 1 oder an das Beatmungsgerät 1.2 zu übermitteln vermögen,
- einen zentralen Datenspeicher 50, auf den die beiden Signalverarbeitungseinheiten 5 und 5.2 wenigstens zeitweise Schreibzugriff haben,
- eine zentrale Signalverarbeitungseinheit 51, die wenigstens zeitweise Lesezugriff auf den zentralen Datenspeicher 50 hat, und
- eine zentrale Ausgabeeinheit 52.

Dieses System kann auch weitere Beatmungsgeräte und / oder andere medizinische Geräte umfassen.

Die beiden Beatmungsgeräte 1 und 1.2 sowie die zentrale Signalverarbeitungseinheit 51 sind also durch ein Datennetzwerk miteinander verbunden. Die zentrale Signalverarbeitungseinheit 51 steuert die zentrale Ausgabeeinheit 52 an.

Beide lokalen Signalverarbeitungseinheiten 5 und 5.2 empfangen so wie oben beschrieben Messwerte von den Patienten-Sensoren der Beatmungsgeräte 1 bzw. 1.2, erzeugen Signale, prüfen, ob vorgegebene Alarm-Kriterien erfüllt sind, detektieren Alarme und steuern die lokalen Ausgabeeinheit 7 bzw. 7.2 an. Diese Alarme beziehen sich auf das Beatmungsgerät 1 bzw. 1.2. Die lokalen Signalverarbeitungseinheiten 5 und 5.2 schreiben Informationen über die detektierten Alarme in den zentralen Datenspeicher 50. Die zentrale Signalverarbeitungseinheit 51 liest diesen zentralen Datenspeicher 50 aus, beispielsweise mit einer vorgegebenen Abtast-Frequenz. Die zentrale Signalverarbeitungseinheit 51 wertet die Informationen aus, die sie aus dem zentralen Datenspeicher 50 ausgelesen hat, und bewirkt, dass auf der Ausgabeeinheit 52 Alarme von beiden Beatmungsgeräten 1 und 1.2 so wie oben beschrieben angezeigt werden.

In einer Ausgestaltung kann ein Benutzer sich wahlweise Alarme vom Beatmungsgerät 1 oder vom Beatmungsgerät 1.2 anzeigen lassen. In einer anderen Ausgestaltung werden Alarme von beiden Beatmungsgeräten 1 und 1.2 gleichzeitig auf der zentralen Ausgabeeinheit 52 angezeigt.

### BEZUGSZEICHENLISTE

- 1: Beatmungsgerät, umfasst das Verbindungsstück 3, die Signalverarbeitungseinheit 5 und die Ausgabeeinheit 7
- 1.2: weiteres Beatmungsgerät, umfasst die weitere Signalverarbeitungseinheit 5.1 und die weitere Ausgabeeinheit 7.2
- 2.1.1, ..., 2.2.2: Messelektroden, die auf der Haut des Patienten P positioniert sind, fungieren als Patienten-Sensoren
- 3: Verbindungsstück vor dem Mund des Patienten P
- 4: optischer Sensor, misst den Lungen-Füllstand des Patienten P
- 5: Signalverarbeitungseinheit des Beatmungsgeräts 1, steuert die Ausgabeeinheit 7 an
- 5.2: Signalverarbeitungseinheit des weiteren Beatmungsgeräts 1.2, steuert die Ausgabeeinheit 7.2 an
- 6: pneumatischer Sensor in der Speiseröhre Sp des Patienten P
- 7: Ausgabeeinheit des Beatmungsgeräts 1, umfasst einen Bildschirm, wird von der Signalverarbeitungseinheit 5 angesteuert
- 7.1: Ausgabeeinheit mit kleinerem Bildschirm
- 7.2: Ausgabeeinheit des weiteren Beatmungsgeräts 1.2, umfasst einen Bildschirm, wird von der Signalverarbeitungseinheit 5.2 angesteuert
- 10: zentraler Signalverlaufs-Bereich des Bildschirms 7, in dem die zeitlichen Verläufe der drei Signale VT, MV und RR und weiterer Signale dargestellt werden, bezieht sich auf das Referenz-Zeitfenster T1
- 11: Alarm-Beschreibungs-Abfolge des Bildschirms 7, in welchem in vertikaler Richtung eine Abfolge von Alarm-Beschreibungen dargestellt werden, der jüngste Alarm an oberster Stelle
- 12: ausgewählter Alarm der Alarm-Art "MV low" zum Zeitpunkt 13:33 Uhr
- 12.1: Alarm der Alarm-Art "MV low" zum Zeitpunkt 12:03 Uhr
- 12.2: Alarm der Alarm-Art "MV low" zum Zeitpunkt 13:59 Uhr
- 13: Signalwerte-Bereich des Bildschirms 7, in dem der Referenz-Zeitpunkt t0 sowie die Werte der gezeigten Signale zum Referenz-Zeitpunkt t0 angezeigt werden
- 14: Alarm-Übersichts-Darstellung im unteren Bereich des Bildschirms 7, zeigt die Referenz-Zeitachse 15 und die Alarm-Gesamt-Abfolge 16 mit dem Alarm-Referenz-Abschnitt 26
- 15: dargestellte Zeitachse für das Referenz-Zeitfenster T1 in der Alarm-Übersichts-Darstellung 14
- 16: Alarm-Gesamt-Abfolge in der Alarm-Übersichts-Darstellung 14, in der dargestellt wird, zu welchen Zeitpunkten Alarme von welchen Alarm-Arten detektiert wurden, umfasst den Alarm-Referenz-Abschnitt 26 für das Referenz-Zeitfenster T1 der Referenz-Referenz-Zeitachse 15
- 17.1: Symbol für die Relevanz "hoch", ist z.B. der Alarm-Art "MV low" zugeordnet
- 17.2: Symbol für die Relevanz "mittel", ist z.B. der Alarm-Art "VT" zugeordnet
- 17.3: Symbol für die Relevanz "hoch" des ausgewählten Alarms 12
- 18: Alarm-Referenz-Abfolge auf dem Bildschirm 7, zeigt eine Abfolge der Symbole der Alarm-Arten, die im Referenz-Zeitfenster T1 aufgetreten sind
- 19: Bereich auf dem Bildschirm 7, den ein Benutzer berührt und dadurch einen Alarm auswählt oder eine sonstige Benutzer-Interaktion durchführt
- 20: Referenz-Zeitpunkt-Linie, die den Referenz-Zeitpunkt t0 bezüglich der Referenz-Zeitachse 15 veranschaulicht, fungiert als Cursor
- 21.1,: zeitlich veränderliche untere bzw. obere Schranke des Sollbereichs für
- 22.2: das Signal MV
- 22,: Abschnitte des Signals MV, die unterhalb der unteren Schranke 21.1
- 22.1,: liegen und die Alarme 12, 12.1, ... ausgelöst haben
- 23: Bedienelement für das Einblenden oder Ausblenden von Alarm-Beschreibungen
- 24: Anzeige, wie viele weitere Alarme zu derselben Alarm-Art wie der aktuell ausgewählte Alarm gehören und aktuell in der Alarm-Beschreibungs-Abfolge 11 nicht angezeigt werden
- 26: Alarm-Referenz-Abschnitt der Alarm-Gesamt-Abfolge 16, der sich auf das Referenz-Zeitfenster T1 der Referenz-Referenz-Zeitachse 15 bezieht, zeigt diejenigen Alarme der Alarm-Gesamt-Abfolge 16, die im Referenz-Zeitfenster T1 aufgetreten sind, stellt die Positionierungs-Darstellung für das Referenz-Zeitfenster T1 bereit
- 27: Textfeld, welches eine Ursache für einen Alarm erläutert
- 28: Textfeld, welches mögliche Abhilfen zur Beseitigung der im Textfeld 27 beschriebene Ursache eines Alarms erläutert
- 29: Auswahlmenü zur Auswahl eines zusätzlichen patientenbezogenen oder gerätetechnischen Signals, dessen Verlauf angezeigt werden soll
- 30: Zeitdauer-Fenster, zeigt die Dauer des Referenz-Zeitfensters T1 an
- 31: Alarm-Beschreibung für den Alarm 12
- 32: führendes Element des Korrelationsanzeigers, zeigt auf die oberste Alarm-Beschreibung in der Alarm-Beschreibungs-Abfolge 11
- 33: geführtes Element des Korrelationsanzeigers, zeigt auf den entsprechenden Alarm in der Alarm-Referenz-Abfolge 18
- 34: Alarm der Alarm-Art "RR high" zum Zeitpunkt 13:55 Uhr
- 35: Alarm der Alarm-Art "Apnea" zum Zeitpunkt 13:17 Uhr
- 37: DV-Maus
- 38: Bedienelement, welches einen Direkteinstieg ermöglicht
- 39: Feld, in dem ein Alarm angezeigt wird
- 40: Alarm der Alarm-Art "SpO2 low"
- 41: Spalte auf dem Bildschirm 7 mit mehreren Bedienelementen
- 42: Bedienelement in der Spalte 41, um sich die Waveform-Snippet-Ansicht anzeigen zu lassen
- 43: Zeitachse für die Waveform-Snippet-Ansicht
- 44: Bereich, in dem die aktuellen Signalwerte der angezeigten Signalverläufe angezeigt werden
- 50: zentraler Datenspeicher, in welchen die lokalen Signalverarbeitungseinheiten 5 und 5.2 der medizinischen Geräte 1, 1.2 Informationen über die detektierten Alarme einschreiben und auf welche die zentrale Signalverarbeitungseinheit 51 wenigstens zeitweise Lesezugriff hat
- 51: zentrale Signalverarbeitungseinheit, hat Lesezugriff auf den Datenspeicher 50, steuert die zentrale Ausgabeeinheit 52 an
- 52: zentrale Ausgabeeinheit, wird von der zentralen Signalverarbeitungseinheit 51 angesteuert
- HR: Signal, welches die Herzschlagfrequenz kennzeichnet - Anzahl der R-Spitzen pro Minute, im zentralen Signalverlaufs-Bereich 10 dargestellt
- LR: Listen-Richtung, in welche sich die Alarm-Beschreibungs-Abfolge 11 erstreckt, steht senkrecht auf der Zeitachsen-Darstellungs-Richtung ZR
- MV: Signal ("minute volume") - Menge der in die Lunge zugeführten Atemluft in [Liter/min], im zentralen Signalverlaufs-Bereich 10 dargestellt
- RR: Atemfrequenz (respiratory rate) des Patienten P, im zentralen Signalverlaufs-Bereich 10 dargestellt
- MV: Alarm-Art: Wert des Signals MV unterhalb der unteren Schranke 21.1 low
- P: Patient, der vom Beatmungsgerät 1 künstlich beatmet wird
- Sp: Speiseröhre des Patienten P
- SpO2: Sauerstoffgehalt in Blut, nach einer entsprechenden Benutzereingabe im zentralen Signalverlaufs-Bereich 10 dargestellt
- SR: Schreib-Richtung der Alarm-Beschreibung 31, steht senkrecht auf der Listen-Richtung LR
- T: Gesamt-Zeitraum, auf den sich die Alarm-Gesamt-Abfolge 16 bezieht, umfasst das Referenz-Zeitfenster T1
- t0: veränderbarer Referenz-Zeitpunkt, den die Referenz-Zeitpunkt-Linie 20 veranschaulicht, ist der aktuelle Zeitpunkt ("now") oder der Zeitpunkt eines ausgewählten Alarms oder wird vom Benutzer direkt vorgegeben
- T1: Referenz-Zeitfenster, auf welches sich die dargestellten Alarme des Alarm-Referenz-Abschnitts 26 sowie der Alarm-Referenz-Abfolge 18 beziehen, ist ein Abschnitt des Gesamt-Zeitraums T
- VT: Signal "Ventilation" - Menge von Atemluft in [ml], die bei einem einmaligen Atmen in die Lunge fließt, im zentralen Signalverlaufs-Bereich 10 dargestellt
- ZR: Zeitachsen-Darstellungs-Richtung, in welche sich die Referenz-Zeitachse 15 und die Alarm-Gesamt-Abfolge 16 und der Alarm-Referenz-Abschnitt 26 erstreckt, steht senkrecht auf der Listen-Richtung LR
- Zw: Zwerchfell des Patienten P

## Patentansprüche

1. Anordnung umfassend
- ein medizinisches Gerät (1, 1.2), insbesondere ein Beatmungsgerät oder ein Anästhesiegerät,
- mindestens einen Patienten-Sensor, bevorzugt mehrere Patienten-Sensoren (2.1.1 bis 2.2.2, 3, 4, 6),
- eine Signalverarbeitungseinheit (5, 5.2, 51) und
- eine Ausgabeeinheit (7, 7.1, 52) zur visuellen Ausgabe von Informationen an einen Benutzer,
wobei der oder jeder Patienten-Sensor (2.1.1 bis 2.2.2, 3, 4, 6) jeweils mindestens eine an oder in einem mit dem medizinischen Gerät (1, 1.2) verbundenen Patienten (P) auftretende Größe zu messen vermag,
wobei die Signalverarbeitungseinheit (5, 5.2, 51) dazu ausgestaltet ist, automatisch
- Messwerte von dem oder mindestens einem Patienten-Sensor (2.1.1 bis 2.2.2, 3, 4, 6) zu empfangen,
- durch Auswertung von empfangenen Messwerten mindestens ein Signal (VT, MV, RR, SpO2) zu erzeugen,
- zu entscheiden, ob mindestens ein vorgegebenes Alarm-Kriterium (MV low, SPO2 low) erfüllt ist,
wobei das oder jedes vorgegebene Alarm-Kriterium (MV low, SPO2 low) sich auf das oder jeweils mindestens ein von der Signalverarbeitungseinheit (5, 5.2, 51) erzeugbares Signal (VT, MV, RR, SpO2) bezieht,
- als Reaktion auf eine Entscheidung, dass das oder ein Alarm-Kriterium (MV low, SPO2 low) erfüllt ist, einen Alarm (12, 12.1, 12.2, 34, 35) sowie einen Zeitpunkt, an dem dieser Alarm aufgetreten ist, zu detektieren,
wobei das Erfülltsein des Alarm-Kriteriums (MV low, SPO2 low) das Auftreten des Alarms bedeutet, und
- die Ausgabeeinheit (7, 7.1, 52) anzusteuern,
wobei die Signalverarbeitungseinheit (5, 5.2, 51) weiterhin dazu ausgestaltet ist, die Ausgabeeinheit (7, 7.1, 52) so anzusteuern, dass die angesteuerte Ausgabeeinheit (7, 7.1, 52) wenigstens zeitweise folgendes gleichzeitig darstellt:
- eine Alarm-Gesamt-Abfolge (16), das ist eine Darstellung einer zeitlichen Abfolge von Alarmen, die in einem vorgegebenen Gesamt-Zeitraum (T) detektiert wurden,
bevorzugt eine Darstellung einer zeitlichen Abfolge von allen Alarmen im Gesamt-Zeitraum (T),
wobei die Darstellung der Alarm-Gesamt-Abfolge (16) sich in einer Zeitachsen-Darstellungs-Richtung (ZR) erstreckt,
- einen Alarm-Referenz-Abschnitt (26), das ist eine Darstellung derjenigen in der Alarm-Gesamt-Abfolge (16) gezeigten Alarme, die in einem vorgegebenen Referenz-Zeitfenster (T1) detektiert wurden,
bevorzugt eine Darstellung aller im Referenz-Zeitfenster (T1) detektierten Alarmen,
wobei das Referenz-Zeitfenster (T1) ein Ausschnitt des Gesamt-Zeitraums (T) ist, und
- eine Signalverlaufs-Darstellung (10) oder eine Alarm-Referenz-Abfolge (18) oder sowohl eine Signalverlaufs-Darstellung (10) als auch eine Alarm-Referenz-Abfolge (18),
wobei die Darstellung des Alarm-Referenz-Abschnitts (26) sich in die Zeitachsen-Darstellungs-Richtung (ZR) erstreckt,
wobei der Alarm-Referenz-Abschnitt (26) eine Positionierungs-Darstellung bereitstellt, das ist eine Darstellung der zeitlichen Positionierung des Referenz-Zeitfensters (T1) relativ zum Gesamt-Zeitraum (T),
wobei die Signalverlaufs-Darstellung (10) eine Darstellung des jeweiligen zeitlichen Verlaufs des oder mindestens eines erzeugten Signals (VT, MV, RR, SpO2) im Referenz-Zeitfenster (T1) ist,
wobei die Alarm-Referenz-Abfolge (18) eine Darstellung einer Abfolge von Alarmen ist, die im Referenz-Zeitfenster (T1) aufgetreten sind, bevorzugt von allen Alarmen, die im Referenz-Zeitfenster (T1) aufgetreten sind,
wobei die Signalverlaufs-Darstellung (10) und die Alarm-Referenz-Abfolge (18) sich in die Zeitachsen-Darstellungs-Richtung (ZR) erstrecken und
wobei
- der Zeitmaßstab für die Signalverlaufs-Darstellung (10) und der Zeitmaßstab für die Alarm-Referenz-Abfolge (18) feiner sind als
- der Zeitmaßstab für die Alarm-Gesamt-Abfolge (16) und der Zeitmaßstab für den Alarm-Referenz-Abschnitt (26).

2. Anordnung nach Anspruch 1,
**dadurch gekennzeichnet, dass**
mindestens zwei unterschiedliche Alarm-Arten (MV low, RR high) vorgegeben sind,
wobei jede Alarm-Art durch jeweils ein vorgegebenes Alarm-Kriterium (MV low, SPO2 low) definiert ist,
wobei die Signalverarbeitungseinheit (5, 5.2, 51) dazu ausgestaltet ist, die Auswahl eines Alarms (12) durch einen Benutzer zu erfassen und
wobei die Signalverarbeitungseinheit (5, 5.2, 51) weiterhin dazu ausgestaltet ist, nach Auswahl eines Alarms (12) die Ausgabeeinheit (7, 7.1, 52) so anzusteuern, dass die angesteuerte Ausgabeeinheit (7, 7.1, 52) in der Alarm-Gesamt-Abfolge (16) und / oder in dem Alarm-Referenz-Abschnitt (26) und / oder in der Alarm-Referenz-Abfolge (18)
jeden weiteren Alarm (12.1, 12.2), der zu der gleichen Alarm-Art (MV low) wie der ausgewählte Alarm (12) gehört, im Vergleich zu den übrigen dargestellten Alarmen (34, 35) hervorgehoben darstellt.

3. Anordnung nach einem der vorhergehenden Ansprüche,
**dadurch gekennzeichnet, dass**
die Signalverarbeitungseinheit (5, 5.2, 51) dazu ausgestaltet ist, die Ausgabeeinheit (7, 7.1, 52) so anzusteuern,
dass die angesteuerte Ausgabeeinheit (7, 5.2, 51)
- die Alarm-Gesamt-Abfolge (16) und den Alarm-Referenz-Abschnitt (26) mit demselben Zeitmaßstab darstellt und
- den Alarm-Referenz-Abschnitt (26) zeitrichtig relativ zur Alarm-Gesamt-Abfolge positioniert darstellt.

4. Anordnung nach einem der vorhergehenden Ansprüche,
**dadurch gekennzeichnet, dass**
die Signalverarbeitungseinheit (5, 5.2, 51) dazu ausgestaltet ist, die Ausgabeeinheit (7, 7.1, 52) so anzusteuern,
dass die angesteuerte Ausgabeeinheit (7, 5.2, 51) in der Signalverlaufs-Darstellung (10) und / oder in dem Alarm-Referenz-Abschnitt (26) und / oder in der Alarm-Referenz-Abfolge (18) einen im Referenz-Zeitfenster (T1) liegenden Referenz-Zeitpunkt (t0) darstellt,
wobei die Signalverarbeitungseinheit (5, 5.2, 51) dazu ausgestaltet ist, eine Benutzereingabe zur Veränderung des dargestellten Referenz-Zeitpunkts (t0) zu erfassen und
wobei die Signalverarbeitungseinheit (5, 5.2, 51) dazu ausgestaltet ist, nach der Erfassung der Benutzereingabe zur Veränderung des Referenz-Zeitpunkts (t0', t0")
- dann, wenn der veränderte Referenz-Zeitpunkt (t0', t0") nicht im Referenz-Zeitfenster (T1) liegt, das Referenz-Zeitfenster (T1) oder den Referenz-Zeitpunkt (t0) so zu verändern, dass der veränderte Referenz-Zeitpunkt (t0', t0") im Referenz-Zeitfenster (T1) liegt, und
- in der Signalverlaufs-Darstellung (10) und / oder in dem Alarm-Referenz-Abschnitt (26) und / oder in der Alarm-Referenz-Abfolge (18) den veränderten Referenz-Zeitpunkt (t0', t0") darstellt.

5. Anordnung nach Anspruch 4,
**dadurch gekennzeichnet, dass**
die Signalverarbeitungseinheit (5, 5.2, 51) dazu ausgestaltet ist,
- die Auswahl eines Alarms (12), der in der Signalverlaufs-Darstellung (10) und / oder in dem Alarm-Referenz-Abschnitt (26) und / oder in der Alarm-Referenz-Abfolge (18) dargestellt ist, durch einen Benutzer zu erfassen,
- nach Auswahl eines Alarms (12) denjenigen Zeitpunkt (t0', t0"), an dem der ausgewählte Alarm (12) aufgetreten ist, als eine Benutzereingabe zur Veränderung des Referenz-Zeitpunkts (t0) zu verwenden und
- den Zeitpunkt (t0', t0"), an dem der ausgewählte Alarm (12) aufgetreten ist, als den veränderten Referenz-Zeitpunkt (t0', t0") zu verwenden.

6. Anordnung nach einem der vorhergehenden Ansprüche,
**dadurch gekennzeichnet, dass**
die Signalverarbeitungseinheit (5, 5.2, 51) dazu ausgestaltet ist,
die Auswahl eines Alarms (12), der in der Signalverlaufs-Darstellung (10) und / oder in dem Alarm-Referenz-Abschnitt (26) und / oder in der Alarm-Referenz-Abfolge (18) dargestellt ist, durch einen Benutzer zu erfassen und
nach Auswahl eines Alarms (12) jeden weiteren Alarm, der in der Signalverlaufs-Darstellung (10) und / oder in dem Alarm-Referenz-Abschnitt (26) und / oder in der Alarm-Referenz-Abfolge (18) dargestellt ist und von der gleichen Art wie der ausgewählte Alarm (12) ist, hervorgehoben darzustellen.

7. Anordnung nach einem der vorhergehenden Ansprüche,
**dadurch gekennzeichnet, dass**
die Signalverarbeitungseinheit (5, 5.2, 51) dazu ausgestaltet ist,
eine Benutzereingabe zur Veränderung des Referenz-Zeitfensters (T1) zu erfassen und
nach der Erfassung zur Veränderung des Referenz-Zeitfensters (T1) die Ausgabeeinheit (7, 7.1, 52) so anzusteuern, dass die angesteuerte Ausgabeeinheit (7, 7.1, 52)
- den Alarm-Referenz-Abschnitt (26) und die Signalverlaufs-Darstellung (10) und / oder die Alarm-Referenz-Abfolge (18) an die Veränderung des Referenz-Zeitfensters (T1) anpasst und
- die Alarm-Gesamt-Abfolge (16) unverändert lässt.

8. Anordnung nach einem der vorhergehenden Ansprüche,
**dadurch gekennzeichnet, dass**
die Signalverarbeitungseinheit (5, 5.2, 51) dazu ausgestaltet ist, die Ausgabeeinheit (7, 7.1, 52) so anzusteuern, dass die angesteuerte Ausgabeeinheit (7, 7.1, 52) eine Alarm-Beschreibungs-Abfolge (11) darstellt, wobei die Alarm-Beschreibungs-Abfolge (11) für eine Sequenz von Alarmen, die zu der in der Alarm-Alarm-Gesamt-Abfolge (16) dargestellten zeitlichen Abfolge gehören, jeweils eine textliche Alarm-Beschreibung (31) umfasst,
wobei die Listen-Richtung (LR), in welche sich die Alarm-Beschreibungs-Abfolge (11) erstreckt, senkrecht auf der Zeitachsen-Darstellungs-Richtung (ZR) steht und wobei die jeweilige Schreibrichtung (SR) jeder Alarm-Beschreibung (31) senkrecht auf der Listen-Richtung (LR) steht.

9. Anordnung nach Anspruch 8,
**dadurch gekennzeichnet, dass**
die Signalverarbeitungseinheit (5, 5.2, 51) dazu ausgestaltet ist, die Ausgabeeinheit (7, 7.1, 52) so anzusteuern, dass die angesteuerte Ausgabeeinheit (7, 7.1, 52)
- die Alarm-Referenz-Abfolge (18) und
- einen Korrelationsanzeiger (32, 33) mit einem führenden Element (32) und einem geführten Element (33)
darstellt,
wobei
- das führende Element (32) auf eine Alarm-Beschreibung (31) in der Alarm-Beschreibungs-Abfolge (11) zeigt und
- das geführte Element (33) auf denjenigen Alarm, auf den sich diese Alarm-Beschreibung (31) bezieht, in der Alarm-Referenz-Abfolge (18) zeigt oder
wobei
- das führende Element (32) auf einen Alarm in der Alarm-Referenz-Abfolge (18) zeigt und
- das geführte Element (33) auf diejenige Alarm-Beschreibung (31) in der Alarm-Beschreibungs-Abfolge (11), die sich auf diesen Alarm bezieht, zeigt.

10. Anordnung nach einem der vorhergehenden Ansprüche,
**dadurch gekennzeichnet, dass**
die Signalverarbeitungseinheit (5, 5.2, 51) ein erstes Signalverarbeitungsgerät (5, 5.2) und ein zweites Signalverarbeitungsgerät (51) umfasst, die räumlich voneinander getrennt sind,
wobei das erste Signalverarbeitungsgerät (5, 5.2) dem medizinischen Gerät (1, 1.2) zugeordnet ist und dazu ausgestaltet ist,
- die Messwerte zu empfangen,
- das oder mindestens ein Signal (VT, MV, RR, SpO2) zu erzeugen,
- zu entscheiden, ob das oder ein Alarm-Kriterium (MV low, SPO2 low) erfüllt ist,
- einen Alarm (12, 12.1, 12.2, 34, 35) sowie den Zeitpunkt, an dem dieser Alarm (12, 12.1, 12.2, 34, 35) aufgetreten ist, zu detektieren und
- eine Nachricht an das zweite Signalverarbeitungsgerät (51) zu übermitteln, wobei diese Nachricht eine Information über den Alarm (12, 12.1, 12.2, 34, 35) und über den Zeitpunkt umfasst, und
wobei das zweite Signalverarbeitungsgerät (51) dazu ausgestaltet ist, die Ausgabeeinheit (52) so anzusteuern, dass die angesteuerte Ausgabeeinheit (52)
- die Alarm-Gesamt-Abfolge (16),
- den Alarm-Referenz-Abschnitt (26),
- die Darstellung der zeitlichen Positionierung sowie
- die Signalverlaufs-Darstellung (10) und / oder die Alarm-Referenz-Abfolge (18) darstellt.

11. Verfahren zum Erzeugen von Alarmen (12, 12.1, 12.2, 34, 35) und zum Darstellen der Alarme auf einer Ausgabeeinheit (7, 7.1, 52) zur visuellen Ausgabe von Informationen an einen Benutzer,
wobei die Ausgabeeinheit (7, 7.1, 52) ein Bestandteil einer Anordnung ist, die zusätzlich
- ein medizinisches Gerät (1, 1.2), insbesondere ein Beatmungsgerät oder ein Anästhesiegerät,
- mindestens einen Patienten-Sensor, bevorzugt mehrere Patienten-Sensoren (2.1.1 bis 2.2.2, 3, 4, 6) und
- eine Signalverarbeitungseinheit (5, 5.2, 51)
umfasst,
wobei der oder jeder Patienten-Sensor (2.1.1 bis 2.2.2, 3, 4, 6) jeweils mindestens eine an oder in einem mit dem medizinischen Gerät (1, 1.2) verbundenen Patienten (P) auftretende Größe zu messen vermag,
wobei das Verfahren die Schritte umfasst, dass die Signalverarbeitungseinheit (5, 5.2, 51) automatisch
- Messwerte von dem oder mindestens einen Patienten-Sensor (2.1.1 bis 2.2.2, 3, 4, 6) empfängt,
- durch Auswertung von empfangenen Messwerten mindestens ein Signal (VT, MV, RR, SpO2) erzeugt,
- entscheidet, ob mindestens ein vorgegebenes Alarm-Kriterium (MV low, SPO2 low) erfüllt ist,
wobei das oder jedes vorgegebene Alarm-Kriterium (MV low, SPO2 low) sich auf das oder jeweils mindestens ein von der Signalverarbeitungseinheit (5, 5.2, 51) erzeugtes Signal (VT, MV, RR, SpO2) bezieht,
- als Reaktion auf eine Entscheidung, dass das oder ein Alarm-Kriterium (MV low, SPO2 low) erfüllt ist, einen Alarm (12, 12.1, 12.2, 34, 35) sowie einen Zeitpunkt, an dem dieser Alarm (12, 12.1, 12.2, 34, 35) aufgetreten ist, detektiert,
wobei das Erfülltsein des Alarm-Kriteriums (MV low, SPO2 low) das Auftreten des Alarms (12, 12.1, 12.2, 34, 35) bedeutet, und
- die Ausgabeeinheit (7, 7.1, 52) ansteuert,
wobei die Signalverarbeitungseinheit (5, 5.2, 51) die Ausgabeeinheit (7, 7.1, 52) so ansteuert, dass die Ausgabeeinheit (7, 7.1, 52) wenigstens zeitweise folgendes gleichzeitig darstellt:
- eine Alarm-Gesamt-Abfolge (16), das ist eine Darstellung einer zeitlichen Abfolge von Alarmen, die in einem vorgegebenen Gesamt-Zeitraum (T) detektiert wurden,
bevorzugt eine Darstellung einer zeitlichen Abfolge von allen Alarmen im Gesamt-Zeitraum (T),
wobei die Darstellung der Alarm-Gesamt-Abfolge (16) sich in einer Zeitachsen-Darstellungs-Richtung (ZR) erstreckt,
- einen Alarm-Referenz-Abschnitt (26), das ist eine Darstellung derjenigen in der Alarm-Gesamt-Abfolge (16) gezeigten Alarme, die in einem vorgegebenen Referenz-Zeitfenster (T1) detektiert wurden,
bevorzugt eine Darstellung aller im Referenz-Zeitfenster (T1) detektierten Alarmen,
wobei das Referenz-Zeitfenster (T1) ein Ausschnitt des Gesamt-Zeitraums (T) ist, und
- eine Signalverlaufs-Darstellung (10) oder eine Alarm-Referenz-Abfolge (18) oder sowohl eine Signalverlaufs-Darstellung (10) als auch eine Alarm-Referenz-Abfolge (18),
wobei die Darstellung des Alarm-Referenz-Abschnitts (26) sich in die Zeitachsen-Darstellungs-Richtung (ZR) erstreckt,
wobei der Alarm-Referenz-Abschnitt (26) eine Positionierungs-Darstellung bereitstellt, das ist eine Darstellung der zeitlichen Positionierung des Referenz-Zeitfensters (T1) relativ zum Gesamt-Zeitraum (T),
wobei die Signalverlaufs-Darstellung (10) eine Darstellung des jeweiligen zeitlichen Verlaufs des oder mindestens eines erzeugten Signals (VT, MV, RR, SpO2) im Referenz-Zeitfenster (T1) ist,
wobei die Alarm-Referenz-Abfolge (18) eine Darstellung einer Abfolge von Alarmen ist, die im Referenz-Zeitfenster (T1) aufgetreten sind, bevorzugt von allen Alarmen, die im Referenz-Zeitfenster (T1) aufgetreten sind,
wobei die Signalverlaufs-Darstellung (10) und die Alarm-Referenz-Abfolge (18) sich in die Zeitachsen-Darstellungs-Richtung (ZR) erstrecken und
wobei
- der Zeitmaßstab für die Signalverlaufs-Darstellung (10) und der Zeitmaßstab für die Alarm-Referenz-Abfolge (18) feiner sind als
- der Zeitmaßstab für die Alarm-Gesamt-Abfolge (16) und der Zeitmaßstab für den Alarm-Referenz-Abschnitt (26).

12. Computerprogramm, welches auf einer Signalverarbeitungseinheit (5, 5.2, 51) ausführbar ist,
wobei die Signalverarbeitungseinheit (5, 5.2, 51) ein Bestandteil einer Anordnung ist, die zusätzlich
- ein medizinisches Gerät (1, 1.2), insbesondere ein Beatmungsgerät oder ein Anästhesiegerät,
- mindestens einen Patienten-Sensor, bevorzugt mehrere Patienten-Sensoren (2.1.1 bis 2.2.2, 3, 4, 6),
- eine Signalverarbeitungseinheit (5, 5.2, 51) und
- eine Ausgabeeinheit (7, 7.1, 52) zur visuellen Ausgabe von Informationen an einen Benutzer
umfasst,
wobei der oder jeder Patienten-Sensor (2.1.1 bis 2.2.2, 3, 4, 6) jeweils mindestens eine an oder in einem Patienten (P) auftretende Größe zu messen vermag,
wobei das Computerprogramm bei einer Ausführung auf der Signalverarbeitungseinheit (5, 5.2, 51) dann, wenn die Signalverarbeitungseinheit (5, 5.2, 51)
- Messwerte von dem oder mindestens einen Patienten-Sensor (2.1.1 bis 2.2.2, 3, 4, 6) empfängt und
- die Ausgabeeinheit (7, 7.1, 52) ansteuert,
bewirkt, dass die Signalverarbeitungseinheit (5, 5.2, 51) ein Verfahren nach Anspruch 11 durchführt.

13. Signalfolge, umfassend Befehle, die auf einer Signalverarbeitungseinheit (5, 5.2, 51) ausführbar sind,
wobei die Signalverarbeitungseinheit (5, 5.2, 51) ein Bestandteil einer Anordnung ist, die zusätzlich
- ein medizinisches Gerät (1, 1.2), insbesondere ein Beatmungsgerät oder ein Anästhesiegerät,
- mindestens einen Patienten-Sensor, bevorzugt mehrere Patienten-Sensoren (2.1.1 bis 2.2.2, 3, 4, 6),
- eine Signalverarbeitungseinheit (5, 5.2, 51) und
- eine Ausgabeeinheit (7, 7.1, 52) zur visuellen Ausgabe von Informationen an einen Benutzer
umfasst,
wobei der oder jeder Patienten-Sensor (2.1.1 bis 2.2.2, 3, 4, 6) jeweils mindestens eine an oder in einem Patienten (P) auftretende Größe zu messen vermag, wobei die Signalfolge bei einer Ausführung auf der Signalverarbeitungseinheit (5, 5.2, 51) dann, wenn die Signalverarbeitungseinheit (5, 5.2, 51)
- Messwerte von dem oder mindestens einen Patienten-Sensor (2.1.1 bis 2.2.2, 3, 4, 6) empfängt und
- die Ausgabeeinheit (7, 7.1, 52) ansteuert,
bewirkt, dass die Signalverarbeitungseinheit (5, 5.2, 51) ein Verfahren nach Anspruch 11 durchführt.

## Claims

1. Arrangement comprising
- a medical device (1, 1.2), in particular a ventilator or an anesthesia device,
- at least one patient sensor, preferably a plurality of patient sensors (2.1.1 to 2.2.2, 3, 4, 6),
- a signal processing unit (5, 5.2, 51) and
- an output unit (7, 7.1, 52) for visually outputting information to a user,
wherein the or each patient sensor (2.1.1 to 2.2.2, 3, 4, 6) is capable of measuring at least one variable occurring on or in a patient (P) connected to the medical device (1, 1.2),
wherein the signal processing unit (5, 5.2, 51) is designed to automatically
- receive measured values from the or at least one patient sensor (2.1.1 to 2.2.2, 3, 4, 6),
- generate at least one signal (VT, MV, RR, SpO2) by evaluating received measured values,
- decide whether at least one predetermined alarm criterion (MV low, SPO2 low) is fulfilled,
wherein the or each predetermined alarm criterion (MV low, SPO2 low) relates to the or at least one signal (VT, MV, RR, SpO2) that can be generated by the signal processing unit (5, 5.2, 51),
- detect an alarm (12, 12.1, 12.2, 34, 35) and a time at which this alarm occurred in response to a decision that the or an alarm criterion (MV low, SPO2 low) is fulfilled,
wherein the fulfillment of the alarm criterion (MV low, SPO2 low) means the occurrence of the alarm, and
- control the output unit (7, 7.1, 52),
wherein the signal processing unit (5, 5.2, 51) is further designed to control the output unit (7, 7.1, 52) in such a way that the controlled output unit (7, 7.1, 52) at least temporarily displays the following simultaneously:
- an overall alarm sequence (16), which is a representation of a temporal sequence of alarms that were detected in a predetermined overall period (T),
preferably a representation of a temporal sequence of all alarms in the overall period (T),
wherein the representation of the overall alarm sequence (16) extends in a time axis representation direction (ZR),
- an alarm reference portion (26), which is a representation of the alarms shown in the overall alarm sequence (16) that were detected in a predetermined reference time window (T1),
preferably a representation of all alarms detected in the reference time window (T1),
wherein the reference time window (T1) is a portion of the overall period (T), and
- a signal waveform representation (10) or an alarm reference sequence (18) or both a signal waveform representation (10) and an alarm reference sequence (18),
wherein the representation of the alarm reference portion (26) extends in the time axis representation direction (ZR),
wherein the alarm reference portion (26) provides a positioning representation, that is a representation of the temporal positioning of the reference time window (T1) relative to the overall period (T),
wherein the signal waveform representation (10) is a representation of the particular temporal waveform of the or at least one generated signal (VT, MV, RR, SpO2) in the reference time window (T1),
wherein the alarm reference sequence (18) is a representation of a sequence of alarms that occurred in the reference time window (T1), preferably of all alarms that occurred in the reference time window (T1),
wherein the signal waveform representation (10) and the alarm reference sequence (18) extend in the time axis representation direction (ZR) and
wherein
- the time scale for the signal waveform representation (10) and the time scale for the alarm reference sequence (18) are finer than
- the time scale for the overall alarm sequence (16) and the time scale for the alarm reference portion (26).

2. Arrangement according to claim 1,
**characterized in that**
at least two different alarm types (MV low, RR high) are predetermined,
each alarm type being defined by a predetermined alarm criterion (MV low, SPO2 low),
the signal processing unit (5, 5.2, 51) being designed to detect the selection of an alarm (12) by a user and
the signal processing unit (5, 5.2, 51) being further designed to control the output unit (7, 7.1, 52) after selection of an alarm (12) in such a way that the controlled output unit (7, 7.1, 52) in the overall alarm sequence (16) and/or in the alarm reference portion (26) and/or in the alarm reference sequence (18)
highlights each further alarm (12.1, 12.2) belonging to the same alarm type (MV low) as the selected alarm (12) in comparison with the other displayed alarms (34, 35).

3. Arrangement according to either of the preceding claims,
**characterized in that**
the signal processing unit (5, 5.2, 51) is designed to control the output unit (7, 7.1, 52) in such a way
that the controlled output unit (7, 5.2, 51)
- displays the overall alarm sequence (16) and the alarm reference portion (26) with the same time scale and
- displays the alarm reference portion (26) positioned correctly in time relative to the overall alarm sequence.

4. Arrangement according to any of the preceding claims,
**characterized in that**
the signal processing unit (5, 5.2, 51) is designed to control the output unit (7, 7.1, 52) in such a way
that the controlled output unit (7, 5.2, 51) displays a reference time (t0) in the reference time window (T1) in the signal waveform representation (10) and/or in the alarm reference portion (26) and/or in the alarm reference sequence (18),
the signal processing unit (5, 5.2, 51) being designed to detect a user input for changing the displayed reference time (t0) and
the signal processing unit (5, 5.2, 51) being designed, after detecting the user input for changing the reference time (t0', t0"),
- if the changed reference time (t0', t0") is not in the reference time window (T1), to change the reference time window (T1) or the reference time (t0) in such a way that the changed reference time (t0', t0") is in the reference time window (T1), and
- to display the changed reference time (t0', t0") in the signal waveform representation (10) and/or in the alarm reference portion (26) and/or in the alarm reference sequence (18).

5. Arrangement according to claim 4,
**characterized in that**
the signal processing unit (5, 5.2, 51) is designed
- to detect the selection of an alarm (12) displayed in the signal waveform representation (10) and/or in the alarm reference portion (26) and/or in the alarm reference sequence (18) by a user,
- after an alarm (12) has been selected, to use the time (t0', t0") at which the selected alarm (12) occurred as a user input for changing the reference time (t0) and
- to use the time (t0', t0") at which the selected alarm (12) occurred as the changed reference time (t0', t0").

6. Arrangement according to any of the preceding claims,
**characterized in that**
the signal processing unit (5, 5.2, 51) is designed
to detect the selection of an alarm (12) displayed in the signal waveform representation (10) and/or in the alarm reference portion (26) and/or in the alarm reference sequence (18) by a user and
after an alarm (12) has been selected, to highlight any further alarm displayed in the signal waveform representation (10) and/or in the alarm reference portion (26) and/or in the alarm reference sequence (18) and of the same type as the selected alarm (12).

7. Arrangement according to any of the preceding claims,
**characterized in that**
the signal processing unit (5, 5.2, 51) is designed
to detect a user input for changing the reference time window (T1) and
after the change in the reference time window (T1) has been detected, to control the output unit (7, 7.1, 52) in such a way that the controlled output unit (7, 7.1, 52)
- adapts the alarm reference portion (26) and the signal waveform representation (10) and/or the alarm reference sequence (18) to the change in the reference time window (T1) and
- leaves the overall alarm sequence (16) unchanged.

8. Arrangement according to any of the preceding claims,
**characterized in that**
the signal processing unit (5, 5.2, 51) is designed to control the output unit (7, 7.1, 52) in such a way that the controlled output unit (7, 7.1, 52) displays an alarm description sequence (11),
the alarm description sequence (11) comprising a textual alarm description (31) for each of a sequence of alarms belonging to the temporal sequence displayed in the alarm overall alarm sequence (16),
the list direction (LR) in which the alarm description sequence (11) extends being perpendicular to the time axis representation direction (ZR) and the particular writing direction (SR) of each alarm description (31) being perpendicular to the list direction (LR).

9. Arrangement according to claim 8,
**characterized in that**
the signal processing unit (5, 5.2, 51) is designed to control the output unit (7, 7.1, 52) in such a way that the controlled output unit (7, 7.1, 52) displays
- the alarm reference sequence (18) and
- a correlation indicator (32, 33) having a leading element (32) and a guided element (33),
- the leading element (32) pointing to an alarm description (31) in the alarm description sequence (11) and
- the guided element (33) pointing to the alarm to which this alarm description (31) refers in the alarm reference sequence (18) or
- the leading element (32) pointing to an alarm in the alarm reference sequence (18) and
- the guided element (33) pointing to the alarm description (31) in the alarm description sequence (11) that relates to this alarm.

10. Arrangement according to any of the preceding claims,
**characterized in that**
the signal processing unit (5, 5.2, 51) comprises a first signal processing device (5, 5.2) and a second signal processing device (51) which are spatially separated from one another,
the first signal processing device (5, 5.2) being assigned to the medical device (1, 1.2) and being designed
- to receive the measured values,
- to generate the or at least one signal (VT, MV, RR, SpO2),
- to decide whether the or an alarm criterion (MV low, SPO2 low) is fulfilled,
- to detect an alarm (12, 12.1, 12.2, 34, 35) and the time at which this alarm (12, 12.1, 12.2, 34, 35) occurred, and
- to transmit a message to the second signal processing device (51), said message comprising information about the alarm (12, 12.1, 12.2, 34, 35) and about the time, and
the second signal processing device (51) being designed to control the output unit (52) in such a way that the controlled output unit (52) displays
- the overall alarm sequence (16),
- the alarm reference portion (26),
- the representation of the temporal positioning and
- the signal waveform representation (10) and/or the alarm reference sequence (18).

11. Method for generating alarms (12, 12.1, 12.2, 34, 35) and for displaying the alarms on an output unit (7, 7.1, 52) for visually outputting information to a user,
wherein the output unit (7, 7.1, 52) is a component of an arrangement which additionally comprises
- a medical device (1, 1.2), in particular a ventilator or an anesthesia device,
- at least one patient sensor, preferably a plurality of patient sensors (2.1.1 to 2.2.2, 3, 4, 6) and
- a signal processing unit (5, 5.2, 51),
wherein the or each patient sensor (2.1.1 to 2.2.2, 3, 4, 6) is capable of measuring at least one variable occurring on or in a patient (P) connected to the medical device (1, 1.2),
wherein the method comprises the steps whereby the signal processing unit (5, 5.2, 51) automatically
- receives measured values from the or at least one patient sensor (2.1.1 to 2.2.2, 3, 4, 6),
- generates at least one signal (VT, MV, RR, SpO2) by evaluating received measured values,
- decides whether at least one predetermined alarm criterion (MV low, SPO2 low) is fulfilled,
wherein the or each predetermined alarm criterion (MV low, SPO2 low) relates to the or at least one signal (VT, MV, RR, SpO2) generated by the signal processing unit (5, 5.2, 51),
- detects an alarm (12, 12.1, 12.2, 34, 35) and a time at which this alarm (12, 12.1, 12.2, 34, 35) occurred in response to a decision that the or an alarm criterion (MV low, SPO2 low) is fulfilled,
wherein the fulfillment of the alarm criterion (MV low, SPO2 low) means the occurrence of the alarm (12, 12.1, 12.2, 34, 35), and
- controls the output unit (7, 7.1, 52),
wherein the signal processing unit (5, 5.2, 51) controls the output unit (7, 7.1, 52) in such a way that the output unit (7, 7.1, 52) at least temporarily displays the following simultaneously:
- an overall alarm sequence (16), which is a representation of a temporal sequence of alarms that were detected in a predetermined overall period (T),
preferably a representation of a temporal sequence of all alarms in the overall period (T),
wherein the representation of the overall alarm sequence (16) extends in a time axis representation direction (ZR),
- an alarm reference portion (26), which is a representation of the alarms shown in the overall alarm sequence (16) that were detected in a predetermined reference time window (T1),
preferably a representation of all alarms detected in the reference time window (T1),
wherein the reference time window (T1) is a portion of the overall period (T), and
- a signal waveform representation (10) or an alarm reference sequence (18) or both a signal waveform representation (10) and an alarm reference sequence (18),
wherein the representation of the alarm reference portion (26) extends in the time axis representation direction (ZR),
wherein the alarm reference portion (26) provides a positioning representation, that is a representation of the temporal positioning of the reference time window (T1) relative to the overall period (T),
wherein the signal waveform representation (10) is a representation of the particular temporal waveform of the or at least one generated signal (VT, MV, RR, SpO2) in the reference time window (T1),
wherein the alarm reference sequence (18) is a representation of a sequence of alarms that occurred in the reference time window (T1), preferably of all alarms that occurred in the reference time window (T1),
wherein the signal waveform representation (10) and the alarm reference sequence (18) extend in the time axis representation direction (ZR) and
wherein
- the time scale for the signal waveform representation (10) and the time scale for the alarm reference sequence (18) are finer than
- the time scale for the overall alarm sequence (16) and the time scale for the alarm reference portion (26).

12. Computer program which can be executed on a signal processing unit (5, 5.2, 51),
wherein the signal processing unit (5, 5.2, 51) is a component of an arrangement which additionally comprises
- a medical device (1, 1.2), in particular a ventilator or an anesthesia device,
- at least one patient sensor, preferably a plurality of patient sensors (2.1.1 to 2.2.2, 3, 4, 6),
- a signal processing unit (5, 5.2, 51) and
- an output unit (7, 7.1, 52) for visually outputting information to a user,
wherein the or each patient sensor (2.1.1 to 2.2.2, 3, 4, 6) is capable of measuring at least one variable occurring on or in a patient (P),
wherein the computer program, when executed on the signal processing unit (5, 5.2, 51), when the signal processing unit (5, 5.2, 51)
- receives measured values from the or at least one patient sensor (2.1.1 to 2.2.2, 3, 4, 6) and
- controls the output unit (7, 7.1, 52),
causes the signal processing unit (5, 5.2, 51) to carry out a method according to claim 11.

13. Signal sequence comprising commands that can be executed on a signal processing unit (5, 5.2, 51),
wherein the signal processing unit (5, 5.2, 51) is a component of an arrangement which additionally comprises
- a medical device (1, 1.2), in particular a ventilator or an anesthesia device,
- at least one patient sensor, preferably a plurality of patient sensors (2.1.1 to 2.2.2, 3, 4, 6),
- a signal processing unit (5, 5.2, 51) and
- an output unit (7, 7.1, 52) for visually outputting information to a user,
wherein the or each patient sensor (2.1.1 to 2.2.2, 3, 4, 6) is capable of measuring at least one variable occurring on or in a patient (P),
wherein the signal sequence, when executed on the signal processing unit (5, 5.2, 51), when the signal processing unit (5, 5.2, 51)
- receives measured values from the or at least one patient sensor (2.1.1 to 2.2.2, 3, 4, 6) and
- controls the output unit (7, 7.1, 52),
causes the signal processing unit (5, 5.2, 51) to carry out a method according to claim 11.

## Revendications

1. Système comprenant
- un appareil médical (1, 1.2), en particulier un respirateur ou un appareil d'anesthésie,
- au moins un capteur pour patient, de préférence plusieurs capteurs pour patient (2.1.1 à 2.2.2, 3, 4, 6),
- une unité de traitement de signaux (5, 5.2, 51) et
- une unité de sortie (7, 7.1, 52) pour la sortie visuelle d'informations à un utilisateur,
dans lequel le ou chaque capteur pour patient (2.1.1 à 2.2.2, 3, 4, 6) est capable de mesurer respectivement au moins une grandeur apparaissant sur ou dans un patient (P) relié à l'appareil médical (1, 1.2),
dans lequel l'unité de traitement de signaux (5, 5.2, 51) est configurée pour, automatiquement,
- recevoir des valeurs de mesure provenant du capteur pour patient ou d'au moins un capteur pour patient (2.1.1 à 2.2.2, 3, 4, 6),
- générer au moins un signal (VT, MV, RR, SpO2) en évaluant les valeurs de mesure reçues,
- décider si au moins un critère d'alarme (MV low, SPO2 low) prédéfini est satisfait,
dans lequel le ou chaque critère d'alarme (MV low, SPO2 low) prédéfini se rapporte au ou à respectivement au moins un signal (VT, MV, RR, SpO2) pouvant être généré par l'unité de traitement de signaux (5, 5.2, 51),
- en réponse à une décision selon laquelle le ou un critère d'alarme (MV low, SPO2 low) est satisfait, détecter une alarme (12, 12.1, 12.2, 34, 35) ainsi qu'un moment où ladite alarme est apparue,
dans lequel le fait de satisfaire au critère d'alarme (MV low, SPO2 low) signifie l'apparition de l'alarme, et
- commander l'unité de sortie (7, 7.1, 52),
dans lequel l'unité de traitement de signaux (5, 5.2, 51) est en outre configurée pour commander l'unité de sortie (7, 7.1, 52) de sorte que l'unité de sortie (7, 7.1, 52) commandée représente simultanément, au moins temporairement, les éléments suivants :
- une séquence totale d'alarmes (16) qui est une représentation d'une séquence temporelle d'alarmes qui ont été détectées dans une période totale (T) prédéfinie,
de préférence une représentation d'une séquence temporelle de toutes les alarmes dans la période totale (T),
dans lequel la représentation de la séquence totale d'alarmes (16) s'étend dans une direction de représentation d'axe temporel (ZR),
- une section de référence d'alarmes (26), qui est une représentation des alarmes présentées dans la séquence totale d'alarmes (16) qui ont été détectées dans un intervalle de temps de référence (T1) prédéfini,
de préférence une représentation de toutes les alarmes détectées dans l'intervalle de temps de référence (T1),
dans lequel l'intervalle de temps de référence (T1) est une section de la période totale (T), et
- une représentation d'évolution de signal (10) ou une séquence de référence d'alarmes (18) ou à la fois une représentation d'évolution de signal (10) et une séquence de référence d'alarmes (18),
dans lequel la représentation de la section de référence d'alarmes (26) s'étend dans la direction de représentation d'axe temporel (ZR),
dans lequel la section de référence d'alarmes (26) fournit une représentation de positionnement qui est une représentation du positionnement temporel de l'intervalle de temps de référence (T1) par rapport à la période totale (T),
dans lequel la représentation d'évolution de signal (10) est une représentation de l'évolution temporelle respective du ou d'au moins un signal (VT, MV, RR, SpO2) généré dans l'intervalle de temps de référence (T1),
dans lequel la séquence de référence d'alarmes (18) est une représentation d'une séquence d'alarmes qui sont apparues dans l'intervalle de temps de référence (T1), de préférence de toutes les alarmes qui sont apparues dans l'intervalle de temps de référence (T1),
dans lequel la représentation d'évolution de signal (10) et la séquence de référence d'alarmes (18) s'étendent dans la direction de représentation d'axe temporel (ZR) et
dans lequel
- l'échelle de temps pour la représentation d'évolution de signal (10) et l'échelle de temps pour la séquence de référence d'alarmes (18) sont plus précises que
- l'échelle de temps pour la séquence totale d'alarmes (16) et l'échelle de temps pour la section de référence d'alarmes (26).

2. Système selon la revendication 1,
**caractérisé en ce que**
au moins deux types d'alarme différents (MV low, RR high) sont prédéfinis,
dans lequel chaque type d'alarme est défini par respectivement un critère d'alarme (MV low, SPO2 low) prédéfini,
dans lequel l'unité de traitement de signaux (5, 5.2, 51) est configurée pour détecter la sélection d'une alarme (12) par un utilisateur et
dans lequel l'unité de traitement de signaux (5, 5.2, 51) est en outre configurée pour, après la sélection d'une alarme (12), commander l'unité de sortie (7, 7.1, 52) de sorte que l'unité de sortie (7, 7.1, 52) commandée représente, dans la séquence totale d'alarmes (16) et/ou dans la section de référence d'alarmes (26) et/ou dans la séquence de référence d'alarmes (18),
chaque autre alarme (12.1, 12.2) appartenant au même type d'alarme (MV low) que l'alarme (12) sélectionnée, par rapport aux autres alarmes (34, 35) représentées.

3. Système selon l'une des revendications précédentes,
**caractérisé en ce que**
l'unité de traitement de signaux (5, 5.2, 51) est configurée pour commander l'unité de sortie (7, 7.1, 52) de sorte que
l'unité de sortie (7, 5.2, 51) commandée
- représente la séquence totale d'alarmes (16) et la section de référence d'alarmes (26) avec la même échelle de temps et
- représente la section de référence d'alarmes (26) positionnée au moment exact par rapport à la séquence totale d'alarmes.

4. Système selon l'une des revendications précédentes,
**caractérisé en ce que**
l'unité de traitement de signaux (5, 5.2, 51) est configurée pour commander l'unité de sortie (7, 7.1, 52) de sorte que
l'unité de sortie (7, 5.2, 51) commandée représente, dans la représentation d'évolution de signal (10) et/ou dans la section de référence d'alarmes (26) et/ou dans la séquence de référence d'alarmes (18), un instant de référence (t0) situé dans l'intervalle de temps de référence (T1),
dans lequel l'unité de traitement de signaux (5, 5.2, 51) est configurée pour détecter une entrée utilisateur pour la modification de l'instant de référence (t0) représenté et
dans lequel l'unité de traitement de signaux (5, 5.2, 51) est configurée pour, après la détection de l'entrée utilisateur pour la modification de l'instant de référence (t0', t0"),
- si l'instant de référence modifié (t0', t0") ne se situe pas dans l'intervalle de temps de référence (T1), modifier l'intervalle de temps de référence (T1) ou l'instant de référence (t0) de sorte que l'instant de référence modifié (t0', t0") se situe dans l'intervalle de temps de référence (T1), et
- dans la représentation d'évolution de signal (10) et/ou dans la section de référence d'alarmes (26) et/ou dans la séquence de référence d'alarmes (18), représenter l'instant de référence modifié (t0', t0").

5. Système selon la revendication 4,
**caractérisé en ce que**
l'unité de traitement de signaux (5, 5.2, 51) est configurée pour
- détecter la sélection par un utilisateur d'une alarme (12) représentée dans la représentation d'évolution de signal (10) et/ou dans la section de référence d'alarmes (26) et/ou dans la séquence de référence d'alarmes (18),
- après la sélection d'une alarme (12), utiliser l'instant (t0', t0") auquel l'alarme (12) sélectionnée est apparue comme entrée utilisateur pour la modification de l'instant de référence (t0) et
- utiliser l'instant (t0', t0") auquel l'alarme (12) sélectionnée est apparue comme instant de référence modifié (t0', t0").

6. Système selon l'une des revendications précédentes,
**caractérisé en ce que**
l'unité de traitement de signaux (5, 5.2, 51) est configurée pour
détecter la sélection par un utilisateur d'une alarme (12) représentée dans la représentation d'évolution de signal (10) et/ou dans la section de référence d'alarmes (26) et/ou dans la séquence de référence d'alarmes (18) et
après la sélection d'une alarme (12), représenter, en la mettant en avant, chaque autre alarme qui est représentée dans la représentation d'évolution de signal (10) et/ou dans la section de référence d'alarmes (26) et/ou dans la séquence de référence d'alarmes (18) et qui est du même type que l'alarme (12) sélectionnée.

7. Système selon l'une des revendications précédentes,
**caractérisé en ce que**
l'unité de traitement de signaux (5, 5.2, 51) est configurée pour
détecter une entrée utilisateur pour la modification de l'intervalle de temps de référence (T1) et
après la détection pour la modification de l'intervalle de temps de référence (T1), commander l'unité de sortie (7, 7.1, 52) de sorte que l'unité de sortie (7, 7.1, 52) commandée
- adapte la section de référence d'alarmes (26) et la représentation d'évolution de signal (10) et/ou la séquence de référence d'alarmes (18) à la modification de l'intervalle de temps de référence (T1) et
- laisse la séquence totale d'alarmes (16) sans modification.

8. Système selon l'une des revendications précédentes,
**caractérisé en ce que**
l'unité de traitement de signaux (5, 5.2, 51) est configurée pour commander l'unité de sortie (7, 7.1, 52) de sorte que l'unité de sortie (7, 7.1, 52) commandée représente une séquence de descriptions d'alarmes (11),
dans lequel la séquence de descriptions d'alarmes (11) comprend respectivement une description d'alarmes textuelle (31) pour une séquence d'alarmes appartenant à la séquence temporelle représentée dans la séquence totale d'alarmes d'alarmes (16),
dans lequel la direction de liste (LR) dans laquelle s'étend la séquence de descriptions d'alarmes (11) est perpendiculaire à la direction de représentation d'axe temporel (ZR) et dans lequel la direction d'écriture (SR) respective de chaque description d'alarme (31) est perpendiculaire à la direction de liste (LR).

9. Système selon la revendication 8,
**caractérisé en ce que**
l'unité de traitement de signaux (5, 5.2, 51) est configurée pour commander l'unité de sortie (7, 7.1, 52) de sorte que l'unité de sortie (7, 7.1, 52) commandée représente
- la séquence de référence d'alarmes (18) et
- un indicateur de corrélation (32, 33) comportant un élément de guidage (32) et un élément guidé (33)
dans lequel
- l'élément de guidage (32) pointe vers une description d'alarme (31) dans la séquence de descriptions d'alarmes (11) et
- l'élément guidé (33) pointe vers l'alarme à laquelle se rapporte ladite description d'alarme (31) dans la séquence de référence d'alarmes (18) ou
dans lequel
- l'élément de guidage (32) pointe vers une alarme dans la séquence de référence d'alarmes (18) et
- l'élément guidé (33) pointe vers la description d'alarme (31) dans la séquence de descriptions d'alarmes (11) qui se rapporte à ladite alarme.

10. Système selon l'une des revendications précédentes,
**caractérisé en ce que**
l'unité de traitement de signaux (5, 5.2, 51) comprend un premier appareil de traitement de signaux (5, 5.2) et un second appareil de traitement de signaux (51) qui sont séparés spatialement l'un de l'autre,
dans lequel le premier appareil de traitement de signaux (5, 5.2) est associé à l'appareil médical (1, 1.2) et est configuré pour
- recevoir les valeurs de mesure,
- générer le ou au moins un signal (VT, MV, RR, SpO2),
- décider si le ou un critère d'alarme (MV low, SPO2 low) est satisfait,
- détecter une alarme (12, 12.1, 12.2, 34, 35) ainsi que l'instant à laquelle ladite alarme (12, 12.1, 12.2, 34, 35) est apparue et
- transmettre un message au second appareil de traitement de signaux (51), dans lequel ledit message comprend des informations concernant l'alarme (12, 12.1, 12.2, 34, 35) et concernant l'instant, et
dans lequel le second appareil de traitement de signaux (51) est configuré pour commander l'unité de sortie (52) de sorte que l'unité de sortie (52) commandée représente
- la séquence totale d'alarmes (16),
- la section de référence d'alarmes (26),
- la représentation du positionnement dans le temps ainsi que
- la représentation d'évolution de signal (10) et/ou la séquence de référence d'alarmes (18).

11. Procédé pour la génération d'alarmes (12, 12.1, 12.2, 34, 35) et pour la représentation des alarmes sur une unité de sortie (7, 7.1, 52) pour la sortie visuelle d'informations à un utilisateur,
dans lequel l'unité de sortie (7, 7.1, 52) est un élément d'un système qui comprend en outre
- un appareil médical (1, 1.2), en particulier un respirateur ou un appareil d'anesthésie,
- au moins un capteur pour patient, de préférence plusieurs capteurs pour patient (2.1.1 à 2.2.2, 3, 4, 6), et
- une unité de traitement de signaux (5, 5.2, 51)
dans lequel le ou chaque capteur pour patient (2.1.1 à 2.2.2, 3, 4, 6) est capable de mesurer respectivement au moins une grandeur apparaissant sur ou dans un patient (P) relié à l'appareil médical (1, 1.2),
dans lequel le procédé comprend les étapes consistant à ce que, automatiquement, l'unité de traitement de signaux (5, 5.2, 51)
- reçoive des valeurs de mesure provenant du capteur pour patient ou d'au moins un capteur pour patient (2.1.1 à 2.2.2, 3, 4, 6),
- génère au moins un signal (VT, MV, RR, SpO2) par évaluation des valeurs de mesure reçues,
- décide si au moins un critère d'alarme (MV low, SPO2 low) prédéfini est satisfait,
dans lequel le ou chaque critère d'alarme (MV low, SPO2 low) prédéfini se rapporte au ou à respectivement au moins un signal (VT, MV, RR, SpO2) généré par l'unité de traitement de signaux (5, 5.2, 51),
- en réponse à une décision selon laquelle le ou un critère d'alarme (MV low, SPO2 low) est satisfait, détecte une alarme (12, 12.1, 12.2, 34, 35) ainsi qu'un instant auquel ladite alarme (12, 12.1, 12.2, 34, 35) est apparue,
dans lequel le fait de satisfaire au critère d'alarme (MV low, SPO2 low) signifie l'apparition de l'alarme (12, 12.1, 12.2, 34, 35), et
- commande l'unité de sortie (7, 7.1, 52),
dans lequel l'unité de traitement de signaux (5, 5.2, 51) commande l'unité de sortie (7, 7.1, 52) de sorte que l'unité de sortie (7, 7.1, 52) représente simultanément, au moins temporairement, les éléments suivants :
- une séquence totale d'alarmes (16) qui est une représentation d'une séquence temporelle d'alarmes qui ont été détectées dans une période totale (T) prédéfinie,
de préférence une représentation d'une séquence temporelle de toutes les alarmes dans la période totale (T),
dans lequel la représentation de la séquence totale d'alarmes (16) s'étend dans une direction de représentation d'axe temporel (ZR),
- une section de référence d'alarmes (26), qui est une représentation des alarmes présentées dans la séquence totale d'alarmes (16) qui ont été détectées dans un intervalle de temps de référence (T1) prédéfini,
de préférence une représentation de toutes les alarmes détectées dans l'intervalle de temps de référence (T1),
dans lequel l'intervalle de temps de référence (T1) est une section de la période totale (T), et
- une représentation d'évolution de signal (10) ou une séquence de référence d'alarmes (18) ou à la fois une représentation d'évolution de signal (10) et une séquence de référence d'alarmes (18),
dans lequel la représentation de la section de référence d'alarmes (26) s'étend dans la direction de représentation d'axe temporel (ZR),
dans lequel la section de référence d'alarmes (26) fournit une représentation de positionnement qui est une représentation du positionnement temporel de l'intervalle de temps de référence (T1) par rapport à la période totale (T),
dans lequel la représentation d'évolution de signal (10) est une représentation de l'évolution temporelle respective du ou d'au moins un signal (VT, MV, RR, SpO2) généré dans l'intervalle de temps de référence (T1),
dans lequel la séquence de référence d'alarmes (18) est une représentation d'une séquence d'alarmes qui sont apparues dans l'intervalle de temps de référence (T1), de préférence de toutes les alarmes qui sont apparues dans l'intervalle de temps de référence (T1),
dans lequel la représentation d'évolution de signal (10) et la séquence de référence d'alarmes (18) s'étendent dans la direction de représentation d'axe temporel (ZR) et
dans lequel
- l'échelle de temps pour la représentation d'évolution de signal (10) et l'échelle de temps pour la séquence de référence d'alarmes (18) sont plus précises que
- l'échelle de temps pour la séquence totale d'alarmes (16) et l'échelle de temps pour la section de référence d'alarmes (26).

12. Programme informatique pouvant être exécuté sur une unité de traitement de signaux (5, 5.2, 51),
dans lequel l'unité de traitement de signaux (5, 5.2, 51) est un élément d'un système qui comprend en outre
- un appareil médical (1, 1.2), en particulier un respirateur ou un appareil d'anesthésie,
- au moins un capteur pour patient, de préférence plusieurs capteurs pour patient (2.1.1 à 2.2.2, 3, 4, 6),
- une unité de traitement de signaux (5, 5.2, 51) et
- une unité de sortie (7, 7.1, 52) pour la sortie visuelle d'informations à un utilisateur
dans lequel le ou chaque capteur pour patient (2.1.1 à 2.2.2, 3, 4, 6) est capable de mesurer respectivement au moins une grandeur apparaissant sur ou dans un patient (P),
dans lequel le programme informatique, lorsqu'il est exécuté sur l'unité de traitement de signaux (5, 5.2, 51), lorsque l'unité de traitement de signaux (5, 5.2, 51)
- reçoit des valeurs de mesure provenant du capteur pour patient ou d'au moins un capteur pour patient (2.1.1 à 2.2.2, 3, 4, 6) et
- commande l'unité de sortie (7, 7.1, 52),
provoque la mise en œuvre par l'unité de traitement de signaux (5, 5.2, 51) d'un procédé selon la revendication 11.

13. Séquence de signaux, comprenant des instructions qui peuvent être exécutées sur une unité de traitement de signaux (5, 5.2, 51),
dans laquelle l'unité de traitement de signaux (5, 5.2, 51) est un élément d'un système qui comprend en outre
- un appareil médical (1, 1.2), en particulier un respirateur ou un appareil d'anesthésie,
- au moins un capteur pour patient, de préférence plusieurs capteurs pour patient (2.1.1 à 2.2.2, 3, 4, 6),
- une unité de traitement de signaux (5, 5.2, 51) et
- une unité de sortie (7, 7.1, 52) pour la sortie visuelle d'informations à un utilisateur
dans laquelle le ou chaque capteur pour patient (2.1.1 à 2.2.2, 3, 4, 6) est capable de mesurer respectivement au moins une grandeur apparaissant sur ou dans un patient (P),
dans laquelle la séquence de signaux, lorsqu'elle est exécutée sur l'unité de traitement de signaux (5, 5.2, 51), lorsque l'unité de traitement de signaux (5, 5.2, 51)
- reçoit des valeurs de mesure provenant du capteur pour patient ou d'au moins un capteur pour patient (2.1.1 à 2.2.2, 3, 4, 6) et
- commande l'unité de sortie (7, 7.1, 52),
provoque la mise en œuvre par l'unité de traitement de signaux (5, 5.2, 51) d'un procédé selon la revendication 11.
